# EUROPEAN PATENT APPLICATION

(11) **EP 3 121 273 A1**
(43) Date of publication of application: **25.01.2017**
(21) Application number: 15764290.1
(22) Date of filing: 20.03.2015
(51) Int. Cl.: C12N 15/00, A61K 39/395, A61P 1/16, A61P 31/14, C07K 16/10, C07K 16/46, C12N 1/15, C12N 1/19, C12N 1/21, C12N 5/10, C12N 15/09, C12P 21/08, G01N 33/576

(54) **ANTIBODY HAVING INFECTION-INHIBITING ACTIVITY AGAINST HEPATITIS C VIRUS**

(30) Priority: 20.03.2014 JP 2014058936
(71) Applicant: JAPAN AS REPRESENTED BY DIRECTOR-GENERAL OF NATIONAL INSTITUTE OF INFECTIOUS DISEASES, Shinjuku-ku Tokyo 162-8640 (JP); Medical & Biological Laboratories Co., Ltd., Nagoya-shi, Aichi 460-0008 (JP); Toray Industries, Inc., Tokyo 103-8666 (JP)
(72) Inventor: WAKITA, Takaji, Tokyo 169-0073 (JP); SHINOHARA, Midori, Aichi 460-0008 (JP); YOKOKAWA, Hiroshi, Kanagawa 248-8555 (JP); NAKAMURA, Noriko, Kanagawa 248-8555 (JP)
(74) Representative: Kador & Partner
(86) International application number: PCT/JP2015/058446
(87) International publication number: WO 2015/141826

(57) **Abstract**

The present invention provides an anti-hepatitis C virus E2 protein antibody or antigen-binding antibody fragment thereof having infection inhibiting activity against hepatitis C virus (HCV). The present invention further provides an anti-hepatitis C virus E2 protein antibody or antigen-binding antibody fragment thereof comprising a certain variable region, which have infection inhibiting activity against hepatitis C virus (HCV) and exhibits an escape mutant emergence suppressive property.

## Description

### Technical Field

The present invention relates to an antibody or fragment thereof having infection inhibiting activity against hepatitis C virus.

### Background Art

Hepatitis C virus (hereinafter also referred to as HCV) is an RNA virus classified as the family *Flaviviridae,* genus *Hepacivirus,* and was identified as a major causative virus of non-A, non-B hepatitis (Non Patent Literature 1).

HCV infects mainly through blood transfusion, but the number of patients newly infected with HCV through blood transfusion has been greatly reduced because a highly sensitive HCV detection method has been established today. On the other hand, the number of HCV carriers, including virus carriers which have not developed hepatitis, is estimated as 2 million or more in Japan, and 170 million or more in the world. This is majorly because the chronicity rate of hepatitis caused by the HCV infection is as high as 70 to 80%, and drug therapy does not provide sufficient effects. Chronic hepatitis caused by the HCV infection, namely, chronic hepatitis C, can progress to liver cirrhosis within over two decades and ultimately become worse to a liver cancer. A chronic hepatitis C patient whose condition has proceeded to liver cirrhosis can be treated by living liver transplantation, but it is known that about a half of patients have recurrence of hepatitis after the living liver transplantation, and there is currently no way to prevent the recurrence of hepatitis (Non Patent Literature 2).

For preventing the recurrence of hepatitis after the living liver transplantation, development of an antibody pharmaceutical intended for infection inhibition or exclusion of the virus is desired. It is regarded that envelope proteins of HCV are responsible for the binding of HCV to a cell surface, which is the first process of the HCV infection, and hence, the studies have been made on production of an antibody against the HCV envelope proteins. However, although about 10% of hepatitis C patients are found to have an anti-HCV envelope protein antibody in their serums, only about 10% of such patients have spontaneous cure of hepatitis C owing to the appearance of the anti-HCV envelope protein antibody, (Non Patent Literature 3). That is, a ratio of patients regarded to be cured by the anti-HCV envelope protein antibody is merely about 1% of the whole hepatitis C patients. This is considered to be due to a mechanism to inhibit or suppress the production of an antibody against HCV envelope proteins (Non Patent Literature 4). Besides, preparations containing a mixture of immunoglobulins obtained from blood plasma of a plurality of anti-HCV antibody-positive chronic hepatitis C patients have been also examined. However, it has been revealed that patient-derived anti-HCV antibody mixed preparations do not reduce the amount of HCV in plasma even if administered since the living liver transplantation (Non Patent Literature 5).

On the other hand, Non Patent Literature 4 discloses that when expressing E2 protein, which is one of the HCV envelope proteins, in a mammal, the E2 protein specifically binds to CD81 present on a human cell surface. Attempts have been made, based on this experimental result, to isolate an antibody having NOB (neutralization of binding) activity which inhibits the binding between the E2 protein and the CD81, from a hepatitis C patient. As a typical example, an antibody having the NOB activity was isolated, by using a phage display method, from an antibody gene library prepared from bone marrow lymphocytes of chronic hepatitis C patients infected with HCV of genotype 1a (Patent Literature 1). An antibody having the NOB activity was also isolated from a hybridoma produced from a peripheral B cell of a hepatitis C patient infected with HCV of genotype 1b (Non Patent Literature 6 and Patent Literature 2). It is also reported, however, that an antibody having the NOB activity does not always inhibit the infection (Non Patent Literature 7).

When an anti-HCV antibody is used as an antibody pharmaceutical, there is concern about the emergence of a virus mutant having acquired resistance, through virus genome mutation, to the inhibitory activity of the HCV infection-inhibiting antibody, namely, an escape mutant. The emergence of an HCV escape mutant caused by administration of an anti-HCV antibody has been proved in an experiment using a chimpanzee (Non Patent Literature 8).

Non Patent Literature 9 suggests that anti-HCV antibodies having infection inhibiting activity against HCV of genotype 2a suppresses the emergence of an escape mutant, but actually also discloses data indicating the emergence of an escape mutant (Patent Literature 3). Patent Literature 3 does not show infection inhibiting activity of the antibodies against HCV of genotype 1b, and since their binding activities to HCV of genotype 1b as shown therein are weak, the antibodies are thought to have no sufficient infection inhibiting activity against HCV of genotype 1b. In addition, Patent Literature 3 does not reveal whether or not the antibodies suppress the emergence of an escape mutant of HCV of another genotype different from genotype 2a.

### Citation List

### Patent Literature

Patent Literature 1: International Publication No. WO2003/064473
Patent Literature 2: International Publication No. WO2004/005316
Patent Literature 3: International Publication No. WO2013/033319

### Non Patent Literature

Non Patent Literature 1: Choo et al., Science, 1989, vol. 244, pp. 359-362
Non Patent Literature 2: Gane et al., Liver Transplantation, 2003, vol. 9, ps28-s34
Non Patent Literature 3: Matsuura et al., J. Virol., 1992, vol. 66, pp. 1425-1431
Non Patent Literature 4: Gerlach et al., Gastroenterology, 1999, vol. 117, pp. 933-941
Non Patent Literature 5: Davis et al., Liver Transplantation, 2005, vol. 11, pp. 941-949
Non Patent Literature 6: Hadlock et al., J. Virol., 2000, vol. 74, pp. 10407-10416
Non Patent Literature 7: Burioni et al., J. Virol., 2002, vol. 76, pp. 11775-11779
Non Patent Literature 8: Morin et al., Plos Pathogens, 2012, vol. 8, e1002895
Non Patent Literature 9: Keck et al., Plos Pathogens, 2012, vol. 8, e1002653

### Summary of Invention

### Technical Problem

An anti-HCV antibody having infection inhibiting activity against HCV not limited to a specific genotype but of a plurality of genotypes is particularly useful as a medicament because it is effective not only for patients infected with HCV of a plurality of genotypes but also a wide spectrum of target patients. Accordingly, there is a demand for an anti-HCV antibody having high infection inhibiting activity against HCV of a plurality of genotypes. Besides, there is a demand for an antibody strongly inhibiting infection of HCV of genotype 1b refractory to treatment with interferon and ribavirin.

Further, the emergence of an escape mutant is a major obstacle to develop an anti-HCV antibody as a medicament. Accordingly, there is a demand for an anti-HCV antibody exhibiting an escape mutant emergence suppressive property, which induces less emergence of an escape mutant.

Therefore, an object of the present invention is to provide an anti-HCV antibody inhibiting infection with HCV of a plurality of genotypes. Another object of the present invention is to provide an anti-HCV antibody exhibiting an HCV escape mutant emergence suppressive property.

### Solution to Problem

The present inventors have made earnest studies to overcome the above-described problems, resulting in finding an anti-HCV antibody having high infection inhibiting activity against HCV of a plurality of genotypes, and further finding an anti-HCV antibody exhibiting an escape mutant emergence suppressive property.

Specifically, the present invention provides the followings.
(1) An anti-hepatitis C virus E2 protein antibody or antigen-binding antibody fragment thereof, which has infection inhibiting activity against hepatitis C virus (HCV).
(2) The antibody or antigen-binding antibody fragment according to (1) above, which exhibits an escape mutant emergence suppressive property.
(3) The antibody or antigen-binding antibody fragment according to (1) or (2) above, wherein said antibody or antigen-binding antibody fragment comprises a heavy chain variable region of the following (a) or (b):
   (a) a heavy chain variable region comprising a CDR1 consisting of the amino acid sequence shown by SEQ ID NO: 5, a CDR2 consisting of the amino acid sequence shown by SEQ ID NO: 6, and a CDR3 consisting of the amino acid sequence shown by SEQ ID NO: 7; or
   (b) a heavy chain variable region comprising the amino acid sequence shown by SEQ ID NO: 2.
(4) The antibody or antigen-binding antibody fragment according to (3) above, further comprising a light chain variable region.
(5) The antibody or antigen-binding antibody fragment according to any one of (1) to (4) above, wherein said antibody or antigen-binding antibody fragment comprises a light chain variable region of any one of the following (c) to (f):
   (c) a light chain variable region comprising a CDR1 consisting of the amino acid sequence shown by SEQ ID NO: 8, a CDR2 consisting of the amino acid sequence shown by SEQ ID NO: 9 and a CDR3 consisting of the amino acid sequence shown by SEQ ID NO: 10;
   (d) a light chain variable region comprising a CDR1 consisting of the amino acid sequence shown by SEQ ID NO: 23, a CDR2 consisting of the amino acid sequence shown by SEQ ID NO: 24 and a CDR3 consisting of the amino acid sequence shown by SEQ ID NO: 25;
   (e) a light chain variable region comprising the amino acid sequence shown by SEQ ID NO: 4; and
   (f) a light chain variable region comprising the amino acid sequence shown by SEQ ID NO: 22.
(6) An antibody or antigen-binding antibody fragment, which recognizes the same conformational epitope as the antibody or antigen-binding antibody fragment according to (5) above.
(7) The antibody or antigen-binding antibody fragment according to (1) above, comprising a heavy chain variable region of the following (g) or (h) and a light chain variable region of the following (i) or (j):
   (g) a heavy chain variable region comprising a CDR1 consisting of the amino acid sequence shown by SEQ ID NO: 15, a CDR2 consisting of the amino acid sequence shown by SEQ ID NO: 16 and a CDR3 consisting of the amino acid sequence shown by SEQ ID NO: 17;
   (h) a heavy chain variable region comprising an amino acid sequence shown by SEQ ID NO: 12;
   (i) a light chain variable region comprising a CDR1 consisting of the amino acid sequence shown by SEQ ID NO: 18, a CDR2 consisting of the amino acid sequence shown by SEQ ID NO: 19 and a CDR3 consisting of the amino acid sequence shown by SEQ ID NO: 20; and
   (j) a light chain variable region comprising an amino acid sequence shown by SEQ ID NO: 14.
(8) The antibody or antigen-binding antibody fragment according to any one of (1) to (7) above, wherein said antibody or antigen-binding antibody fragment is an IgG, Fab, Fab', F(ab')₂, single chain antibody, dsFv, (scFv)₂, or single domain antibody.
(9) The antibody or antigen-binding antibody fragment according to any one of (1) to (8) above, wherein said antibody is a human antibody or humanized antibody.
(10) The antibody or antigen-binding antibody fragment according to any one of (1) to (9) above, wherein said antibody or antigen-binding antibody fragment is chemically modified.
(11) A nucleic acid encoding the antibody or antigen-binding antibody fragment according to any one of (1) to (9) above.
(12) A nucleic acid encoding a heavy chain variable region comprising a CDR1 consisting of the amino acid sequence shown by SEQ ID NO: 5, a CDR2 consisting of the amino acid sequence shown by SEQ ID NO: 6 and a CDR3 consisting of the amino acid sequence shown by SEQ ID NO: 7.
(13) A vector comprising the nucleic acid according to (11) or (12) above.
(14) A host cell into which the vector according to (13) above has been introduced.
(15) A method for producing an anti-hepatitis C virus E2 protein antibody or antigen-binding antibody fragment thereof, comprising the steps of: culturing the host cell according to (14) above; and collecting the antibody or antigen-binding antibody fragment expressed.
(16) A medicament comprising the antibody or antigen-binding antibody fragment according to any one of (1) to (10) above, as an active ingredient.
(17) The medicament according to (16) above, which is an agent for treatment or prevention of hepatitis C.
(18) The medicament according to (17) above, which is an agent for prevention of hepatitis C in liver transplantation.
(19) A reagent for detecting hepatitis C virus, comprising the antibody or antigen-binding antibody fragment according to any one of (1) to (10) above.
(20) A method for treating or preventing hepatitis C virus infection, comprising administering the antibody or antigen-binding antibody fragment according to any one of (1) to (10) above, to a subject.

The present application includes the entire contents disclosed in Japanese Patent Application No. 2014-058936 from which the present application claims the benefit of priority.

### Advantageous Effects of Invention

An antibody or antigen-binding fragment thereof of the present invention has HCV infection inhibiting activity against HCV of a plurality of genotypes.

### Brief Description of Drawings

[Figure 1] Figure 1 is a diagram illustrating a nucleotide sequence (upper one) encoding a VH region displayed by the antibody phage e2d066 and the antibody phage e2d081, and an amino acid sequence (lower one) of the VH region. Each of FR1, FR2, FR3 and FR4 represents a framework region. Each of CDR1, CDR2 and CDR3 represents a complementarity-determining region (CDR).
[Figure 2] Figure 2 illustrates a nucleotide sequence (upper one) encoding a VL region displayed by the antibody phage e2d066 and an amino acid sequence (lower one) of the VL region.
[Figure 3] Figure 3 illustrates a nucleotide sequence (upper one) encoding a VH region displayed by the antibody phage e2d073 and the amino acid sequence (lower one) of the VH region.
[Figure 4] Figure 4 illustrates a nucleotide sequence (upper one) encoding a VL region displayed by the antibody phage e2d073 and the amino acid sequence (lower one) of the VL region.
[Figure 5] Figure 5 illustrates a nucleotide sequence (upper one) encoding a VL region displayed by the antibody phage e2d081 and an amino acid sequence (lower one) of the VL region.
[Figure 6] Figure 6 indicates HCV infection inhibiting activity of IgG antibodies of the present invention against HCVcc (J6/JFH-1 HCVcc) of genotype 2a.
[Figure 7] Figure 7 indicates HCV infection inhibiting activity of scFvs of the present invention against HCVcc (J6/JFH-1 HCVcc) of genotype 2a.
[Figure 8] Figure 8 indicates HCV infection inhibiting activity of the IgG antibodies of the present invention against HCVcc (S310/JFH-1 HCVcc) of genotype 3a.
[Figure 9] Figure 9 indicates HCV infection inhibiting activity of the scFvs of the present invention against HCVcc (S310/JFH-1 HCVcc) of genotype 3a.
[Figure 10] Figure 10 indicates HCV infection inhibiting activity of the IgG antibodies of the present invention against HCVcc (TH/JFH-1 HCVcc) of genotype 1b.
[Figure 11] Figure 11 indicates HCV infection inhibiting activity of the scFvs of the present invention against HCVcc (TH/JFH-1 HCVcc) of genotype 1b.
[Figure 12] Figure 12 indicates HCV infection inhibiting activity of the IgG antibodies of the present invention against HCVcc (H77/JFH-1 HCVcc) of genotype 1a.
[Figure 13] Figure 13 indicates HCV infection inhibiting activity of the scFvs of the present invention against HCVcc (H77/JFH-1 HCVcc) of genotype 1a.
[Figure 14] Figure 14 indicates binding property of the IgG antibodies of the present invention to native E2 protein (Figure 14A) and denatured E2 protein (Figure 14B) (epitope analysis). As the E2 protein, TH E2-Fc protein was used.
[Figure 15] Figure 15 indicates a binding property of the IgG antibodies of the present invention to a linear peptide (consisting of 12 amino acid residues in the amino acid sequence of the E2 protein) (epitope analysis). Figure 15A: e2d066 IgG, Figure 15B: e2d073 IgG, Figure 15C: e2d081 IgG, Figure 15D: MBL-HCV1 (IgG).
[Figure 16] Figure 16 indicates competitive inhibitory effect of other antibodies, against binding of biotinylated e2d066 IgG to E2 protein (TH E2Fc protein) (epitope analysis).
[Figure 17] Figure 17 indicates the competitive inhibitory effect of other antibodies, against binding of biotinylated e2d066 IgG to E2 protein (TH E2Fc protein) (epitope analysis).
[Figure 18] Figure 18 indicates binding properties of the antibodies of the present invention and other antibodies to the E2 protein of J6CF strain of genotype 2a (Figure 18A) or the E2 protein of TH strain of genotype 1b (Figure 18B).
[Figure 19] Figure 19 indicates infection spread-inhibitory effect by treatment with the e2d066 IgG after infection with HCVcc (J6/JFH-1 HCVcc) of genotype 2a.

### Description of Embodiments

The present invention relates to an anti-hepatitis C virus E2 protein antibody or an antigen-binding antibody fragment thereof having infection inhibiting activity against hepatitis C viruses (HCV) of a plurality of genotypes.

The present invention also relates to an anti-hepatitis C virus E2 protein antibody or an antigen-binding antibody fragment thereof having infection inhibiting activity against hepatitis C viruses (HCV) of a plurality of genotypes and exhibiting an escape mutant emergence suppressive property. The antibody or the antigen-binding antibody fragment of the present invention preferably exhibits the escape mutant emergence suppressive property against HCV of a plurality of genotypes.

Virus growth is generally suppressed once in the presence of a neutralizing antibody, but the growth may start again in some cases. Such a virus growing again has neutralization-resistance against the antibody, and the virus is called as an escape mutant. This regrowth is caused because mutation occurs in an envelope protein of the virus in the presence of the neutralizing antibody, and this leads to the inability of the neutralizing antibody to bind to the envelope protein. An antibody that does not cause mutation, even in the presence of a neutralizing antibody, in an epitope of a viral envelope protein to which the antibody bind, and thus is less likely to induce the emergence of an escape mutant, are known. Herein, such an antibody is an antibody exhibiting the "escape mutant emergence suppressive property". The antibody or the antigen-binding fragment of the present invention is less likely to cause mutation in an epitope of a viral envelope protein than conventional antibodies, and is less likely to induce the emergence of an escape mutant.

The antibody or the antigen-binding antibody fragment of the present invention preferably binds to a conformational epitope and exhibits escape mutant emergence suppressive property.

Ten virus proteins of hepatitis C virus (HCV) (core protein, E1 protein, E2 protein, p7 protein, NS2 protein, NS3 protein, NS4A protein, NS4B protein, NS5A protein and NS5B protein) are translated as a single precursor protein (polyprotein) in which the virus proteins are joined together in this order, and then ten mature virus proteins (core protein, E1 protein, E2 protein, p7 protein, NS2 protein, NS3 protein, NS4A protein, NS4B protein, NS5A protein and NS5B protein) are each produced from the precursor protein by cellular and viral proteases. The entity of hepatitis C virus (HCV) is present as a virus particle. The virus particle of HCV (HCV particle) contains an HCV genome inside a viral shell composed of structural proteins (core protein, E1 protein, E2 protein and p7 protein) of HCV. Herein, the term "hepatitis C virus (HCV)" and the "HCV particle" are used in the same meaning.

Herein, the position of an amino acid residue (also referred to as amino acid residue number or amino acid number) in a virus protein of hepatitis C virus (HCV) is indicated by a number counted based on the HCV precursor protein on the assumption that the N terminal amino acid (initiating methionine) of the precursor protein is the 1st. For example, the E2 protein of TH strain of genotype 1b starts from amino acid residue 384 and ends at amino acid residue 717.

A conformational epitope (also referred to as a structural epitope) is an epitope that depends on a higher-order structure of protein, which is composed of discontinuous amino acid residues away from one another in the primary structure, unlike a linear epitope composed of continuous amino acid residues in the primary structure. The discontinuous amino acid residues constituting the conformational epitope may be partially continuous in some cases. The ability of the antibody or the antigen-binding antibody fragment of the present invention to recognize a conformational epitope can be confirmed on the basis of the elimination or decrease of its binding to a denatured E2 protein with a reducing agent or by heating or the like.

The E2 protein refers to an envelope protein involved in the binding to a receptor (HCV receptor) present on the surface of a host cell. Hepatitis C virus (HCV) infects host cells via the HCV receptor. CD81 is identified as one of such HCV receptors, and has been reported as one of essential factors of HCV infection (Akazawa et al., J. Virol., 2007, vol. 81, pp. 5036-5045).

An antibody (full antibody) refers to a protein generally also called as immunoglobulin (Ig), and is a protein having a structure composed of one, two or more assembled heterotetramers each of which is a minimum unit having two heavy chains (H chains) and two light chains (L chains). Typically, each of the light chains is linked to the heavy chain through one disulfide covalent bond, and the number of disulfide bonds between the heavy chains vary in different immunoglobulin isotypes. Each of the heavy chains and the light chains also has an intrachain disulfide bond. Each of the heavy chains has a heavy chain variable region (hereinafter referred to as the VH region) followed by constant regions (CH regions: CH1, CH2, and CH3 for IgG). Each of the light chains has a light chain variable region (hereinafter referred to as the VL region) followed by one constant region (hereinafter referred to as the CL region). The CL region aligns with the CH1, which is the first constant region of the heavy chain, and the VL region aligns with the VH region. Variable regions (Fv) of an antibody have a region that is referred to as a complementarity-determining region (hereinafter abbreviated as CDR) and has specific variability to impart binding specificity to the antibody. The variable region includes, in addition to the CDRs, a relatively conserved region that is referred to as a framework region (hereinafter abbreviated as FR). The variable regions of whole heavy chains and light chains each contains four FRs (FR1, FR2, FR3 and FR4) linked via three CDRs (CDR1, CDR2 and CDR3), and the FR1, CDR1, FR2, CDR2, FR3, CDR3 and FR4 are arranged in this order from the amino terminus to the carboxyl terminus of the variable region. The full antibody may be, for example, IgG, IgM or IgA.

The anti-hepatitis C virus E2 protein antibody (full antibody) of the present invention has heavy chain variable regions and light chain variable regions that cause a specific binding to the hepatitis C virus E2 protein, and constant regions (CL, CH1 and Fc).

The antigen-binding antibody fragment of the present invention refers to a protein that comprises a subset of the components of the full antibody, and retains an antigen-binding property of the antibody to the hepatitis C virus E2 protein. The antigen-binding antibody fragment of the present invention still has infection inhibiting activity against HCV. Advantageously, an antigen-binding antibody fragment has a smaller molecular weight than a full antibody (an immunoglobulin molecule) and hence generally is excellent in cell permeability, and also is comparatively inexpensive of production because it can be produced using a microorganism.

Examples of the antigen-binding antibody fragment include, but are not limited to, an Fab (an antibody fragment composed of VH, VL, CL and CH1; obtained by treating an IgG antibody with papain), an F(ab)'₂ (an antibody fragment composed of two Fab fragments crosslinked by disulfide bond at a hinge region; obtained by treating an IgG antibody with pepsin), an Fab', an Fv, a single chain antibody (such as an scFv), a dsFv, a (scFv)₂, a diabody and a minibody. The single chain antibody includes an scFv, and the scFv (single chain Fv) typically refers to an antibody in which variable regions (Fv), i.e., a VH region and a VL region, are linked via a linker into single chain. The dsFv (disulfide-stabilized Fv) refers to an antibody in which a VH region and a VL region of an Fv are linked together through an intrachain disulfide bond introduced into a framework region for stabilization.

The antibody or the antigen-binding antibody fragment of the present invention may be, for example, chemically synthesized (a synthesized antibody), produced by genetic engineering techniques (a recombinant antibody), a human antibody, a humanized antibody, prepared by linking heavy chain and light chain variable regions via a linker or a disulfide bond introduced therein (e.g., a single chain antibody or a diabody), a multispecific antibody (such as a bispecific antibody), a chimeric antibody or the like. The recombinant antibody typically refers to an antibody or an antigen-binding antibody fragment expressed by using a recombinant expression vector transfected into a host cell. The antibody or the antigen-binding antibody fragment of the present invention may be a polyclonal antibody but is preferably a monoclonal antibody.

In one embodiment, the antibody or the antigen-binding antibody fragment of the present invention contains a VH region comprising a CDR1 consisting of the amino acid sequence shown by SEQ ID NO: 5, a CDR2 consisting of the amino acid sequence shown by SEQ ID NO: 6, and a CDR3 consisting of the amino acid sequence shown by SEQ ID NO: 7. The VH region preferably comprises the amino acid sequence shown by SEQ ID NO: 2.

The antibody or the antigen-binding antibody fragment of the present invention may be a single domain antibody containing a VH region but not comprising a VL region (also referred to as an immunoglobulin VH region fragment). This is, for example, an antibody fragment consisting of a VH region alone. An example thereof includes a single domain antibody containing a VH region comprising a CDR1 the amino acid sequence shown by SEQ ID NO: 5, a CDR2 having the amino acid sequence shown by SEQ ID NO: 6 and a CDR3 having the amino acid sequence shown by SEQ ID NO: 7, but not containing a VL region, such as an antibody fragment consisting of the VH region alone.

Alternatively, the antibody or the antigen-binding antibody fragment of the present invention may be an antibody containing a VH region comprising a CDR1 having the amino acid sequence shown by SEQ ID NO: 5, a CDR2 having the amino acid sequence shown by SEQ ID NO: 6 and a CDR3 having the amino acid sequence shown by SEQ ID NO: 7, and also containing a VL region. The VL region (the light chain variable region) is not especially limited as long as it does not substantially affect the specific binding property to HCV, and may have a sequence derived from an any antibody (preferably derived from a human antibody).

A preferable example of the VL region contained in the antibody or the antigen-binding antibody fragment of the present invention includes a VL region comprising a CDR1 having the amino acid sequence shown by SEQ ID NO: 8, a CDR2 having the amino acid sequence shown by SEQ ID NO: 9 and a CDR3 having the amino acid sequence shown by SEQ ID NO: 10. An example of this VL region includes one comprising the amino acid sequence shown by SEQ ID NO: 4.

A preferred another example of the VL region includes a VL region comprising a CDR1 consisting of the amino acid sequence shown by SEQ ID NO: 23, a CDR2 consisting of the amino acid sequence shown by SEQ ID NO: 24 and a CDR3 consisting of the amino acid sequence shown by SEQ ID NO: 25. An example of this VL region includes one containing an amino acid sequence shown by SEQ ID NO: 22.

A specific example of the antibody or the antigen-binding antibody fragment of the present invention includes one containing a VH region comprising the amino acid sequence shown by SEQ ID NO: 2 and a VL region comprising the amino acid sequence shown by SEQ ID NO: 4. Another specific example of the antibody or the antigen-binding antibody fragment of the present invention includes one containing a VH region comprising the amino acid sequence shown by SEQ ID NO: 2 and a VL region comprising the amino acid sequence shown by SEQ ID NO: 22.

The antibody or the antigen-binding antibody fragment of the present invention may be an antibody or antigen-binding antibody fragment that recognizes the same conformational epitope as the above-described antibody or antigen-binding antibody fragment. For example, the antibody or the antigen-binding antibody fragment of the present invention includes one that recognizes the same conformational epitope as the antibody or the antigen-binding antibody fragment, which contains a VH region comprising a CDR1 having the amino acid sequence shown by SEQ ID NO: 5, a CDR2 having the amino acid sequence shown by SEQ ID NO: 6 and a CDR3 having the amino acid sequence shown by SEQ ID NO: 7 (preferably a VH region comprising the amino acid sequence shown by SEQ ID NO: 2); and also contains a VL region comprising a CDR1 having the amino acid sequence shown by SEQ ID NO: 8, a CDR2 having the amino acid sequence shown by SEQ ID NO: 9 and a CDR3 having the amino acid sequence shown by SEQ ID NO: 10 (preferably a VL region comprising the amino acid sequence shown by SEQ ID NO: 4), or a VL region comprising a CDR1 consisting of the amino acid sequence shown by SEQ ID NO: 23, a CDR2 consisting of the amino acid sequence shown by SEQ ID NO: 24 and a CDR3 consisting of the amino acid sequence shown by SEQ ID NO: 25 (preferably a VL region comprising the amino acid sequence shown by SEQ ID NO: 22).

The antibody or the antigen-binding antibody fragment of the present invention described so far has infection inhibiting activity against hepatitis C virus (HCV) of a plurality of genotypes (preferably including genotypes 1a, 1b, 2a and 3a), and exhibits the escape mutant emergence suppressive property. In a preferred embodiment, the antibody or the antigen-binding antibody fragment of the present invention has a high infection inhibiting activity against HCV of two or more genotypes, preferably of genotype 1a, genotype 1b, genotype 2a and genotype 3a.

In another embodiment, the antibody or the antigen-binding antibody fragment of the present invention may contain a VH region comprising a CDR1 having the amino acid sequence shown by SEQ ID NO: 15, a CDR2 having the amino acid sequence shown by SEQ ID NO: 16 and a CDR3 having the amino acid sequence shown by SEQ ID NO: 17, and a VL region comprising a CDR1 having the amino acid sequence shown by SEQ ID NO: 18, a CDR2 having the amino acid sequence shown by SEQ ID NO: 19 and a CDR3 having the amino acid sequence shown by SEQ ID No: 20. This VH region preferably comprises the amino acid sequence shown by SEQ ID NO: 12. This VL region preferably comprises the amino acid sequence shown by SEQ ID NO: 14. These antibodies have a high infection inhibiting activity against HCV of a plurality of genotypes. A specific example of such an antibody or antigen-binding antibody fragment of the present invention includes one containing a VH region comprising the amino acid sequence shown by SEQ ID NO: 12 and a VL region comprising the amino acid sequence shown by SEQ ID NO: 14. Such an antibody or antigen-binding antibody fragment of the present invention has an infection inhibiting activity against hepatitis C virus (HCV) of a plurality of genotypes (including genotypes 1b and 2a).

A CDR is a region for determining the specificity of the antibody. A region excluding the CDR (such as, in a full antibody, each FR of the heavy chain and the light chain regions and each constant region) of the antibody and the antigen-binding antibody fragment of the present invention is not especially limited in terms of the amino acid sequence as long as the specific binding property to HCV is not substantially affected thereby, and may have a sequence derived from another antibody. Here, another antibody includes also an antibody derived from an organism excluding a human, but is preferably derived from a human from the viewpoint of reduction of adverse reaction. That is, the antibody or the antigen-binding antibody fragment of the present invention preferably contains, as a region excluding the CDR, a corresponding amino acid sequence derived from a human antibody. Since the CDR of the antibody or the antigen-binding antibody fragment of the present invention is derived from a human antibody, the other region contained in the antibody or the antigen-binding antibody fragment of the present invention is more preferably derived from a human antibody, because such an antibody or antigen-binding antibody fragment is composed of only amino acid sequences derived from human antibodies. The light chain of the antibody or the antigen-binding antibody fragment of the present invention may contain a CL region (constant region) comprising an amino acid sequence shown by any one of SEQ ID NOS: 38 to 40.

The antibody or the antigen-binding antibody fragment of the present invention may belong to any class of immunoglobulin molecules, such as IgG, IgE, IgM, IgA, IgD or IgY, or any subclass, such as IgG1, IgG2, IgG3, IgG4, IgA1 or IgA2, and preferably belongs to IgG. The light chain of the antibody of the present invention may be a λ chain or a κ chain.

The antibody of the present invention can be a full antibody containing the heavy chain comprising the above-described heavy chain variable region (VH region) and the light chain comprising the above-described light chain variable region (VL region) as well as the constant regions. Preferred examples of the antibody of the present invention being to a full antibody include an antibody containing a heavy chain consisting of the amino acid sequence shown by SEQ ID NO: 26 and a light chain consisting of the amino acid sequence shown by SEQ ID NO: 27 (corresponding to e2d066 IgG described in Examples below), an antibody containing a heavy chain consisting of the amino acid sequence shown by SEQ ID NO: 30 and a light chain consisting of the amino acid sequence shown by SEQ ID NO: 31 (corresponding to e2d081 IgG described in the Examples below) and an antibody containing a heavy chain consisting of the amino acid sequence shown by SEQ ID NO: 28 and a light chain consisting of the amino acid sequence shown by SEQ ID NO: 29 (corresponding to e2d073 IgG described in the examples below).

The antibody or the antigen-binding antibody fragment of the present invention also includes an antibody recognizing the same conformational epitope as the antibody containing the heavy chain consisting of the amino acid sequence shown by SEQ ID NO: 26 and the light chain consisting of the amino acid sequence shown by SEQ ID NO: 27 (corresponding to e2d066 IgG described in the Examples below) or the antibody containing the heavy chain consisting of the amino acid sequence shown by SEQ ID NO: 30 and the light chain consisting of the amino acid sequence shown by SEQ ID NO: 31 (corresponding to e2d081 IgG described in the Examples below).

The antigen-binding antibody fragment of the present invention is also preferably a single chain antibody containing the above-described heavy chain variable region (VH region) and the above-described light chain variable region (VL region), such as a scFv. A single chain antibody is also called a one-chain antibody, and refers to an antibody fragment that contains at least a VH region and a VL region (and may further contain a CL region) linked into a single chain. The scFv is typically an antibody in which one VH region and one VL region composed of an Fv are linked via a linker. Herein, however, one containing a CL region in addition to a VH region and a VL region is also called scFv. Specific examples of the scFv of the present invention include an antibody fragment having the amino acid sequence shown by SEQ ID NO: 32 (corresponding to e2d066 scFv described in the Examples below), an antibody fragment having the amino acid sequence shown by SEQ ID NO: 34 (corresponding to e2d081 scFv described in the Examples below) and an antibody fragment having the amino acid sequence shown by SEQ ID NO: 33 (corresponding to e2d071 scFv described in the examples below), all of which contains a VH region, a linker, a VL region and a CL region (VLCL region).

The antibody or the antigen-binding antibody fragment of the present invention may contain a sequence not derived from an amino acid sequence of a human immunoglobulin (such as an artificially mutated sequence) as long as it does not have immunogenicity in a human body. The antibody or the antigen-binding antibody fragment of the present invention may be one obtained by substituting, by another amino acid, an amino acid contained in the FR or the constant region in the above-described amino acid sequences constituting the full antibody. Such amino acid substitution is substitution of preferably 1 to 5 amino acids, and more preferably 1 or 2 amino acids. Such an antibody containing the amino acid substitution is preferably functionally equivalent to an antibody before the substitution. Therefore, the amino acid substitution is preferably conservative amino acid substitution, which is substitution between amino acids similar to each other in properties of charge, side chains, polarity, aromaticity or the like. Amino acids similar in properties can be classified as, for example, basic amino acids (arginine, lysine and histidine), acidic amino acids (aspartic acid and glutamic acid), non-charged polar amino acids (glycine, asparagine, glutamine, serine, threonine, cysteine and tyrosine), nonpolar amino acids (leucine, isoleucine, alanine, valine, proline, phenyl alanine, tryptophan and methionine), branched chain amino acids (threonine, valine and isoleucine), and aromatic amino acids (phenylalanine, tyrosine, tryptophan and histidine). A disulfide bond can be introduced by the amino acid substitution of an amino acid residue in a framework region with cysteine to produce a stabilized antibody or antigen-binding antibody fragment (such as a dsFv), and such an antibody or antigen-binding antibody fragment is also included in the scope of the present invention. Here, the term "functionally equivalent" means that an antibody having an amino acid substitution has a similar biological or biochemical activity, particularly, HCV infection inhibiting activity, to an antibody before the substitution.

The antibody or the antigen-binding antibody fragment of the present invention can be produced, for example, by the following method:

A nucleic acid (gene) encoding the antibody or the antibody fragment is inserted into one or a plurality of suitable vectors, the resultant is introduced into a host cell (for example, a mammal cell, a yeast cell, an insect cell or the like), and a protein is produced therefrom, by using genetic engineering techniques (P. J. Delves, ANTIBODY PRODUCTION ESSENTIAL TECHNIQUES, 1997, WILEY; P. Shepherd and C. Dean, Monoclonal Antibodies, 2000, OXFORD UNIVERSITY PRESS; J. W. Goding, Monoclonal Antibodies: principles and practice, 1993, ACADEMIC PRESS). Incidentally, a DNA encoding the antibody or antibody fragment can be produced by genetic engineering techniques (Sambrook et al., Molecular Cloning A Laboratory Manual, 1989, Cold Spring Harbor Laboratory Press) or using a DNA synthesizer.

Specifically, when, for example, a full antibody is to be produced, a DNA encoding a variable region of the antibody and a DNA encoding a constant region of a human IgG may be linked each other, and inserted into an expression vector. Alternatively, a DNA encoding a variable region of the antibody may be inserted into an expression vector containing a DNA encoding a constant region of the antibody. These DNAs are preferably inserted under the control of an expression control region in the expression vector, such as an enhancer and a promoter. The thus obtained expression vector is used for transforming a host cell, and the resultant host cell can be cultured to produce the antibody. Each of these expression vectors may be used alone or in combination of two or more.

The resultant DNA can be linked to a DNA encoding a constant region of a human antibody, and the resultant DNA is inserted into an expression vector, transferred into a host cell for expression to obtain a full antibody (see European Patent Publication No. EP239400, and International Publication No. WO1996/02376). FRs that are linked via CDRs in a human antibody and can be selected for use herein do not obstruct a formation of an antigen-binding site by the complementarity-determining regions, which has a similar structure to an antigen-binding site of full antibody e2d066 or e2d081 as described in the Examples below. Alternatively, the FR may be substituted with any of various human antibody-derived FRs (see International Publication No. WO1999/51743).

The antigen-binding antibody fragment of the present invention can be also produced by a known method such as enzymatic digestion of a full antibody or the genetic engineering techniques. The antigen-binding antibody fragment of the present invention may be fused with another protein as long as its binding activity to the antigen is retained. For example, an antigen-binding antibody fragment such as a scFv or a Fab can be produced by linking an H chain fragment comprising a VH and an L chain fragment comprising a VL to each other via a suitable linker, and expressing the resultant in a host cell, or by associating, in a cultured cell, an H chain protein and an L chain protein expressed from different vectors.

For example, the antigen-binding antibody fragment of the present invention can be produced also by transforming *E. coli* with a phagemid in which VH and VL are inserted by the phage display method. Specifically, the antigen-binding antibody fragment of the present invention can be produced by a two-step cloning method in which VH and VL are each amplified by PCR, the VL is inserted into a phagemid vector and transformed into *E. coli* and a VL-positive phagemid is purified, and then, the VH is inserted into the VL-positive phagemid and expressed in *E. coli.* Alternatively, the antigen-binding antibody fragment of the present invention can be produced also by a VL-VH assembly method in which VH and VL are each amplified by the PCR and then linked to each other, and the resulting construct is inserted into a phagemid and transformed into *E. coli.*

Preparation of scFv is performed, for example, as follows. An scFv can be produced by inserting a DNA encoding a linker between isolated cDNAs each encoding VH and VL (or between cDNAs each encoding VH and VLCL), and inserting the resulting recombinant DNA encoding a single chain antibody into an expression vector, and introducing the resulting vector into a host cell to express it.

In the scFv of the present invention, a linker connecting a VH region and a VL region is not especially limited as long as it does not inhibit the expression of the VH and VL linked to both ends of the linker or the binding to the VH and VL. The length of the linker peptide is generally 1 to 100 amino acids, preferably 1 to 50 amino acids, more preferably 1 to 30 amino acids, and particularly preferably 12 to 18 amino acids (for example, 15 amino acids). In the present invention, a polypeptide consisting of 15 amino acids: (GGGGS)₃ (SEQ ID NO: 35) (wherein G represents glycine and S represents serine) (Kim et al., Protein Engineering Design and Selection, 2007, vol. 20(9), pp. 425-432) can be preferably used as a linker. For preparing a DNA encoding a variable region, for example, a nucleotide sequence designed to link CDRs to FRs of a human antibody can be synthesized by the PCR from several oligonucleotides prepared to have overlap portions at ends.

For selecting an FR, for example, the following two methods may be employed. In the first method, a human antibody frame of which three-dimensional structure has been revealed, such as NEWM or REI, is used (Riechmann L. et al., Nature, 1988, vol. 332, pp. 323-327; Tempst, PR et al., Protein Engineering, 1994, vol. 7, pp. 1501-1507; Ellis JH. et al., J. Immunol., 1995, vol. 155, pp. 925-937). In the second method, amino acids most commonly used in FRs of human antibodies are selected (Sato K. et al., Mol Immunol., 1994, vol. 31, pp. 371-381; Kobinger F. et al., Protein Engineering, 1993, vol. 6, pp. 971-980; Kettleborough CA. et al., Protein Engineering, 1991, vol. 4, pp. 773-783). In the present invention, any of these methods can be employed.

Besides, as a well-known method to those skilled in the art for preparing a polypeptide functionally equivalent to a given polypeptide, there is a method comprising introducing a mutation into a polypeptide. For example, those skilled in the art can employ a site-directed mutagenesis method (Hashimoto-Gotoh T. et al., Gene, 1995, vol. 152, pp. 271-275; Zoller MJ. and Smith M., Methods Enzymol., 1983, vol. 100, pp. 468-500; Kramer W. et al., Nucleic Acids Res., 1984, vol. 12, pp. 9441-9456; Kramer W. and Fritz HJ., Methods Enzymol., 1987, vol. 154, pp. 350-367; Kunkel TA., Proc. Natl. Acad. Sci. USA., 1985, vol. 82, pp. 488-492; Kunkel, Methods Enzymol., 1988, vol. 85, pp. 2763-2766) or the like to introduce a mutation into a given nucleic acid encoding the antibody or the antigen-binding antibody fragment of the present invention as appropriate, to prepare an antibody or an antigen-binding antibody fragment functionally equivalent to the antibody or the antigen-binding antibody fragment. A kit for carrying out site-directed mutagenesis is commercially available and can be used for the production of the antibody and the antigen-binding antibody fragment of the present invention.

The antibody and the antigen-binding antibody fragment of the present invention may be chemically modified (subjected to chemical modification). In other words, the antibody and antigen-binding antibody fragment of the present invention encompasses a chemically modified antibody and antigen-binding antibody fragment. The chemical modification may be arbitrary as long as the antibody and the antigen-binding antibody fragment of the present invention have a specific binding property to HCV. The chemical modification may be, for example, functional modification or labeling. Examples of such chemical modification include e.g., glycosylation, acetylation, formylation, amidation, phosphorylation and PEGylation (polyethylene glycol). Alternatively, the chemical modification can be modification with, for example, a fluorescent dye (FITC, rhodamine, Texas Red, Cy3 or Cy5), a fluorescent protein (such as PE, APC or GFP), an enzyme (such as horseradish peroxidase, alkaline phosphatase or glucose oxidase), or biotin or (strept)avidin. These modifications can be performed by any method known in the art.

The present invention also provides a nucleic acid encoding the antibody or the antigen-binding antibody fragment of the present invention. The nucleic acid encoding the antibody or the antigen-binding antibody fragment of the present invention may, for example, comprise a nucleotide sequence encoding a VH region shown by SEQ ID NO: 1 and a nucleotide sequence encoding a VL region shown by SEQ ID NO: 3, and may further comprise a nucleotide sequence encoding a CL region consisting of the amino acid sequence shown by SEQ ID NO: 38. Alternatively, the nucleic acid encoding the antibody or the antigen-binding antibody fragment of the present invention may comprise a nucleotide sequence encoding a VH region shown by SEQ ID NO: 11 and a nucleotide sequence encoding a VL region shown by SEQ ID NO: 13, and may further comprise a nucleotide sequence encoding a CL region consisting of the amino acid sequence shown by SEQ ID NO: 39. Alternatively, the nucleic acid encoding the antibody or the antigen-binding antibody fragment of the present invention may comprise a nucleotide sequence encoding a VH region shown by SEQ ID NO: 1 and a nucleotide sequence encoding a VL region shown by SEQ ID NO: 21, and may further comprise a nucleotide sequence encoding a CL region consisting of the amino acid sequence shown by SEQ ID NO: 40. The present invention also provide a nucleic acid encoding a heavy chain variable region comprising a CDR1 consisting of the amino acid sequence shown by SEQ ID NO: 5, a CDR2 consisting of the amino acid sequence shown by SEQ ID NO: 6 and a CDR3 consisting of the amino acid sequence shown by SEQ ID NO: 7. The present invention further provides a vector containing a nucleic acid encoding the antibody or the antigen-binding antibody fragment of the present invention, and a host cell into which the vector has been introduced.

The host cell refers to a target cell into which a vector is to be introduced, and may be, for example, a bacterial cell, a mammal cell (such as a human cell), a fungal cell (such as a yeast cell) or an insect cell. The host cell into which a vector has been introduced can be changed, due to subsequent mutation or environmental influence, to a cell having a trait (phenotype) that is not completely the same as that of the cell at the time of introduction of the vector, but such a cell progeny is also included into the "host cell".

The present invention also provides a method for producing an anti-hepatitis C virus E2 protein antibody or an antigen-binding antibody fragment antibody thereof comprising the steps of: culturing a host cell into which a vector comprising a nucleic acid encoding the antibody or the antigen-binding antibody fragment of the present invention has been introduced; and collecting the antibody or the antigen-binding antibody fragment antibody expressed via the culture. More specifically, the antibody or the antigen-binding antibody fragment of interest can be collected from a culture obtained by culturing the host cell under conditions where the expression of the antibody or the antigen-binding antibody fragment of the present invention is induced from the vector. The antibody or the antigen-binding antibody fragment thus collected can be purified by a known method.

Now, a method for producing a humanized IgG antibody will be described as a more specific example of the method for producing the antibody of the present invention. However, other types of antibodies can also be obtained in a similar way to the method.

An expression vector to be used in the production of a humanized IgG antibody (a humanized antibody expression vector) is an expression vector into which genes encoding a heavy chain constant region of a human antibody and a light chain constant region of a human antibody are incorporated. The vector can be constructed by cloning genes encoding the heavy chain constant region of the human antibody and the light chain constant region of the human antibody into an expression vector.

The constant regions of the human antibody may be heavy chain and light chain constant regions of any human antibody, and examples thereof include a constant region of an IgG1 subclass of a heavy chain of a human antibody, and a constant region of κ class of a light chain of a human antibody. The genes encoding the heavy chain and light chain constant regions of the human antibody may be chromosomal DNAs containing exons and introns, or cDNAs.

As an animal cell expression vector used for producing the humanized antibody expression vector any vector may be used as long as a gene encoding a constant region of a human antibody can be incorporated therein and expressed therefrom. Examples of the animal cell expression vector include pAGE107 (Cytotechnology, 3, 133 (1990)), pAGE103 (J. Biochem., 101, 1307 (1987)), pHSG274 (Gene, 27, 223 (1984)), pKCR (Proc. Natl. Acad. Sci. U.S.A., 78, 1527 (1981)), and pSG1βd2-4 (Cytotechnology, 4, 173 (1990)). Examples of a promoter or enhancer used in the animal cell expression vector include the initial promoter and enhancer of SV40 (J. Biochem., 101, 1307 (1987)), and the LTR of the Moloney murine leukemia virus (Biochem. Biophys. Res. Commun., 149, 960 (1987)), and the promoter (Cell, 41, 479 (1985)) and the enhancer (Cell, 33, 717 (1983)) of an immunoglobulin heavy chain.

The humanized antibody expression vector may be either of a type in which the antibody heavy chain and light chain are present on separate vectors, and a type in which the chains are present on the same vector (hereinafter referred to as the tandem type), but the humanized antibody expression vector of the tandem type is preferable from the viewpoint of ease of construction of the humanized antibody expression vector, ease of introduction of the humanized antibody expression vector into an animal cell, and balanced expression levels of the antibody heavy chain and light chain in animal cells (J. Immunol. Methods, 167, 271 (1994)). Examples of the humanized antibody expression vector of the tandem type include pKANTEX93 (Mol. Immnol., 37, 1035 (2000)) and pEE18 (Hybridoma, 17, 559 (1998)).

The thus constructed humanized antibody expression vector can be used for expression of a humanized chimeric antibody and a humanized CDR-transplanted antibody in an animal cell.

Stable production of the humanized antibody can be achieved by introducing the humanized antibody expression vector into an appropriate animal cell to obtain a transformant strain stably producing a humanized chimeric antibody or a humanized CDR-transplanted antibody (both of which will be hereinafter collectively referred to as the humanized antibody). As a method for introducing the humanized antibody expression vector into an animal cell, for example, electroporation method (JP Patent Publication (Kokai) No. 2-257891 A (1990); Cytotechnology, 3, 133 (1990)) may be employed. As the animal cell into which the humanized antibody expression vector will be introduced, any animal cell may be used as long as the animal cell can produce a humanized antibody. Specific examples of the animal cell include an NSO cell and an SP2/0 cell that are mouse myeloma cells, a CHO/dhfr- cell and CHO/DG44 cell that are Chinese hamster ovary cells, a rat myeloma YB2/0 cell and IR983F cell, a BHK cell derived from a Syrian hamster kidney, and a Namalwa cell that is a human myeloma cell, and preferably a Chinese hamster ovary cell CHO/DG44 and a rat myeloma YB2/0 cell.

After the introduction of the humanized antibody expression vector, the transformant strain stably producing the humanized antibody can be selected with an animal cell culture medium containing a drug such as G418 sulfate (hereinafter referred to as G418; Sigma-Aldrich) or puromycin (Sigma-Aldrich, P8833), in accordance with a method disclosed in JP Patent Publication (Kokai) No. 2-257891 A (1990). As the animal cell culture medium, for example, RPMI 1640 medium (Nissui Pharmaceutical Co., Ltd.), GIT medium (Nihon Pharmaceutical Co., Ltd.), EX-CELL302 medium (SAFC Biosciences), IMDM medium (Thermo Fisher Sicentific), Hybridoma-SFM medium (Thermo Fisher Scientific), EX-CELL CD CHO Fusion (Nichirei Biosciences Inc.; 14365C), or a medium supplemented with any of various additives such as a fetal calf serum (hereinafter abbreviated as FCS) to any of these media can be used. The obtained transformant strain can be cultured in the medium to produce the humanized antibody and accumulate it in a culture supernatant. The amount of the produced humanized antibody in the culture supernatant and the antigen binding activity of the humanized antibody are determined by enzyme-linked immunosorbent assay (hereinafter abbreviated as the ELISA method; Antibodies: A Laboratory Manual, Cold Spring Harbor Laboratory, Chapter 14, 1998, or Monoclonal Antibodies: Principles and Practice, Academic Press Limited, 1996) or the like. The amount of the humanized antibody production of the transformant strain can be increased in accordance with the method disclosed in JP Patent Publication (Kokai) No. 2-257891 A (1990) using DHFR gene amplification system or the like.

The humanized antibody can be purified from the culture supernatant of the transformant strain using a Protein A column (Antibodies: A Laboratory Manual, Cold Spring Harbor Laboratory, Chapter 8, 1988, or Monoclonal Antibodies: Principles and Practice, Academic Press Limited, 1996). Alternatively, for purifying the humanized antibody, any of purification methods generally used for purifying a protein can be employed. For example, gel filtration, ion exchange chromatography, ultrafiltration and the like can be appropriately combined for the purification. The molecular weight of the heavy chain, the light chain or the whole antibody molecule of the purified humanized antibody can be determined by polyacrylamide gel electrophoresis (hereinafter abbreviated as SDS-PAGE; Nature, 227, 680 (1970)), Western blotting (Antibodies: A Laboratory Manual, Cold Spring Harbor Laboratory, Chapter 12, 1988, or Monoclonal Antibodies: Principles and Practice, Academic Press Limited, 1996) or the like.

The method for producing the antibody using an animal cell as a host has been illustrated so far. However, as described above, the antibody can be produced also in yeast, an insect cell, a plant cell or a non-human animal individual or plant individual by similar methods to the method with an animal cell.

The antibody or the antigen-binding antibody fragment of the present invention has high infection inhibiting activity against HCV of a plurality of genotypes. The antibody or the antigen-binding antibody fragment of the present invention has infection inhibiting activity against HCV of preferably two or more genotypes, such as HCV of two or more genotypes selected from the group consisting of genotypes 1a, 1b, 2a, 3a, 4a, 5a and 6a, and particularly, HCV of two or more genotypes including genotypes 1b and 2a. Examples of HCV of genotype 1a include the H77 strain, the HCV-1 strain, the HCV-H strain and the J1 strain, examples of HCV of genotype 1b include the TH strain, the con1 strain, the HCV-J strain, the JT strain and the BK strain, examples of HCV of genotype 2a include the J6CF strain, the JFH-1 strain, the JFH-2 strain and the JCH-1 strain, examples of HCV of genotype 3a include the S310 strain and the E-b1 strain, examples of HCV of genotype 4a include the ED43, examples of HCV of genotype 5a include the EUH1480, and examples of HCV of genotype 6a includes EUK2.

In the context of the present invention, the term "HCV infection" refers to processes of a HCV particle first binding to a cell surface of a host cell, growing in the host cell and being released out of the cell. Accordingly, the term "HCV infection inhibiting activity" used in the context of the present invention means an activity to inhibit the progress of HCV infection in a body or a cell population by inhibiting or suppressing at least one of the above-mentioned processes of the HCV infection. The HCV infection inhibiting activity of the antibody or the antigen-binding antibody fragment of the present invention is exhibited preferably by inhibiting a pathway through which HCV binds to a virus receptor present on the cell surface of a host cell and/or a pathway through which the HCV genome enters the host cell. It is noted that the term "HCV particle" refers to HCV itself or an HCV-like construct composed of HCV envelope proteins and an HCV genome packaged therewith.

The HCV infection inhibiting activity of the antibody or the antigen-binding antibody fragment of the present invention can be assayed by, for example, measuring inhibiting activity exhibited when an HCV sensitive cell (such as a Huh-7 cell) is infected with a chimeric HCV particle infectious to a cultured cell. The chimeric HCV particle infectious to a cultured cell can be prepared by, for example, causing a recombination of coding sequences for structural genes: the Core protein, the E1 protein, the E2 protein and the p7 protein, of an HCV genome of a given genotype, with the structural genes of the HCV JFH-1 strain of genotype 2a that highly efficiently autonomously replicates in a cultured cell, introducing it into cultured cells and culturing the cells to produce HCV particles. This chimeric HCV particle can be used to assay the infection inhibiting activity against HCV of various genotypes. The underlying technique of this assay is described in, for example, Wakita et al., Nature Medicine, 2005, vol. 11, pp. 791-796; Lindenbach et al., Science, 2005, vol. 309, pp. 623-6262; and Pietschmann et al., Proce. Natl. Acad. Sci. U.S.A., 2007, vol. 103, pp. 7408-7413.

Specifically, the HCV infection inhibiting activity can be assayed by, for example, a method comprising culturing a mixture of an HCV particle and an HCV sensitive cell (such as a Huh-7 cell) or an HCV infected cell obtained by infection with an HCV particle in the presence and in the absence of an antibody to be assayed for the HCV infection inhibiting activity, and detecting an HCV genome RNA or an HCV particle released into the resulting culture. The detection can be generally performed by measuring (for example, quantifying) an amount of the HCV genome RNA or the HCV particle in the culture. In this assay, if there is, in the culture, no HCV genome RNA or HCV particle, or a lower level of HCV genome RNA or HCV particles compared with those in the absence of the antibody, the antibody is evaluated as having the infection inhibiting activity against the HCV.

More specifically, for example, the following method can be employed for assaying the HCV infection inhibiting activity. First, a sample containing an antibody and an HCV particle are mixed and reacted at 37°C for 1 hour to prepare a mixed sample. Next, 50 µL of the mixed sample is added to Huh-7 cells cultured in a 96-well plate at 5 x 10³ cells/well on the previous day, and the resultant is cultured at 37°C for 2.5 hours. The culture medium is then removed, and the cells are washed with PBS, and a fresh medium is added thereto to continue the culture. After 48 hours, the culture medium is removed, the cells are washed once with PBS, 100 µL of ISOGEN (Nippon Gene Co., Ltd.) is added thereto and an RNA is prepared from the cells. After quantifying the RNA, an amount of HCV genome RNA is measured by quantitative RT-PCR. The detection of the HCV genome RNA by the quantitative RT-PCR may be performed by detecting a 5' non-coding region RNA of the HCV genome RNA in accordance with a method of Takeuchi et al., (Gastroenterology, 1999, vol. 116, pp. 636-642). On the basis of the amount of the HCV genome RNA determined by this measurement, the HCV infection inhibiting activity can be calculated.

As another assay method for the HCV infection inhibiting activity, the following method may be employed. A sample containing an antibody and an HCV particle is mixed, and reacted at room temperature for 30 minutes to prepare a mixed sample. Next, 100 µL of the mixed sample is added to Huh-7 cells cultured in a 48-well plate at 2 x 10⁴ cells/well on the previous day, and the resultant is cultured at 37°C for 3 hours. The culture medium is then removed, and the cells are washed with PBS, and a fresh medium is added thereto to continue the culture. After 72 hours, the culture medium is removed, the cells are washed with PBS, and 100 µL/well of Passive Lysis Buffer (Promega) is added to prepare a cell lysate. HCV core protein contained in the collected cell lysate is quantified by Lumipulse G1200 (Fujirebio Inc., Ortho Clinical Diagnostics). On the basis of a molar concentration of the HCV core protein determined by this measurement, the HCV infection inhibiting activity can be calculated.

In one embodiment, the antibody or the antigen-binding antibody fragment of the present invention exhibits the HCV escape mutant emergence suppressive property. The escape mutant refers to a virus mutant that has acquired a resistant to an inhibiting activity of an infection inhibiting antibody through mutation of an HCV virus genome. The escape mutant emergence suppressive property refers to a property of lowering an induction rate of the emergence of the escape mutant, or of not inducing the emergence of the escape mutant.

The ability of the antibody or the antigen-binding antibody fragment of the present invention to exhibit the escape mutant emergence suppressive property can be evaluated in accordance with a method of Meital et al., (Proc. Natl. Acad. Sci. U.S.A., 2008, vol. 105, pp. 19450-19455) by repeating the steps of infecting cells with HCV particles in the presence of an antibody to be evaluated, collecting a culture supernatant and then mixing the culture supernatant with the antibody for infection of a non-infected cell; and then measuring infectious titer of HCV finally contained in the culture supernatant (evaluation by repeated subculture infection). Specifically, for example, a chimeric HCV particle between HCV of any of the genotypes and JFH-1 is mixed with a human anti-HCV antibody and allowed to react at 37°C for 1 hour to prepare a mixed sample. Next, the mixed sample is added to a non-infected Huh-7 cell seeded on a 12-well plate for causing infection. The cell is subcultured into a 6-well plate 3 days after the infection, and a culture supernatant is collected on the 3rd day and the 6th day of the subculture. The inclusion of chimeric HCV particle in the collected culture supernatant is verified by measuring infectious titer of HCV and then the collected culture supernatant after verifying the infectious titer (the culture supernatant collected on the 3rd or 6th day) is mixed again with the antibody and allowed to newly infect a non-infected cell, which are repeated 8 times (the number of repeating the subculture infection: 8 in total). The infectious titer of HCV contained in the finally-obtained culture supernatant is measured and an infection inhibiting concentration IC₅₀ is calculated thereon. If the infection inhibiting concentration IC₅₀ is not remarkably increased and the infection inhibiting activity is not greatly lowered (for example, if the infection inhibiting concentration IC₅₀ is 1 µg/mL or less) even after the repeated subculture infection of 8 times, the antibody can be determined to exhibit the escape mutant emergence suppressive property.

The literature of Meital et al., (Proc. Natl. Acad. Sci. U.S.A., 2008, vol. 105, pp. 19450-19455) shows that in evaluation of the AP33 antibody by the above-described repeated subculture infection that an escape mutant did not emerge after repeating the subculture infection 3 times but an escape mutant emerged after repeating it 5 times. It is regarded, based on this result, that the AP33 antibody is not an antibody exhibiting the escape mutant emergence suppressive property. The literature of Keck et al., (Plos Pathogens, 2012, vol. 8, e1002653) discloses that as a result of the evaluation by the above-described repeated subculture infection, an escape mutant emerged when using CBH-2 antibody after repeating the subculture infection 3 times, while the emergence of an escape mutant was suppressed when using HC-84.1 antibody or HC-84.25 antibody after repeating the subculture infection 4 times or 5 times respectively, and that, based on the results, HC-84.1 antibody and HC-84.25 antibody are antibodies exhibiting the escape mutant emergence suppressive property. With respect to these antibodies, however, International Publication No. WO2013/033319 reports data indicating an increase of the amount of the antibody necessary for keeping a constant concentration of virus (namely, increase of the value IC₅₀) during the repeated infection, which suggests the emergence of an escape mutant, when these antibodies are evaluated by a method different from the above-described evaluation method through the repeated subculture infection, in particular, by a method which is different from the method of Meital et al. in the amount of virus and the concentration of the antibody treatment, and in which the repeated infection is started from a concentration corresponding to an infection inhibiting activity value IC₅₀, (International Publication No. WO2013/033319). Accordingly, based on the known information, preferably when carrying out the evaluation through the above-described repeated subculture infection, if the emergence of an escape mutant is inhibited even after repeating the subculture infection 4 to 5 times, the antibody can be determined to exhibit the escape mutant emergence suppressive property. More preferably, when carrying out the evaluation through the above-described repeated subculture infection, if an escape mutant does not emerge after repeating the subculture infection 8 times, the antibody can be determined to exhibit the escape mutant emergence suppressive property.

When using the known anti-HCV antibodies exhibiting no (or low) suppressive property against escape mutant emergence, an escape mutant emerges during the repeated subculture infection of 4 to 5 times, and therefore, the infection inhibiting activity is greatly decreased. The emergence of an escape mutant can be confirmed by analyzing a nucleotide sequence of HCV contained in the culture supernatant obtained during or at the last of the repeated infection culture, and identifying a mutated amino acid residue therein.

As one example, the antibody or the antigen-binding antibody fragment of the present invention binding to a conformational epitope preferably exhibits the escape mutant emergence suppressive property.

The antibody or the antigen-binding antibody fragment of the present invention specifically binds to the HCV E2 protein. In one embodiment, the antibody or the antigen-binding antibody fragment of the present invention specifically recognizes (binds to) particularly a structural epitope (conformational epitope) of the HCV E2 protein.

Since the antibody or the antigen-binding antibody fragment of the present invention has high infection inhibiting activity against HCV of a plurality of genotypes, it can be used as a medicament, particularly, an agent for treatment or prevention of hepatitis C. The present invention also provides a medicament, particularly, an agent for treatment or prevention of hepatitis C, comprising the antibody or the antigen-binding antibody fragment of the present invention, as an active ingredient.

The medicament of the present invention may be a pharmaceutical composition also comprising a pharmaceutically acceptable carrier. The term "pharmaceutically acceptable carrier" refers to a solvent and/or an additive that may be generally used in the art of drug formulation technology.

Examples of the pharmaceutically acceptable solvent include water and pharmaceutically acceptable organic agents (such as ethanol, propylene glycol, ethoxylated isostearyl alcohol, polyoxylated isostearyl alcohol, and polyoxyethylene sorbitan fatty acid esters). These solvents are preferably sterilized, and preferably adjusted to be isotonic with blood if necessary.

Examples of the pharmaceutically acceptable additive include collagen, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, agar, polyethylene glycol, diglycerine, glycerine, propylene glycol, vaseline, paraffin, stearyl alcohol, stearic acid, human serum albumin (HSA), mannitol, sorbitol, lactose, and a surfactant acceptable as a pharmaceutical additive, and the like.

As other pharmaceutically acceptable additives, an excipient, a binder, a disintegrator, a filler, an emulsifier, a flow control agent, a lubricant, a corrective agent, a solubilizing agent (solubilizer), a suspending agent, a diluent, a surfactant, a stabilizer, an absorption enhancer, an expander, a moisturizer, a humectant (such as glycerin or starch), an absorbent, a disintegration inhibitor, a coating agent, a colorant, a preservative, an antioxidant, a perfume, a flavor, a sweetener, a buffer or the like may be further contained if necessary.

The solvents or the additives may be used alone or in any combination depending on a dosage form. When used as, for example, an injectable preparation, the purified antibody can be dissolved in a solvent (such as a saline, a buffer or a glucose solution), and an adsorption inhibitor (such as Tween 80, Tween 20, gelatin or human serum albumin) can be added. Alternatively, the antibody may be lyophilized to prepare a dosage form that can be dissolved to be reconstituted before use. For example, an excipient (such as a sugar alcohol or a sugar such as mannitol or glucose) can be used for lyophilization.

The medicament of the present invention can be formulated in accordance with a conventional method. For example, see, Remington's Pharmaceutical Science, latest edition, Mark Publishing Company, Easton, U.S.A. for the formulation.

The antibody or the antigen-binding antibody fragment, or the medicament of the present invention can be administered by oral administration, intratissue administration (such as subcutaneous administration, intramuscular administration or intravenous administration), local administration (such as transdermal administration) or transrectal administration. The dosage form is preferably a form suitable to the administration method. For example, if the intratissue administration is employed, an injection through bloodstream is preferred, and in this case, the dosage form is typically a liquid.

If the injection is employed, the injection site is not especially limited. The injection can be, for example, intravenous, intraarterial, intrahepatic, intramuscular, intraarticular, intrabone marrow, intrathecal, intraventricular, transdermal, subcutaneous, intradermal, intraperitoneal, intranasal, intestinal or sublingual injection. Preferably, it is an intravascular injection such as intravenous injection or intraarterial injection. By using such a injection, the medicament of the present invention can be immediately spread all over the body through bloodstream, and invasiveness is comparatively low, and hence a burden on a subject of a patient or the like is small. Alternatively, it can be injected in liver or hepatic portal vein. This is because, when using the injections, the medicament of the present invention can directly act on a site of HCV localization.

When the antibody or the antigen-binding antibody fragment, or the medicament of the present invention is administered to a subject (such as a patient), each dosage unit preferably contains the antibody or the antigen-binding antibody fragment of the present invention in an effective amount to exhibit the HCV infection inhibiting activity. The term "effective amount" refers to an amount that is necessary for an active ingredient to exhibit its function, namely in the present invention, an amount necessary for inhibiting HCV infection, and causes little or no harmful adverse effect in a subject of the administration. The effective amount can vary depending on various conditions including information of a subject (such as a patient), the dosage form, the administration pathway and the like. The "information of a subject (such as a patient)" includes the progress degree or severity of a disease, systemic physical conditions, the age, the weight, the gender, dietary habit, drug sensitivity, whether or not another medicament is used in combination, and resistance to the treatment. Ultimate dose and effective amount of the medicament of the present invention are determined by a physician depending on the information and the like of each subject. When it is necessary to administer a large amount of the medicament of the present invention for attaining the HCV infection inhibiting effect, it can be administered dividedly in several times for reducing a burden on the subject.

When the medicament of the present invention is administered to a subject, a dose of the antibody or the antigen-binding antibody fragment of the present invention being an active ingredient, is selected in a range of 0.001 mg to 1000 mg per kilogram of body weight per administration. Alternatively, a dose of 0.01 to 100000 mg/body for each subject can be selected, but the present invention is not limited to such a dose. As for timing of the administration, it can be administered no matter whether clinical symptoms of the disease have occurred.

As a specific example of the dose, for example, if it is administered to a human adult male (having a body weight of 60 kg) that is at an initial stage of hepatitis C after onset and does not require to use another medicament in combination, the effective amount per day for the above-mentioned medicament is generally 1 to 2000 mg, more preferably 1 to 1000 mg, and further preferably 1 to 500 mg. Depending on the information of the subject, the administration pathway and the like, a dose smaller than or beyond the above-described range can be administered.

A subject to which the antibody or the antigen-binding antibody fragment, or the medicament of the present invention is to be administered is, but not limited to, a mammal including preferably primates such as a human, a domestic animal, a pet or a laboratory animal (a test animal), and is particularly preferably primates such as a human. The subject may be affected with hepatitis C or may have not been affected but have a risk of acquiring hepatitis C. The subject may be a chronic hepatitis C patient who is going to receive a liver transplant, or may be a patient who is to undergo an operation requiring blood transfusion.

The antibody or the antigen-binding antibody fragment, or the medicament of the present invention can be effective against any hepatitis C, and preferably is effective against chronic hepatitis C or fulminant hepatitis C, and is particularly effective against hepatitis C caused by HCV of various genotypes (such as 1a, 1b, 2a, 3a, 4a, 5a or 6a), for example, HCV of genotypes 1a, 2a, 3a or 1b. The antibody or the antigen-binding antibody fragment, or the medicament of the present invention can also suppress infection expansion at the cellular level (spread of HCV to other cells) in a living body once infected with HCV. Accordingly, the present invention also relates to a method for treating or preventing hepatitis C virus infection comprising administering the antibody or the antigen-binding antibody fragment, or the medicament of the present invention to a subject.

The antibody or the antigen-binding antibody fragment, or the medicament of the present invention can be suitably used as an agent for prevention of hepatitis C to be used in liver transplantation. For example, it is preferably administered during living liver transplantation to a chronic hepatitis C patient for preventing recurrence of hepatitis C, before the liver transplantation, during the liver transplantation or after the liver transplantation.

The medicament of the present invention may be mixed, in an appropriate amount, with or used in combination of another agent in order to supplement or enhance the treatment or preventive effect, or to reduce the dose. Examples of the agent to be used in combination include existing antiviral agents such as interferon and ribavirin.

The antibody or the antigen-binding antibody fragment of the present invention can be used as an HCV detection reagent for use in detecting HCV in a sample. The present invention relates also to a method for detecting HCV in a sample using the antibody or the antigen-binding antibody fragment of the present invention.

A sample refers to any of various samples that can contain HCV (an HCV particle or an envelope protein thereof). Examples of the sample include a cultured cell, a cultured cell disrupted solution, a culture supernatant and a sample from a subject, such as a human sample. A human sample refers to any of human-derived biological samples such as tissue collected from a human (such as tissue collected by an operation), and body fluids such as blood, serum, plasma, urine, spinal fluid, saliva, lymph fluid and seminal fluid, and is preferably blood, serum, plasm or urine. Alternatively, the sample may be a liquid sample as well as a solid sample. For example, the sample may be a donor organ for organ transplantation, a tissue section sample or the like.

The detection of HCV can be performed by any of known immunological detection methods using a labeled antibody, such as an ELISA method, an EIA method, a fluoroimmunoassay method, a radioimmunoassay method and a luminescence immunoassay method; a surface plasmon resonance method (SPR method); and a quartz crystal microbalance method (QCM method), and is preferably performed by an immunological detection method using a labeled antibody.

The ELISA method is also called an enzyme immunosorbent assay, and is a method for quantifying a target antigen by detecting a small amount of the target antigen in a sample using an antibody or an antigen labeled with an enzyme, via antigen-antibody reaction based on the action of the enzyme, as a coloring density or a fluorescence intensity. In the method, for example, the antibody or the antigen-binding antibody fragment of the present invention is immobilized on a solid-phase support, and an immunological reaction between the antibody and HCV in a sample is enzymatically detected. Known methods (for example, "Koso Meneki Sokutei Ho (Enzyme Immunoassay)" edited by Eiji Ishikawa et al., 1987, the third edition, Igaku-Shoin Ltd.) can be seen for the measurement via the ELISA method. As the solid-phase support, an insoluble support made of polystyrene, polycarbonate, polyvinyl toluene, polypropylene, polyethylene, polyvinyl chloride, nylon, polymethacrylate, latex, gelatin, agarose, cellulose, Sepharose, glass, a metal, ceramics, or a magnetic material, in the shape of a bead, a microplate, a test tube, a stick, a test piece or the like can be used. The immobilization of the antibody or the antigen-binding antibody fragment of the present invention to the solid-phase support can be attained by binding them by any of known methods such as a physical adsorption method, a chemical binding method and a combination of these.

Examples of a labeling substance used for labeling the antibody or the antigen-binding antibody fragment of the present invention include, but not limited to, peroxidase (POD), alkaline phosphatase, β-galactosidase, urease, catalase, glucose oxidase, lactate dehydrogenase, amylase, and a biotin-avidin complex in employing, for example, the ELISA method; fluorescein isothiocyanate, tetramethylrhodamine isothiocyanate, substituted rhodamine isothiocyanate, dichlorotriazine isochiocyanate, Alexa 480 and Alexa Fluor 488 in employing the fluoroimmunoassay; and tritium, iodine-125 (¹²⁵I) or iodine-131 (¹³¹I) in employing the radioimmunoassay. Alternatively, a NADH-FMNH2-luciferase system, a luminol-hydrogen peroxide-POD system, an acridinium ester system, or a dioxetane compound system or the like can be used in employing the luminescence immunoassay. For binding a labeled antigen to an antibody, any of known methods such as a glutaraldehyde method, a maleimide method, a pyridyl disulfide method, and a periodic acid method can be employed for the ELISA method, and any of known methods such as a chloramine T method and a Bolton-Hunter method can be employed for the radioimmunoassay.

Alternatively, the immunological measurement method described above can be performed by measuring generation of immune complex aggregates in immunonephelometry, latex agglutination, latex turbidimetry, hemagglutination or particle agglutination, via measuring a transmitted light or scattered light with an optical method, or via visually measuring it. In this case, a phosphate buffer, a glycine buffer, a Tris buffer or a Good's buffer can be used as a solvent, and a reaction accelerator such as polyethylene glycol, or a non-specific reaction inhibitor may be further contained in the solvent.

As a specific example of the HCV detection method, application of ELISA sandwich assay will now be simply described with reference to an example. First, the antibody of the present invention is immobilized on an insoluble support. The antibodies to be immobilized may be one type or a plurality of types of antibodies as long as they can specifically recognize HCV. Next, a sample that can contain HCV is applied to act on the surface having the immobilized antibody to form a complex of the immobilized antibody and HCV on the surface of the support. Thereafter, unbound substances in the sample, excluding HCV, are removed by sufficiently washing with a wash solution. Furthermore, another anti-HCV labeled antibody specifically recognizing HCV is prepared, and this labeled antibody is applied to act on the support to which the complex of the immobilized antibody and HCV binds, the support is sufficiently washed with a wash solution, and then detection based on the labelling is carried out, which can detect HCV present in the sample.

Alternatively, a labeled antibody and a sample containing HCV can be mixed to form an antigen-antibody complex, and then applied to act on the immobilized antibody. If the antibody to be immobilized is labeled with biotin, when an antigen-antibody complex is formed by mixing all of the biotinylated immobilized antibody, a sample containing HCV and an antibody having a label different from biotin, and then avidin is applied to act on the immobilized support, the antigen-antibody complex can be detected using the label different from biotin.

An immunochromatographic test strip can be used in the immunological measurement method. The immunochromatographic test strip may be composed of, for example, a sample accepting section made of a material easily absorbing a sample, a reagent section containing a diagnostic agent of the present invention, a development section for allowing a reaction product between the sample and the diagnostic agent to move, a labeling section for coloring the developed reaction product, and a display section where the colored reaction product develops. A commercially available pregnancy test kit has a similar form thereto. The principle of this measurement method is as follows. First, when a sample is applied to the sample accepting section, the sample accepting section absorbs the sample and allows the sample to reach the reagent section. Subsequently, an antigen-antibody reaction is caused between HCV in the sample and an antibody, and the thus formed reaction complex moves through the development section to reach the labelling portion. In the labeling portion, a reaction is caused between the reaction complex and a labeled secondary antibody, and then when the resulting reaction product with the labeled secondary antibody reaches the display section, coloring is shown. The immunochromatographic test strip is an extremely low invasive technique, and causes no pain and no risk of reagent use in a user, and therefore can be used for monitoring at home, and a result obtained by the test strip can be carefully examined and the user can be treated (by surgical resection or the like) at each medical institution level so that metastasis/recurrence can be prevented. Such a test strip can be advantageously inexpensively mass-produced.

### Examples

The present invention will be more specifically described below with reference to Examples.

### (Example 1) Screening of Antibody Phage Library

For screening of human anti-HCV antibodies as described below, a general phage display method described in Chan et al., J. Gen. Virol, 1996, vol. 77, pp. 2531-2539; Bugli et al., J. Virol, 2001, vol. 75, pp. 9986-9990; and Jostock et al., J. Immunol. Methods, 2004, vol. 289, pp. 65-80 was employed.

### 1. Preparation of Antibody Phage Library

The antibody phage library described below was prepared in accordance with a method sufficiently described in Gejima et al., Human antibodies, (2002), vol. 11, pp. 121-129.

mRNAs encoding an antibody gene were isolated from a human chronic hepatitis C patient, and were used for synthesizing cDNA by RT-PCR. A VH region gene, and a VLCL region gene (a VL region gene and a CL region gene) were separately amplified by PCR from the cDNA. The amplified VH region genes and VLCL region genes were inserted into a phagemid vector pTZ19R which contains a linker DNA encoding a linker peptide sequence such as (GGGGS)₃ (SEQ ID NO: 35) to prepare scFv genes in which the VH region gene, the linker, the VL region gene and the CL region gene are placed in this order. The thus prepared scFv genes were incorporated into a phagemid vector pTZ19R by utilizing a restriction enzyme site *Hin*dIII to construct a scFv gene library. The scFv gene library was transformed into *E. coli* such as TG-1 (SupF), and then superinfected with a helper phage M13K07 to prepare a scFv phage library. The thus prepared antibody phage is a phage displaying a peptide in which the VH region and the VLCL region of the human antibody are linked via the linker.

### 2. Preparation of HCV E2 Protein used in Screening

HCV E2 protein used in the screening was prepared by using Drosophila Expression System (Thermo Fisher Scientific). The employed method is sufficiently described in Krey et al., PLOS PATHOGENS, vol. 6, e1000762.

Each of a DNA encoding the E2 protein (amino acids 384-711; not containing a transmembrane region) of the HCV TH strain (genotype 1b, Wakita T. et al., J. Biol. Chem. 1994, vol. 269, pp. 14205-14210, International Publication No. WO2006/022422) or a DNA encoding the E2 protein (amino acids 384-714; not containing a transmembrane region) of the HCV JFH-1 strain (genotype 2a, International Publication No. WO2004/104198) was inserted with restriction enzyme sites *Bgl*II and *Xba*I of a S2 cell expression vector pMT/BiP/V5-His (Thermo Fisher Scientific).

Transfection was performed by the calcium phosphate precipitation method. A transfection solution was prepared as follows. 19 µg of the pMT/BiP/V5-His in which the insert was recombined with the HCV E2 protein encoding gene, 1 µg of a selection plasmid vector pCoHygro, and 36 µL of 2 M CaCl₂ were combined and adjusted with purified water to a volume of 300 µL. The mixed solution was slowly added dropwise to HEPES-Buffered Saline (HBS; 50 mmol/L HEPES, 1.5 mmol/L Na₂HPO₄, 280 mmol/L NaCl, pH 7.1), and mixed well with gentle vortex to prepare the transfection solution.

In a 6-well plate, 2 mL of S2 cells were seeded at 1 x 10⁶ cells/mL, and cultured at 28°C for about 12 hours to give a density of 2 to 4 x 10⁶ cells/mL. The above-prepared transfection solution was allowed to stand at room temperature for 30 minutes, and then equally dropped over the 6-well plate in which the S2 cells had been seeded, and then cultured at 28°C for 24 hours. After 24 hours, the medium was exchanged (Shneider's Drosophila Medium, 10% FBS, penicillin (50 units/mL), streptomycin (50 µg/mL), 300 µg/mL Hygromycin B), and the cells were subcultured with the medium exchanged every 3 or 4 days. A stable cell strain was established by performing the selective culture for about 3 weeks.

For induction of expression of the E2 protein, CuSO₄ (at a final concentration of 0.5 mmol/L) was added to 2 x 10⁶ S2 cells (40 mL per 225 cm² flask) and the cells cultured for 5 days. The culture medium was collected and centrifuged (2000 rpm, 5 minutes) to remove cells and obtain a culture supernatant. The culture supernatant was purified by using a chromatography support (Ni-NTA agarose) for purifying His-tagged proteins, to obtain an E2 protein of the HCV TH strain (genotype 1b) or an E2 protein of the HCV JFH-1 (genotype 2a).

### 3. Screening of Antibody Phage Binding to HCV E2 Protein

The screening of antibody phages as described below was performed on the basis of a method sufficiently described in Gejima et al., Human antibodies, (2002), vol. 11, pp. 121-129.

The HCV E2 protein prepared as described in Item 2 above (the E2 protein of the TH strain (genotype 1b) or the E2 protein of the JFH-1 strain (genotype 2a)) was allowed to bind to a plastic surface of an immunotube or the like or a magnetic bead surface, the scFv phage library prepared as described in Item 1 above was added thereto to react them, and thereafter, the resultant was washed with PBS containing 0.1% Tween 20 to remove a non-specific phage. A specifically bound phage was eluted with 0.1 mol/L Glycine-HCl (pH 2.2), allowed to infect *E. coli* and amplified. The steps were repeated 3 times to concentrate and obtain phages specific to the HCV E2 protein (the E2 protein of the TH strain (genotype 1b) or the E2 protein of the JFH-1 strain (genotype 2a)).

### 4. Variable Regions of Selected Antibody Phage

The nucleotide sequences of the VH region and the VL region in a peptide displayed by each of three antibody phages (e2d066, e2d073 and e2d081) selected in Item 3 above were analyzed by a conventional method. Further, the nucleotide sequence of the CL region was also analyzed by a conventional method.

### (1) Antibody Phage e2d066

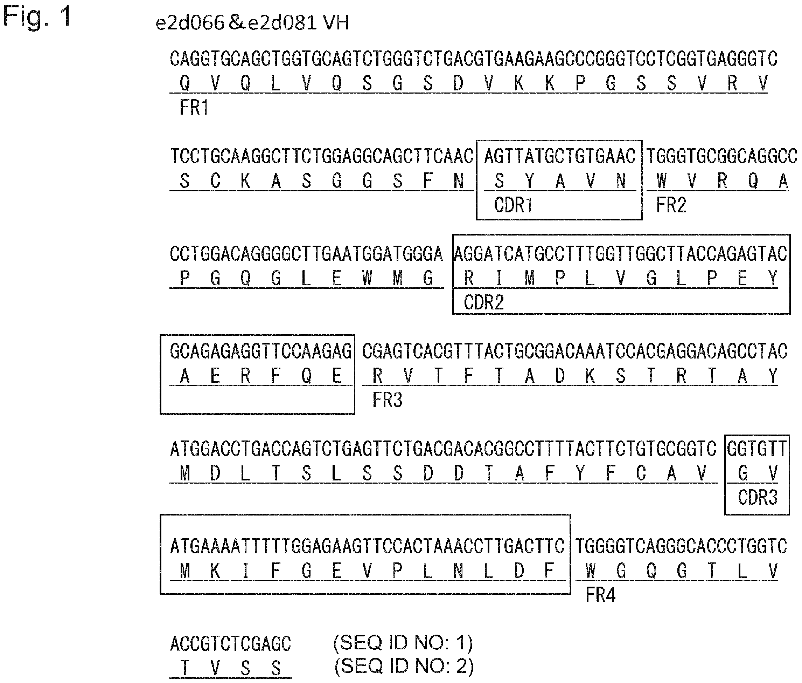

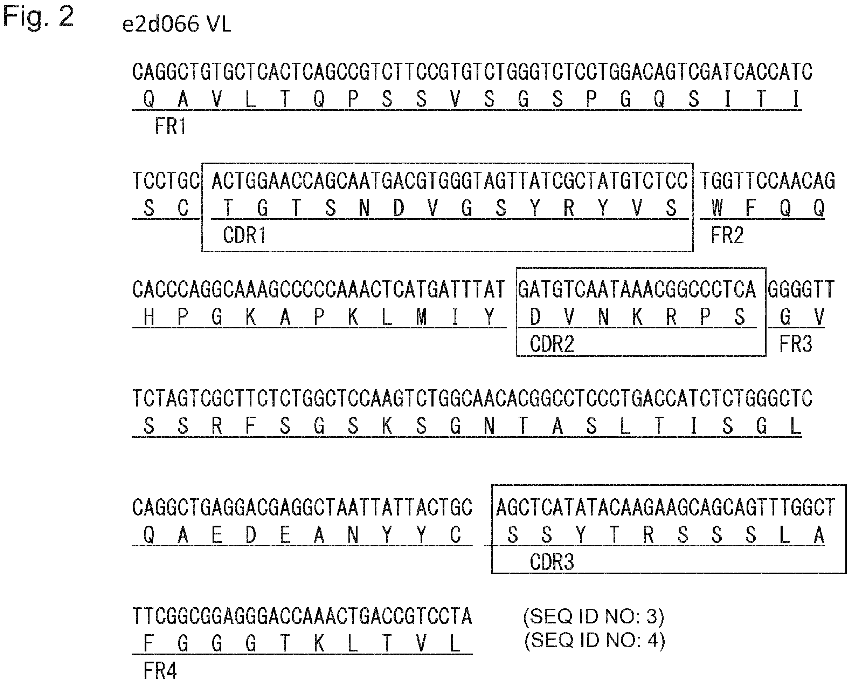

The nucleotide sequence encoding the VH region displayed by the antibody phage e2d066 is set forth in SEQ ID NO: 1, the amino acid sequence thereof is set forth in SEQ ID NO: 2 (Figure 1), and the nucleotide sequence encoding the VL region is set forth in SEQ ID NO: 3, and the amino acid sequence thereof is set forth in SEQ ID NO: 4 (Figure 2). The amino acid sequences of CDR1, CDR2 and CDR3 in the VH region displayed by the antibody phage e2d066 are set forth in SEQ ID NO: 5, SEQ ID NO: 6 and SEQ ID NO: 7 respectively, and the amino acid sequences of CDR1, CDR2 and CDR3 in the VL region are set forth in SEQ ID NO: 8, SEQ ID NO: 9 and SEQ ID NO: 10 respectively. Furthermore, the amino acid sequence of the CL region is set forth in SEQ ID NO: 38.

### (2) Antibody Phage e2d073

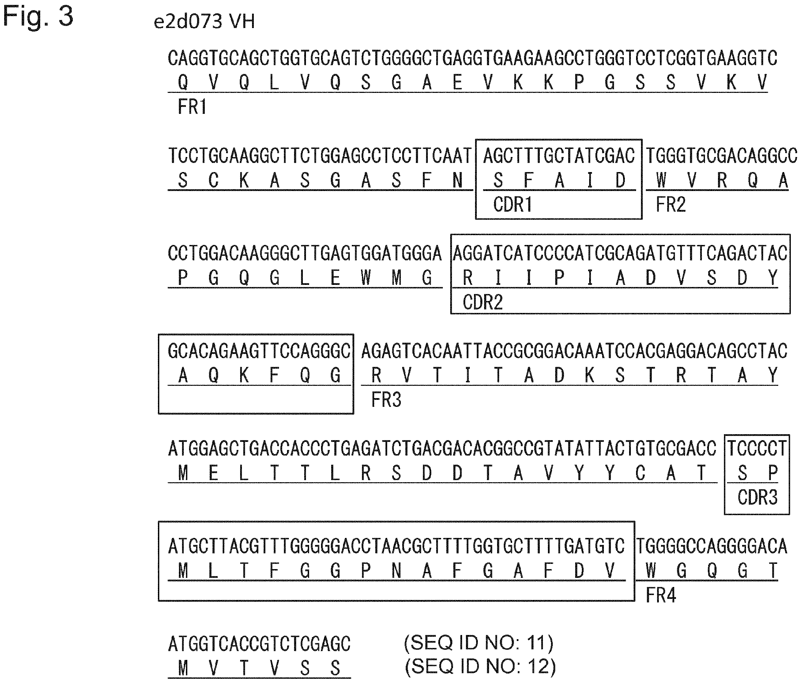

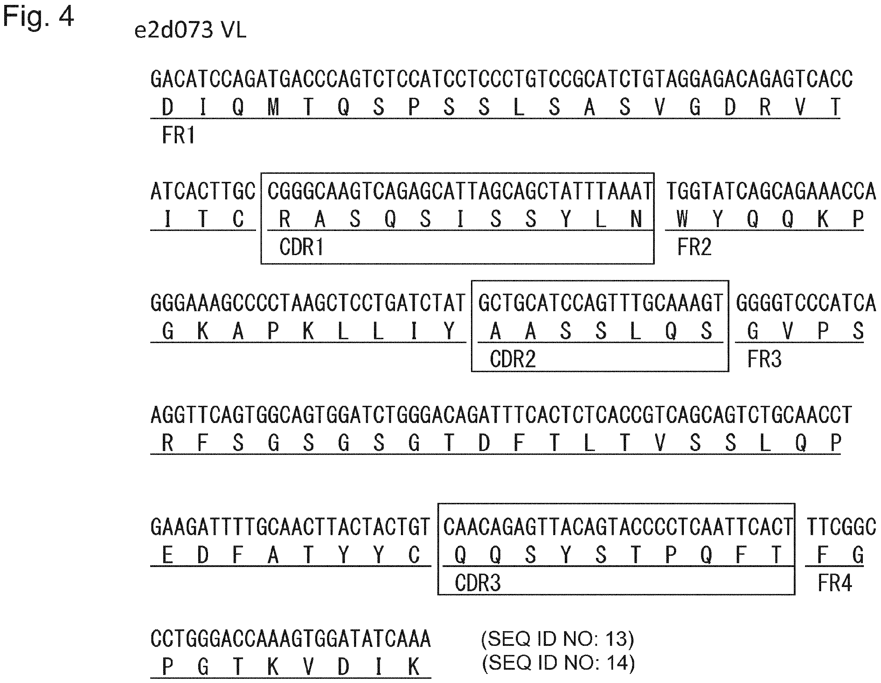

The nucleotide sequence encoding the VH region displayed by the antibody phage e2d073 is set forth in SEQ ID NO: 11, the amino acid sequence thereof is set forth in SEQ ID NO: 12 (Figure 3), and the nucleotide sequence encoding the VL region is set forth in SEQ ID NO: 13, and the amino acid sequence thereof is set forth in SEQ ID NO: 14 (Figure 4). The amino acid sequences of CDR1, CDR2 and CDR3 in the VH region displayed by the antibody phage e2d073 are set forth in SEQ ID NO: 15, SEQ ID NO: 16 and SEQ ID NO: 17 respectively, and the amino acid sequences of CDR1, CDR2 and CDR3 in the VL region are set forth in SEQ ID NO: 18, SEQ ID NO: 19 and SEQ ID NO: 20 respectively. Furthermore, the amino acid sequence of the CL region is set forth in SEQ ID NO: 39.

### (3) Antibody Phage e2d081

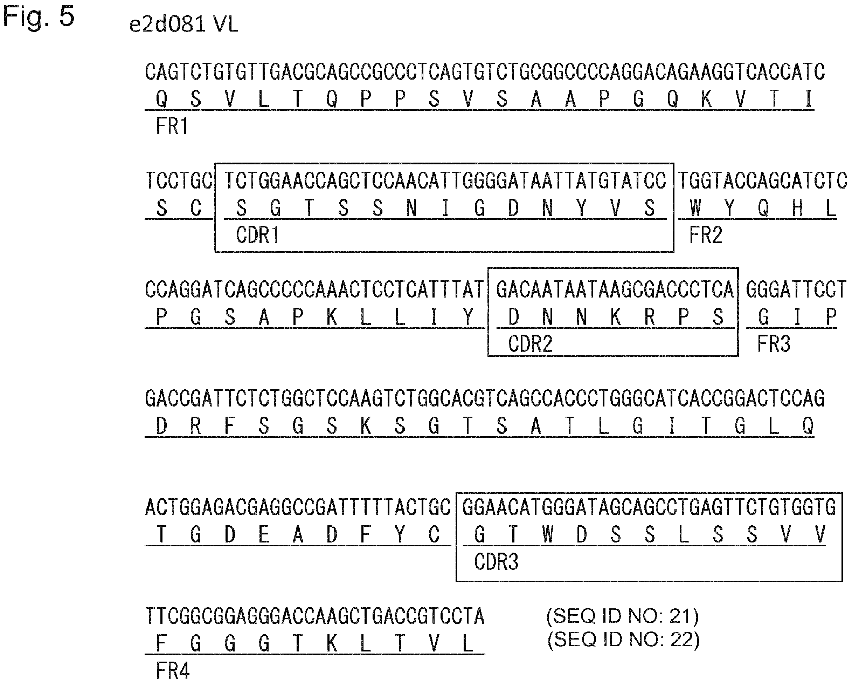

The nucleotide sequence encoding the VH region displayed by the antibody phage e2d081 was the same as the nucleotide sequence encoding the VH region displayed by the antibody phage ed2066. Specifically, the nucleotide sequence encoding the VH region displayed by the antibody phage e2d081 is set forth in SEQ ID NO: 1, the amino acid sequence thereof is set forth in SEQ ID NO: 2 (Figure 1), and the amino acid sequences of CDR1, CDR2 and CDR3 in the VH region are set forth in SEQ ID NO: 5, SEQ ID NO: 6 and SEQ ID NO: 7 respectively. The nucleotide sequence encoding the VL region displayed by the antibody phase e2d081 is set forth in SEQ ID NO: 21, and the amino acid sequence thereof is set forth in SEQ ID NO: 22 (Figure 5), and the amino acid sequences of CDR1, CDR2 and CDR3 in the VL region are set forth in SEQ ID NO: 23, SEQ ID NO: 24 and SEQ ID NO: 25 respectively. Furthermore, the amino acid sequence of the CL region is set forth in SEQ ID NO: 40.

Figures 1 to 5 illustrate a correspondence between the nucleotide sequence (upper one) encoding the VH region or the VL region and the amino acid sequence thereof (lower one), and the FR1, CDR1, FR2, CDR2, FR3, CDR3 and FR4 in the VH region or the VL region. Figure 1 illustrates the VH region displayed by the antibody phage e2d066 and the antibody phage e2d081, Figure 2 illustrates the VL region displayed by the antibody phage e2d066, Figure 3 illustrates the VH region displayed by the antibody phage e2d073, Figure 4 illustrates the VL region displayed by the antibody phage e2d073, and Figure 5 illustrates the VL region displayed by the antibody phage e2d081.

Table 1 shows CDRs displayed by the antibody phages (e2d066, e2d073 and e2d081).

**[Table 1]**

| **VH Chain** | | | |
|---|---|---|---|
| | **CDR1** | **CDR2** | **CDR3** |
| **e2d066,e2d081** | SYAVN (SEQ ID NO: 5) | RIMPLVGLPEYAERFQE (SEQ ID NO: 6) | GVMKIFGEVPLNLDF (SEQ ID NO: 7) |
| **e2d073** | SFAID (SEQ ID NO: 15) | RIIPIADVSDYAQKFQG (SEQ ID NO: 16) | SPMLTFGGPNAFGAFDV (SEQ ID NO: 17) |
| | | | |

| **VL Chain** | | | |
|---|---|---|---|
| | **CDR1** | **CDR2** | **CDR3** |
| **e2d066** | TGTSNDVGSYRYVS (SEQ ID NO: 8) | DVNKRPS (SEQ ID NO: 9) | SSYTRSSSLA (SEQ ID NO: 10) |
| **e2d081** | SGTSSNIGDNYVS (SEQ ID NO: 23) | DNNKRPS (SEQ ID NO: 24) | GTWDSSLSSVV (SEQ ID NO: 25) |
| **e2d073** | RASQSISSYLN (SEQ ID NO: 18) | AASSLQS (SEQ ID NO: 19) | QQSYSTPQFT (SEQ ID NO: 20) |

### (Example 2) Preparation of scFv

On the basis of the nucleotide sequences and the amino acid sequences of the VH regions, the VL regions and the CL regions (VLCL regions) of the selected three antibody phages (e2d066, e2d073 and e2d081), scFvs in which the VH region and the VLCL region of each antibody phage are linked via a linker were prepared by a conventional method. As the linker, (GGGGS)₃ (SEQ ID NO: 35) was used, and the linker was linked to the C terminal of the VH region and the N terminal of the VL region by a conventional method. The amino acid sequence of the scFv comprising the VH region (SEQ ID NO: 2), the VL region (SEQ ID NO: 4) and the CL region (SEQ ID NO: 38) (VLCL region) of the e2d066 and the linker (SEQ ID NO: 35) (hereinafter referred to as the e2d066 scFv) is shown by SEQ ID NO: 32. The amino acid sequence of the scFv comprising the VH region (SEQ ID NO: 12), the VL region (SEQ ID NO: 14) and the CL region (SEQ ID NO: 39) (VLCL region) of the e2d073 and the linker (SEQ ID NO: 35) (hereinafter referred to as the e2d073 scFv) is shown by SEQ ID NO: 33. The amino acid sequence of the scFv comprising the VH region (SEQ ID NO: 2), the VL region (SEQ ID NO: 22) and the CL region (SEQ ID NO: 40) (VLCL region) of the e2d081 and the linker (SEQ ID NO: 35) (hereinafter referred to as the e2d081 scFv) is shown by SEQ ID NO: 34.

Specifically, each of these phagemid DNAs (selected antibody phages) was digested with restriction enzyme *Sal*I*,* converted into a protein A fused antibody (scFv-PP antibody) through self-ligation, and used for transforming *E. coli* DH12S™. After the transformation, the cells were cultured overnight in 25 mL of 2X YTGA at 30°C. 10 mL of the culture was mixed with 1 L of 2X YTA and cultured for 3 hours, and then 1 mL of 1 mol/L IPTG was added and cultured at 30°C for 20 hours. The resulting culture was centrifuged at 4°C and 8000 rpm for 10 minutes to collect a supernatant. To the collected supernatant, 313 g of ammonium sulfate was slowly added, followed by stirring at room temperature for 30 minutes, and the resultant was centrifuged at 4°C and 8500 rpm for 30 minutes. The resulting precipitate was suspended in PBS containing 20 mL of Complete Protease Inhibitor Cocktail (Roche). Subsequently, the resultant was dialyzed against PBS overnight at 4°C, followed by centrifugation at 4°C and 10000 rpm for 10 minutes. A supernatant was collected and applied to a column filled with 2 mL of IgG Sepharose (GE Healthcare) to allow it to pass through the column at room temperature via natural dripping. After washing the column with 300 mL of PBS, the antibody was eluted with 8 mL of 0.2 mol/L glycine (pH 3.0). The eluate was adjusted to pH 7.0 with 2 mol/L Tris buffer, transferred into a tube for dialysis concentration (Millipore, Amicon Ultra-15, 4°C, rotated at 4500 rpm) and dialyzed against PBS and thereby concentrated. Thereafter, it was subjected to SDS-PAGE and the protein concentration was calculated.

### (Example 3) Preparation of IgG Antibody

On the basis of the nucleotide sequences and the amino acid sequences of the VH regions and the VL regions of the selected three antibody phages (e2d066, e2d073 and e2d081), IgG antibodies each comprising a VH region and a VL region were produced by a conventional method.

Specifically, PCR was carried out using the VH and VLCL genes of the scFv antibodies used as templates and with H chain and L chain amplification primers. The VH amplified product was ligated into a construction vector containing a human IgG1 constant region, and the amplified VLCL was ligated into an L chain construction vector, and these were linked to each other to obtain a plasmid DNA containing an IgG antibody gene.

The plasmid DNA was digested with a restriction enzyme and linearized, and 40 µg of the linearized plasmid was introduced into 1 x 10⁷ CHO-K1 cells by electroporation at 250 V and 800 µF. Immediately after the introduction, the cells were suspended in a Ham-F12 medium (Wako Pure Chemical Industries, Ltd.: 087-08335 + MBL268-1) containing 10% FBS, and started to be cultured in a 5% CO₂ incubator at 37°C.

After 24 hours, puromycin (Sigma-Aldrich, P8833) was added thereto at a final concentration of 10 µg/mL for starting selection. After about 10 to 14 days, a selected colony was checked, and the cells were washed with 20 mL of PBS, and treated with 1 mL of 0.05% Trypsin-EDTA (Wako Pure Chemical Industries, Ltd.: 264-16935), 5 mL of Ham-F12 was added thereto, and the cells were detached from the plate and collected, and the number of the cells was counted. On the basis of the counted result, limiting dilution was performed at 0.2 cell/200 µL/well (five 96-well plates). After culturing for 14 days, cells highly expressing an IgG antibody were selected by the ELISA using a culture supernatant of each well. The selected cells were conditioned in a serum-free medium EX-CELL CD CHO Fusion (Nichirei Biosciences Inc.: 14365C), and the resultant cells were cultured at an initial cell count of 2 x 10⁵ for 10 days and then a supernatant was collected.

A column was filled with 1 mL of rProtein A Sepharose Fast Flow (GE Healthcare: 17-1279-02), and the culture supernatant was added thereto and fed at a flow rate of 1 drop/2 seconds to allow an expressed protein (IgG) to bind to the column. 10 mL of PBS was fed at a flow rate of 1 drop/2 seconds for washing off a non-adsorbed component, and then, 10 mL of an elution buffer (0.2 mol/L glycine, pH 3) was fed at a flow rate of 1 drop/second, and an eluate was collected. Amicon Ultra-15 Centrifugal Filter Unit 10 (Millipore: UFC901096) was used to perform ultrafiltration concentration by centrifugation simultaneously with solution displacement (PBS) up to 1 mL, and the concentration of the antibody protein was calculated by the SDS-PAGE.

With respect to the obtained IgG antibody (referred to as e2d066 IgG) containing the VH region (SEQ ID NO: 2) and the VL region (SEQ ID NO: 4) of the e2d066, the amino acid sequence of the entire heavy chain is set forth in SEQ ID NO: 26, and the amino acid sequence of the entire light chain is set forth in SEQ ID NO: 27. With respect to the obtained IgG antibody (referred to as e2d073 IgG) containing the VH region (SEQ ID NO: 12) and the VL region (SEQ ID NO: 14) of the e2d073, the amino acid sequence of the entire heavy chain is set forth in SEQ ID NO: 28, and the amino acid sequence of the entire light chain is set forth in SEQ ID NO: 29. With respect to the obtained IgG antibody (referred to as e2d081 IgG) containing the VH region (SEQ ID NO: 2) and the VL region (SEQ ID NO: 22) of the e2d081, the amino acid sequence of the entire heavy chain is set forth in SEQ ID NO: 30, and the amino acid sequence of the entire light chain is set forth in SEQ ID NO: 31.

### (Example 4) Infection Inhibiting Activity of scFv and IgG Antibodies

### 1. Preparation of Chimeric HCV Particle (HCVcc) of Each Genotype

### (1) Production of J6/JFH-1 Chimeric HCV Particle (J6/JFH-1 HCVcc)

A J6/JFH-1 chimeric HCV particle (J6/JFH-1 HCVcc) was produced as follows, in accordance with a method described in International Publication No. WO2011/052735.

A plasmid DNA (pJFH-1), in which cDNA (genome cDNA) obtained by reverse transcription of the entire region of genome RNA of the HCV JFH-1 strain (genotype 2a) had been cloned downstream of a T7 RNA promoter sequence of a pUC19 plasmid, was prepared in accordance with a method described by Wakita, T. et al., Nat. Med., 2005, vol. 11, pp. 791-796, and International Publication No. WO2004/104198. The pJFH-1 was digested with *Eco*RI*,* and further partially digested with *Bcl*I*,* to prepare a plasmid DNA fragment in which a fragment (about 2840 bp) from an *Eco*RI site to a first *Bcl*I site had been cut off, and the resultant fragment was purified.

On the other hand, a plasmid DNA (pJ6CF), in which genome cDNA (GenBank accession No. AF177036; Yanagi, M. et al., Virology, 1999, vol. 262, pp. 250-263) of the HCV J6CF strain (genotype 2a) had been cloned downstream of the T7 RNA promoter sequence of the pUC19 plasmid, was produced in accordance with a method described in International Publication No. WO2006/022422. The pJ6CF was partially digested with *Eco*RI and *Bcl*I*,* and the resultant fragment of about 2840 bp was purified, and the purified fragment was linked with the above-described pJFH-1 fragment, in which *Eco*RI*-Bcl*I had been cut off, to obtain a recombinant plasmid DNA (pJ6/JFH-1). DNA cloned in this pJ6/JFH-1 is an HCV chimeric genome cDNA in which a 5' non-coding region, sequences encoding the respective proteins of the core protein, the E1 protein, the E2 protein and the p7 protein, and a sequence encoding a region from the N-terminus of the NS2 protein to an amino acid residue at position 16, of the J6CF strain genome cDNA; and a sequence encoding a region from an amino acid residue at position 17 of the NS2 protein to the C-terminus thereof, sequences encoding the respective proteins of the NS3 protein, the NS4A protein, the NS4B protein, the NS5A protein and the NS5B protein, and the 3' non-coding region, of the JFH-1 strain genome cDNA were linked in this order.

After cleavage of the produced pJ6/JFH-1 with *Xba*I*,* Mung Bean Nuclease (20U) was added thereto (at a total amount of the reaction solution of 50 µL), and the resultant was incubated at 30°C for 30 minutes to blunt the ends of the *Xba*I fragment, and thereafter, the resultant was subjected to phenol/chloroform extraction and ethanol precipitation. The excised plasmid was used as a template to synthesize RNA by using MEGAscript T7 Kit (Thermo Fisher Scientific) (see International Publication No. WO2006/022422) The thus synthesized J6/JFH-1 chimeric genome RNA was used for cell introduction as follows. 3 x 10⁶ Huh-7 cells and 5 µg of the J6/JFH-1 chimeric genome RNA were suspended in a Cytomix solution (120 mmol/L KCI, 0.15 mmol/L CaCl₂, 10 mmol/L K₂HPO₄/KH₂PO₄, 25 mmol/L HEPES, 2 mmol/L EGTA, 5 mmol/L MgCl₂, 20 mmol/L ATP, 50 mmol/L glutathione; 400 µL), the resultant suspension was transferred to a 4 mm cuvette, and electroporation was performed to transfer the J6/JFH-1 chimeric genome RNA into the Huh-7 cells using Gene Pulser (BioRad Laboratories) at 260 V and 950 µF. Thereafter, the Huh-7 cells into which the genome RNA had been introduced were seeded in a dish with a diameter of 10 cm for subculture. During the subculture, the HCV core protein in a culture supernatant was quantified using an HCV antigen ELISA test kit (Ortho Clinical Diagnostics) to check the production of an HCV particle. A culture supernatant containing a large amount of the core protein, and namely, having high HCV particle production activity, was selected and stored as a virus stock of J6/JFH-1 chimeric HCV particle (J6/JFH-1 virus stock).

To Huh-7 cells cultured in 10% FCS-DMEM medium (containing 1% MEM nonessential amino acid solution (Thermo Fisher Scientific), 10 mmol/L HEPES-Tris (pH 7.3) and 1 mmol/L sodium pyruvate) in a dish with a diameter of 10 cm, about 100 µL of the J6/JFH-1 virus stock (4 x 10⁴ ffu/mL (focus forming units/mL)) obtained as described above was added to infect the Huh-7 cells with the HCV particle. The cells were appropriately subcultured so as not to be confluent, and expanding cultured from one 225 cm² flask to four flasks, and further to twelve flasks. Subsequently, cells were detached from eight of the 225 cm² flasks, seeded in two five-layer Cellstack (trademark) (Coming), and a culture medium was added thereto to 650 mL in each. Cells obtained from the remaining four flasks were seeded in twelve flasks, and thus, virus particle production was efficiently continued.

On the next day of the subculture, a culture supernatant was discarded, and 650 mL of 2% FCS-DMEM medium (containing 1% MEM nonessential amino acid solution (Thermo Fisher Scientific), 10 mmol/L HEPES-Tris (pH 7.3), and 1 mmol/L sodium pyruvate) was added thereto. Three days after the medium exchange, a culture supernatant was collected, allowed to pass through a 0.45 µm filter, and stored in a deep freezer. 650 mL of 2% FBS-DMEM medium (containing 1% MEM nonessential amino acid solution, 10 mmol/L HEPES-Tris (pH 7.3), and 1 mmol/L sodium pyruvate) was added to Cellstack after collecting the culture supernatant, the culture was further continued. Two days after this, the same steps were performed, and a culture supernatant was collected. The same steps were repeated once again. From the thus collected culture supernatant, chimeric HCV particles (HCVcc) were purified as described below.

### (2) Purification of J6/JFH-1 Chimeric HCV Particles (J6/JFH-1 HCVcc)

The produced virus particles were purified through the following three processes.

### (A) Consentration and Diafiltration

Hollow Fiber Cartridge (GE Healthcare: 500 kDa cut-off, Model No. UFP-500-C-8A, hereinafter referred to as the "Hollow Fiber") was used to concentrate, by 60 times, the culture supernatant containing the HCV particles obtained in (1) above.

### (B) Density Gradient Ultracentrifugation

3 mL of a cooled TNE buffer (10 mM Tris-HCl (pH 7.4), 150 mM NaCl, 1 mM EDTA) containing 60% sucrose was put in Ultra-clear 25 x 89 mm centrifuge tube (Beckman Coulter: catalogue No. 344058), and 7 mL of a TNE buffer containing 20% sucrose was overlaid thereon. 25 mL of a sample was further overlaid on the TNE buffer containing 20% sucrose. Ultracentrifugation was performed using SW-28 (Beckman Coulter) at 28,000 rpm for 4 hours at 4°C.

The bottom of the tube was bored with a 25G needle (Terumo Corporation), and 12 fractions each of 1 mL were collected through the bore. The specific gravity of each fraction solution was measured to confirm that density gradient of sucrose had been formed. The fractions respectively having the third, fourth and fifth largest specific gravity were collected, and then used for concentration and buffer replacement.

### (C) Concentration and Buffer Replacement

Each elution fraction was subjected to buffer replacement as well as concentration by using Amicon Ultra-15 Centrifugal Filter Units (exclusion molecular weight: 100 kDa, Millipore) and a TNE buffer. The thus obtained concentrate was used in an Example described below as a virus solution containing infectious HCV particles (J6/JFH-1 HCVcc).

### (3) Preparation of Chimeric HCV Particles (HCVcc) of Other Genotypes

With respect to production of chimeric HCV particles of the other genotypes, chimeric HCV particles (H77/JFH-1 HCVcc) of the H77 strain (genotype 1a) and the JFH-1 strain were produced with reference to Lindenbach et al., SCIENCE, 2005, vol. 309, pp. 623-626; chimeric HCV particles (TH/JFH-1 HCVcc) of the TH strain (genotype 1b) and the JFH-1 strain were produced with reference to International Publication No. WO2009/014216 and International Publication No. WO2009/131203; and chimeric HCV particles (S310/JFH-1 HCVcc) of S310-A strain (genotype 3a) and the JFH-1 strain were produced with reference to International Publication No. WO2013/031956. These chimeric HCV particles (HCVcc) are described in detail also in a literature of Pietschmann et al., Proc. Natl. Acad. Sci. USA, 2006, vol. 103, pp. 7408-7413, and were produced basically in accordance with this literature, and were purified by the same method as that employed in the preparation (production and purification) of the J6/JFH-1 HCVcc.

Chimeric genome RNA used in the production of the H77/JFH-1 HCVcc is an HCV chimeric genome RNA in which a 5' non-coding region of the JFH-1 strain, sequences encoding the respective proteins of the core protein, the E1 protein, the E2 protein, the p7 protein and the NS2 protein of the H77 strain, sequences encoding the respective proteins of the NS3 protein, the NS4A protein, the NS4B protein, the NS5A protein and the NS5B protein and a 3' non-coding region of the JFH-1 strain are linked in this order.

Chimeric genome RNA used in the production of the TH/JFH-1 HCVcc is an HCV chimeric genome RNA in which a 5' non-coding region of the JFH-1 strain, sequences encoding the respective proteins of the core protein, the E1 protein, the E2 protein and the p7 protein, and a sequence encoding a region from the N-terminus to an amino acid residue at position 33 of the NS2 protein, of the TH strain, and a sequence encoding an amino acid residue at position 34 to the C-terminus of the NS2 protein, sequences encoding the respective proteins of the NS3 protein, the NS4A protein, the NS4B protein, the NS5A protein and the NS5B protein, and a 3' non-coding region, of the JFH-1 strain are linked in this order. The amino acid sequence of a precursor protein of the TH/JFH-1 HCVcc is set forth in SEQ ID NO: 41.

Chimeric genome RNA used in the production of the S310/JFH-1 HCVcc is an HCV chimeric genome RNA in which a 5' non-coding region of the JFH-1 strain, sequences encoding the respective proteins of the core protein, the E1 protein, the E2 protein, the p7 protein and the NS2 protein of the S310 strain (corresponding to amino acids 1 to 1032), and sequences encoding the respective proteins of the NS3 protein, the NS4A protein, the NS4B protein, the NS5A protein and the NS5B protein (corresponding to amino acids 1031-3034) and a 3' non-coding region of the JFH-1 strain are linked in this order.

As described above, the chimeric HCV particles (HCVcc) of each genotype produced herein has a structural gene portion derived from the genome RNAs of the HCV strains other than the JFH-1 strain. The phenotype exhibited by the cell infection of the chimeric HCV particles (HCVcc) of each genotype is genotype 2a for the J6/JFH-1 HCVcc, genotype 1a for the H77/JFH-1 HCVcc, genotype 3a for S310/JFH-1 HCVcc, and genotype 1b for the TH/JFH-1 HCVcc.

### 2. Measurement of Infection Inhibiting Activity

To a known human anti-E2 protein antibody MBL-HCV1 (Broering et al., J. Viol., 2009, vol. 83, pp. 12473-12482), or each of the scFvs or IgG antibodies of the present invention having been diluted with PBS, a dilution containing the chimeric HCV particles (HCVcc) of each genotype (namely, the J6/JFH-1 HCVcc (genotype 2a), the H77/JFH-1 HCVcc (genotype 1a), the S310/JFH-1 HCVcc (genotype 3a) or the TH/JFH-1 HCVcc (genotype 1b)) in PBS was added at the multiplicity of infection (moi) of 0.1, and they were reacted at room temperature for 30 minutes to prepare an antibody-HCVcc mixed solution.

Thereafter, a culture supernatant was removed from a 48-well plate, in which the Huh-7 cells (2 x 10⁴ cells/well; 10% FCS-DMEM medium (containing 1% MEM nonessential amino acid solution (Thermo Fisher Scientific), 10 mmol/L HEPES-Tris (pH 7.3) and 1 mmol/L sodium pyruvate)) had been seeded and cultured, and the antibody-HCVcc mixed solution was added thereto at 100 µL/well, and incubated in a 5% CO₂ incubator at 37°C for 3 hours. After 3 hours, a culture supernatant was removed and replaced with 500 µL of D-MEM, and the cells were further cultured in a 5% CO₂ incubator at 37°C for 72 hours. After the culture, the D-MEM was removed, the cells were washed with PBS, and the passive lysis buffer (Promega) was added thereto at 100 µL/well to prepare a cell lysate, and the lysate was collected in a screw cap tube. HCV core protein in the collected cell lysate was quantified using Lumipulse G1200 (Fujirebio Inc., Ortho Clinical Diagnostics). On the basis of a molar concentration of the HCV core protein (amount of core protein) determined by the measurement, the HCVcc infection inhibiting activity of each antibody (scFv or IgG antibody) was calculated. Specifically, as an evaluation value of the infection inhibiting activity of each antibody, a ratio (%) of the amount of the HCV core protein in the cells to which the HCVcc had been added in the presence of the antibody, to the amount of the HCV core protein in cells having been infected with the chimeric HCV particles (HCVcc) without adding any antibody (control value: "0 µg/mL" on the abscissa axis in the Figures) which was taken as 100%, was calculated. That is, as the amount of the core protein in the cells (namely, % against the control) is smaller, it means that the infection inhibiting activity of the antibody is higher.

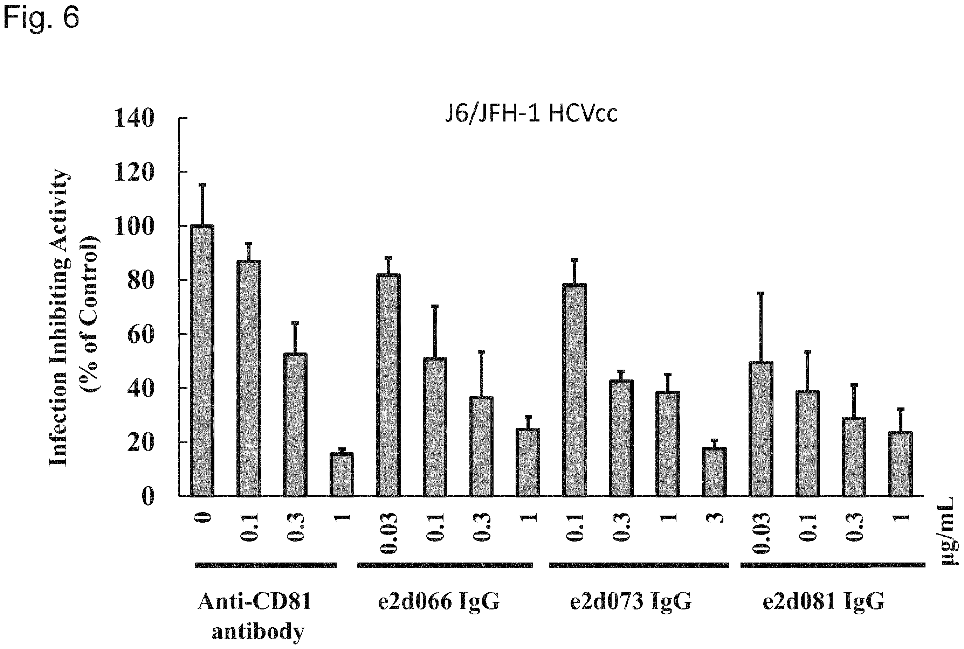

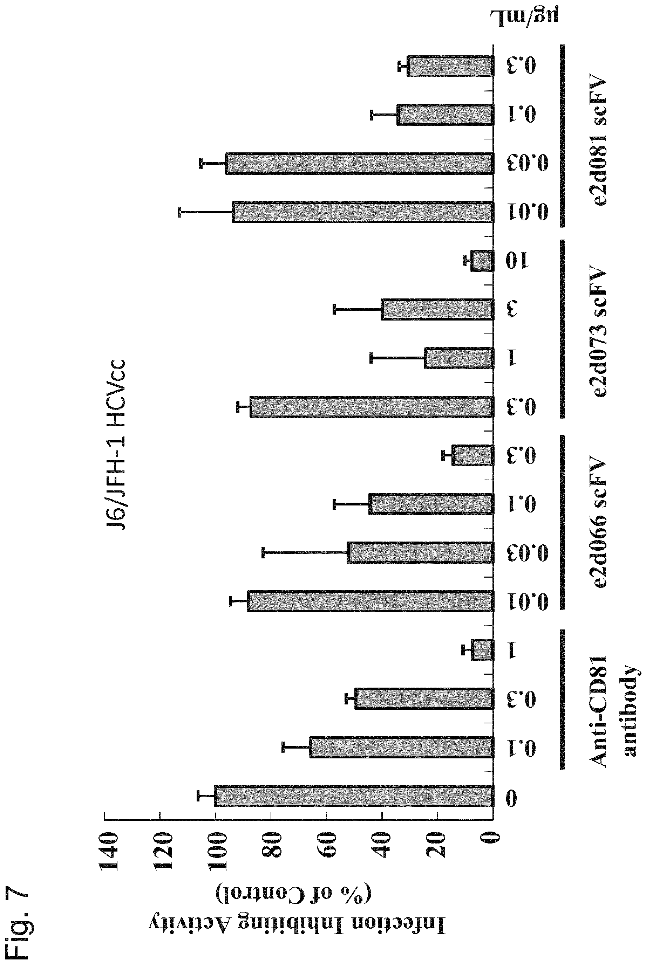

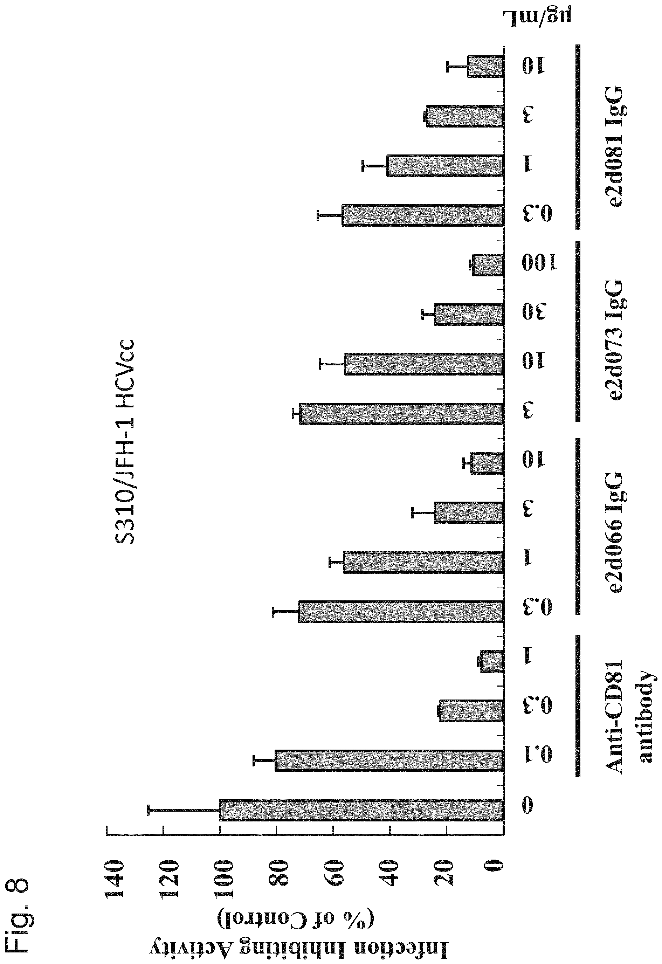

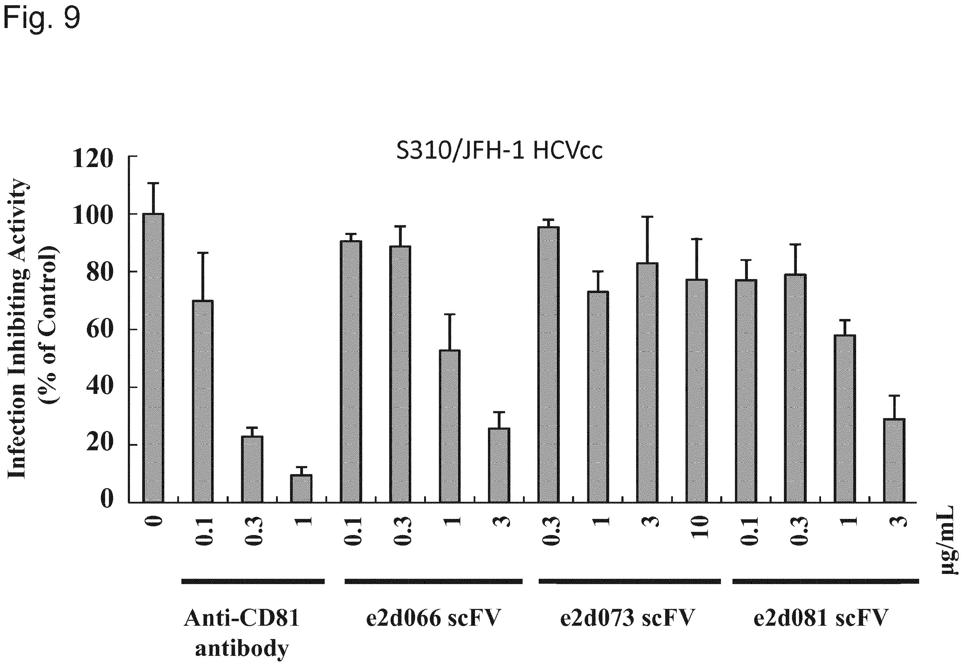

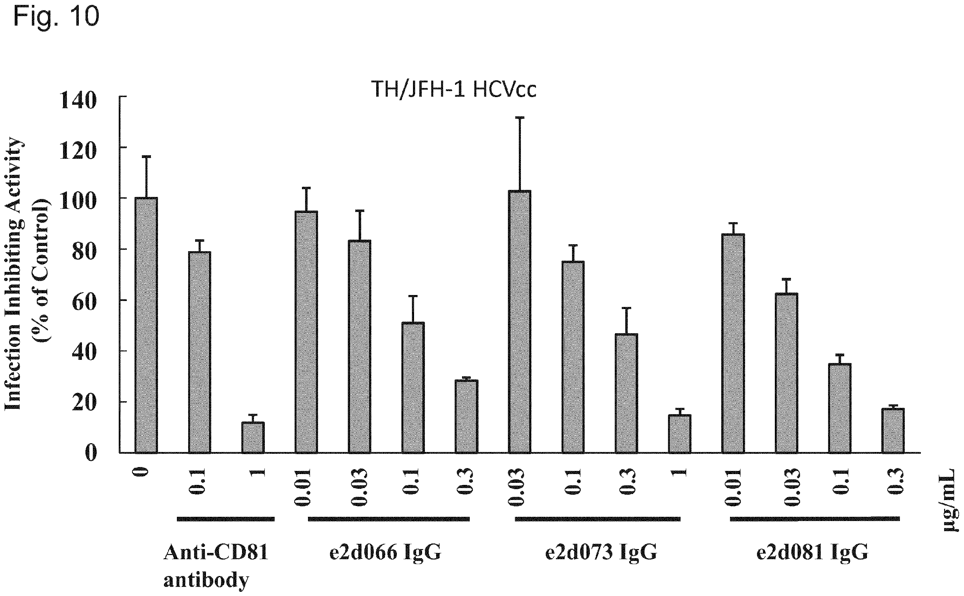

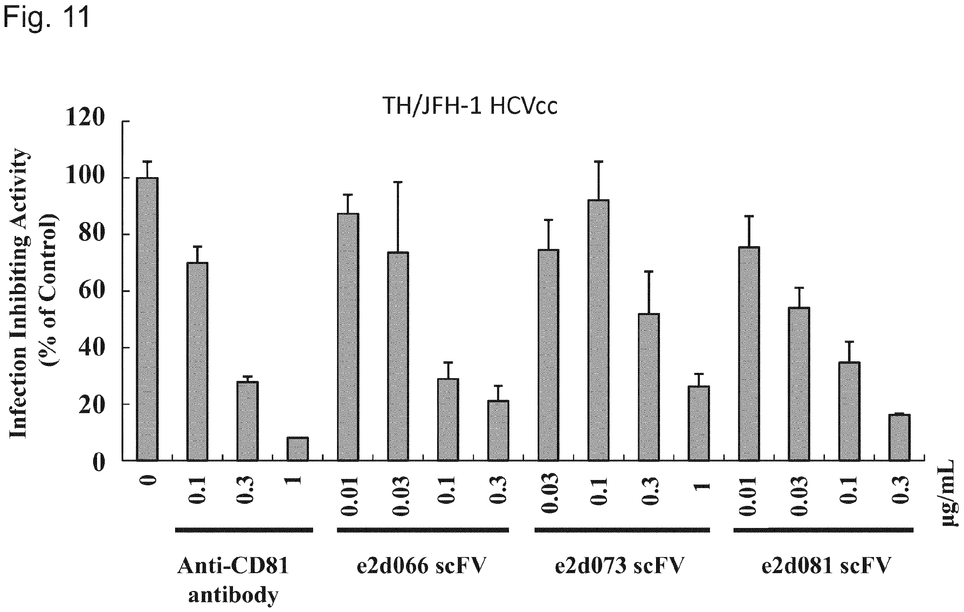

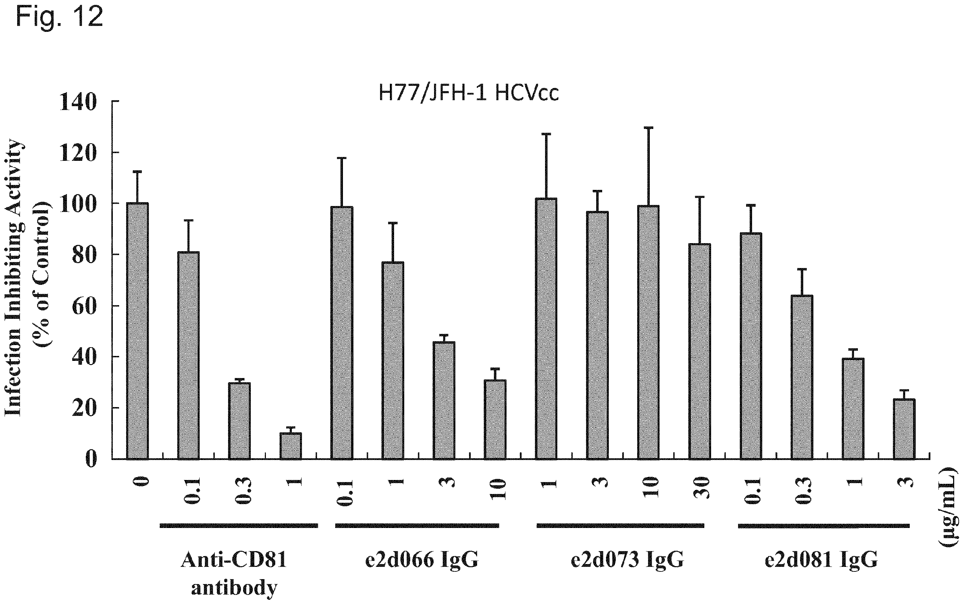

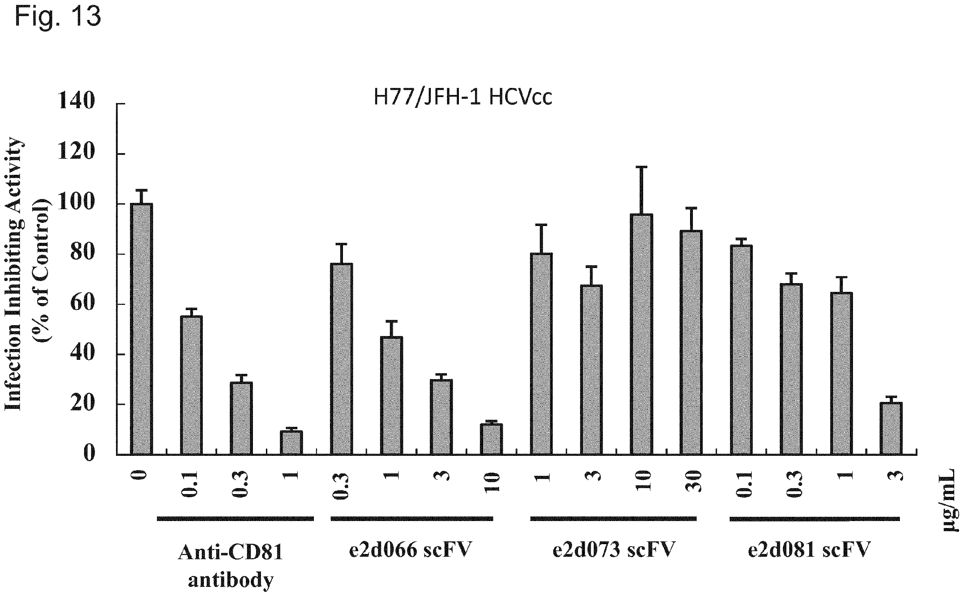

The infection inhibiting activities of the IgG antibodies and scFvs against the HCVcc of genotype 2a (J6/JFH-1 HCVcc) are shown in Figures 6 and 7, respectively, the infection inhibiting activities of the IgG antibodies and scFvs against the HCVcc of genotype 3a (S310/JFH-1 HCVcc) are shown in Figures 8 and 9, respectively, the infection inhibiting activities of the IgG antibodies and scFvs against the HCVcc of genotype 1b (TH/JFH-1 HCVcc) are shown in Figures 10 and 11, respectively, and the infection inhibiting activities of the IgG antibodies and scFvs against the HCVcc of genotype 1a (H77-JFH-1 HCVcc) are shown in Figures 12 and 13, respectively. The ordinate axis indicates the infection inhibiting activity. The abscissa axis indicates the respective antibodies allowed to act and the concentrations thereof (the final concentrations after addition to the cells).

The values of IC₅₀ indicating the infection inhibiting activities, against the HCVcc of each genotype, for the MBL-HCV1 antibody, the IgG antibodies and the scFvs of the present invention are shown in Table 2.

**[Table 2]**

| Antibody | IC₅₀ (µg/mL) | | | |
|---|---|---|---|---|
| | Genotype 1a (H77) | Genotype 1b (TH) | Genotype 2a (J6CF) | Genotype 3a (S310) |
| e2d066 IgG | 3.30 | 0.11 | 0.17 | 1.03 |
| e2d073 IgG | >30 | 0.25 | 0.42 | 10.4 |
| e2d081 IgG | 0.66 | 0.05 | 0.02 | 0.50 |
| e2d066 scFv | 3.13 | 0.06 | 0.02 | 1.23 |
| e2d073 scFv | >30 | 0.35 | 1.03 | >10 |
| e2d081 scFv | 1.38 | 0.04 | 0.23 | 1.14 |
| MBL-HCV1 | 2.20 | 0.74 | 0.86 | 7.26 |

These results revealed that both the scFvs (the e2d066 scFv, the e2d073 scFv and the e2d081 scFv) and the IgG antibodies (the e2d066 IgG, the e2d073 IgG and the e2d081 IgG) of the present invention produced as described above have strong infection inhibiting activity against the chimeric HCV particles (HCVcc) of a plurality of genotypes. In particular, the e2d066 IgG antibody and scFv containing the VH region (SEQ ID NO: 2) and the VL region (SEQ ID NO: 4) of the e2d066, and the e2d081 IgG antibody and scFv containing the VH region (SEQ ID NO: 2) and the VL region (SEQ ID NO: 22) of the e2d081 exhibited the infection inhibiting activity against the HCV of genotypes 1a, 1b, 2a and 3a, and exhibited higher infection inhibiting activity than the anti-CD81 antibody (Emmanuel et al., Proc. Natl. Acad. Sci. U.S.A., 2004, vol. 101, pp. 7270-7274) and the MBL-HCV1 antibody. The anti-CD81 antibody that is an antibody against a cell receptor is known to have infection inhibiting activity against genotype 2a (J6/JFH-1 HCVcc) and genotype 1b (TH/JFH-1HCVcc). This indicates that the antibody and the antigen-binding antibody fragment of the present invention have an effect of inhibiting the HCV infection itself, and hence have an effect of preventing reinfection of HCV (recurrence of hepatitis C) and the like in a liver transplant patient. Therefore, the antibody and the antigen-binding antibody fragment of the present invention can be applied as a preventive agent for hepatitis C to be used in liver transplantation, particularly for preventing recurrence of hepatitis C during living liver transplantation in a chronic hepatitis C patient.

### (Example 5) Evaluation of Escape Mutant Emergence Suppressive Property of IgG Antibodies

Each of the IgG antibodies (the e2d066 IgG, the e2d073 IgG and the e2d081 IgG) was evaluated for the escape mutant emergence suppressive property.

The evaluation of the escape mutant emergence suppressive property was performed in accordance with a method sufficiently described in Meital et al., Proc. Natl. Acad. Sci. U.S.A., 2008, vol. 105, pp. 19450-19455.

Each IgG antibody diluted with PBS and the J6/JFH-1 HCVcc were mixed to react at 37°C for 1 hour, thereby preparing an antibody-HCVcc mixed solution.

Thereafter, the antibody-HCVcc mixed solution was added to Huh-7 cells seeded in a 12-well plate (medium: 10% FCS-DMEM (containing 1% MEM nonessential amino acid solution (Thermo Fisher Scientific), 10 mmol/L HEPES-Tris (pH 7.3), and 1 mmol/L sodium pyruvate)) to infect the cells with the HCVcc. Three days after the infection, the cells were subcultured into a 6-well plate, and a culture supernatant was collected on the 3rd and 6th days of the subculture. It was confirmed that the collected culture supernatant contained the J6/JFH-1 HCVcc by an infectious titer measurement which is similar to a method for measuring an "infectious titer of J6/JFH-1 HCVcc contained in a culture supernatant finally collected" as described below. After confirming the infectious titer, the culture supernatant (the culture supernatant collected on the 3rd day or 6th day) was used for subsequent infection.

The collected culture supernatant confirmed for the infectious titer (containing the J6/JFH-1 HCVcc produced through infection culture) was mixed again with the IgG antibody, and the mixed solution was added to uninfected Huh-7 cells to infect the cells with the HCVcc. This steps were repeated 8 times (the number of repeating the subculture infection: 8 in total).

The infectious titer of the J6/JFH-1 HCVcc contained in the culture supernatant finally collected (hereinafter referred to as the J6/JFH-1 HCVcc-8W) was measured as follows: On a previous day of the measurement of the infectious titer, Huh-7 cells were seeded in a poly-D-lysine-coated 96-well plate (Coming) at 1 x 10⁴ cells/well. The J6/JFH-1 HCVcc-8W was added to each well to culture at 37°C for 72 hours. After the culture, a culture supernatant was removed, the cells were washed with PBS, and the cells were immobilized by treating with methanol at -20°C for 20 minutes. The immobilized cells were blocked by a Block Ace solution (DS Pharma Biomedical) for 1 hour, and washed with PBS, and thereafter, an anti-core antibody (2H9; Wakita, T. et al., Nat. Med., 2005, vol. 11, pp. 791-796) was added to a concentration of 10 µg/mL, and incubated at room temperature for 1 hour. After removing a supernatant and washing the cells, Alexa Fluor 488-conjugated anti-mouse IgG (Molecular Probes) was added thereto and incubated at room temperature for 1 hour. After washing the cells, 50 µL of PBS was added to each well, and the cells were observed with a fluorescent microscope. The number of fluorescent cells was counted and the infectious titer was shown as focus forming units/mL (ffu/mL).

The infection inhibiting activity of the IgG antibody against the J6/JFH-1 HCVcc-8W, or J6/JFH-1 HCVcc that is a parent strain (comparative control), was measured for detecting an escape mutant. First, to 60 µL of the J6/JFH-1 HCVcc-8W or the J6/JFH-1 HCVcc (both 100 ffu), the same volume of diluted IgG antibody was added, and the resultant was incubated at 37°C for 1 hour to prepare an antibody-HCVcc mixed solution. A culture supernatant was removed from Huh-7 cells, which had been seeded in each well of an 8-well chamber slide (Thermo Fisher Scientific), and 100 µL of the antibody-HCVcc mixed solution was added thereto and the cells were cultured at 37°C for 24 hours. Thereafter, a culture supernatant was removed, and 200 µL of 10% FCS-DMEM medium (containing 1% MEM nonessential amino acid solution (Thermo Fisher Scientific), 10 mmol/L HEPES-Tris (pH 7.3) and 1 mmol/L sodium pyruvate) was added to each well and the cells were further cultured for 48 hours. After the culture, a fluorescent labelled antibody was allowed to act on the cells of each well in the same manner as in the measurement of the infectious titer as described above, and the number of fluorescent cells was counted and the infectious titer was determined. The percentage (%) of the infectious titer shown in the cells to which each antibody-HCVcc mixed solution was added, to the infectious titer shown in the cells infected with the J6-JFH-1 HCVcc-8W or the J6-JFH-1 HCVcc without adding any antibody which was taken as 100%, was calculated, and a 50% infection inhibiting concentration was indicated as IC₅₀ (µg/mL). As a value of IC₅₀ is smaller, it means that the infection inhibiting activity by the antibody is higher.

The results are shown in Table 3. In this table, "J6-JFH-1 HCVcc" shows the infection inhibiting concentration IC₅₀ of each antibody against the parent strain J6/JFH-1 HCVcc, and "J6/JFH-1 HCVcc-8W" shows the infection inhibiting concentration IC₅₀ of each antibody against the J6/JFH-1 HCVcc-8W.

**[Table 3]**

| Antibody | IC₅₀ (µg/mL) | |
|---|---|---|
| | J6/JFH-1 HCVcc | J6/JFH-1 HCVcc-8 w |
| e2d066 IgG | <0.010 (0.003) | <0.010 |
| e2d073 IgG | 0.086 | 59.40 |
| e2d081 IgG | <0.010 (0.003) | 0.670 |
| MBL-HCV1 | 0.16 | >100 |

As shown in Table 3, when the known human anti-E2 protein antibody MBL-HCV1 (Broering et al., J. Viol. 2009, vol. 83, pp. 12473-12482) was evaluated in the same manner as described above, it did not have infection inhibiting activity against the J6/JFH-1 HCVcc-8W even at a concentration of 100 µg/mL, and an escape mutant was found to emerge.

In contrast, the infection inhibiting activities of the e2d066 IgG and the e2d081 IgG against the J6/JFH-1 HCVcc-8W were almost equivalent to or slightly lower than their infection inhibiting activities against the J6/JFH-1 HCVcc. In other words, with respect to the e2d066 IgG and the e2d081 IgG, the emergence of an escape mutant was suppressed even through the repeated subculture infection of 8 times, and it was revealed that these antibodies exhibit the escape mutant emergence suppressive property. In particular, the e2d066 IgG was revealed to exhibit very strong escape mutant emergence suppressive property because no escape mutant emerged.

The amino acid sequence (SEQ ID NO: 2) of the VH region of the e2d066 IgG is identical to the amino acid sequence of the VH region of the e2d081 IgG. Accordingly, it was suggested that there is a possibility that this VH region (SEQ ID NO: 2), and particularly, the VH region comprising the CDR1 shown by SEQ ID NO: 5, the CDR2 shown by SEQ ID NO: 6 and the CDR3 shown by SEQ ID NO: 7, contributes to the escape mutant emergence suppressive property.

### (Example 6) Epitope Analysis

In order to determine whether or not each of the IgG antibodies (the e2d066 IgG, the e2d073 IgG and the e2d081 IgG) recognizes a conformational epitope, it was tested whether or not the IgG antibodies recognized a denatured E2 protein. It can be said that an antibody capable of binding to a denatured E2 protein is an antibody recognizing a linear epitope of the E2 protein, while an antibody incapable of binding to a denatured E2 protein is an antibody recognizing a conformational epitope of the E2 protein.

As control antibodies, the MBL-HCV1 antibody recognizing a linear epitope of the HCV E2 protein and the AR3A antibody (International Publication No. WO2010/047830) recognizing a conformational epitope were also similarly analyzed.

### 1. Production of TH E2-Fc Protein (Fusion Protein of E2 Protein of TH Strain attached to Fc protein of Human IgG)

This protein was produced in accordance with a method described in International Publication No. WO2010/038789.

Using cDNA of the genome RNA of the TH strain (genotype 1b) (Wakita, T. et al., J. Biol. Chem., 1994, vol. 269. pp. 14205-14210, International Publication No. WO2006/022422) as a template, a gene encoding a protein consisting of the region corresponding to amino acid residues from position 384 to position 717 (corresponding to the E2 protein) on the assumption that the initiating methionine at the N-terminus of the precursor protein of the TH strain was in position 1, was amplified by PCR. The amplified DNA fragment was cloned into pCR-TOPO (Thermo Fisher Scientific). The gene fragment encoding the E2 protein was excised by digestion with *Hind*III and *Bam*HI, and was inserted in-frame between the *Hind*III site and the *Bam*HI site downstream of the signal peptide sequence in p3xFLAG-CMV-13 (Sigma-Aldrich). The thus obtained vector was named as CMV-13-THE2. The vector CMV-13-THE2 was digested with *Sac*I and *Bam*HI*,* DNA fragments encoding the signal peptide sequence and the E2 protein were separated through agarose gel electrophoresis, and purified using GeneElute (Sigma-Aldrich). Thereafter, the DNA fragments encoding the signal peptide sequence and the E2 protein were each inserted in-frame between the *Sac*I site and the *Bam*HI site in the vector CDM-mIL7R-Ig (Sudo et al., Proc. Natl. Acad. Sci. USA, 1993, vol. 90, pp. 9125-9129) expressing a chimeric protein consisting of a mouse IL-7 receptor-human immunoglobulin Fc region, to obtain the vector CDM-THE2Fc expressing an antigen E2 protein attached to an Fc domain of human immunogloblin (hereinafter referred to as the TH E2Fc protein).

The vector CDM-THE2Fc was transfected into COS1 cells by the DEAE dextran method to express the TH E2Fc protein. From a culture supernatant of the cells into which the CDM-THE2Fc had been introduced, the TH E2Fc protein was purified using Prosep-A (Millipore) that is a Protein-A-bound support.

### 2. Biotinylation of Each Antibody

Each of the antibodies (the e2d066 IgG, the e2d073 IgG, the e2d081 IgG, the AR3A antibody and the MBL-HCV1 antibody) was biotinylated. The biotinylation was performed using EZ-Link (registered trademark) Sulfo-NHS-LC-Biotin (Thermo Fisher Scientific), in accordance with the instructions attached thereto. 2.4 µL of 20 mmol/L Sulfo-NHS-LC-Biotin was added to 100 µL of each IgG antibody diluted with PBS to 0.1 mg/mL, and mixed and allowed to stand on ice for 2 hours. Next, the mixture was desalted with Zeba (trademark) Spin Desalting Columns (Thermo Fisher Scientific), and an unreacted biotin was removed, thereby preparing a biotinylated antibody.

### 3. Epitope Analysis

The TH E2-Fc protein was denatured by heat treatment at 95°C for 3 minutes in 50 mmol/L Tris-HCl (pH 7.0) containing 2% SDS and 5% 2ME.

Each of native (non-denatured) TH E2-Fc protein and the denatured TH E2-Fc protein was diluted with PBS to the concentration of 0.5 µg/mL, and added at 50 µL to each well of an immunoplate (Thermo Fisher Scientific), and was allowed to stand at 4°C overnight to immobilize the protein on the plate. A protein solution was removed, and 200 µL of a Blocking one solution (Nacalai Tesque) prepared in accordance with the attached manual was added to each well for blocking.

Next, the biotinylated antibody was 3-fold stepwise diluted with PBS, 50 µL of each serial dilution of antibody was added to each well of the immobilized plate to react at room temperature for 1 hour. After completing the reaction, the well was washed with PBS containing 0.05% Tween 20, and 50 µL of avidin-HRP (GE Healthcare) diluted 3000 times with PBS containing 0.05% Tween 20 was added to each well to react at room temperature for 1 hour. After completing the reaction, the well was washed with PBS containing 0.05% Tween 20, and color developed with ELISA POD substrate TMB solution (Nacalai Tesque), the reaction was stopped with 1 mol/L sulfuric acid, and absorbance (at 450 nm) was measured with a microplate reader.

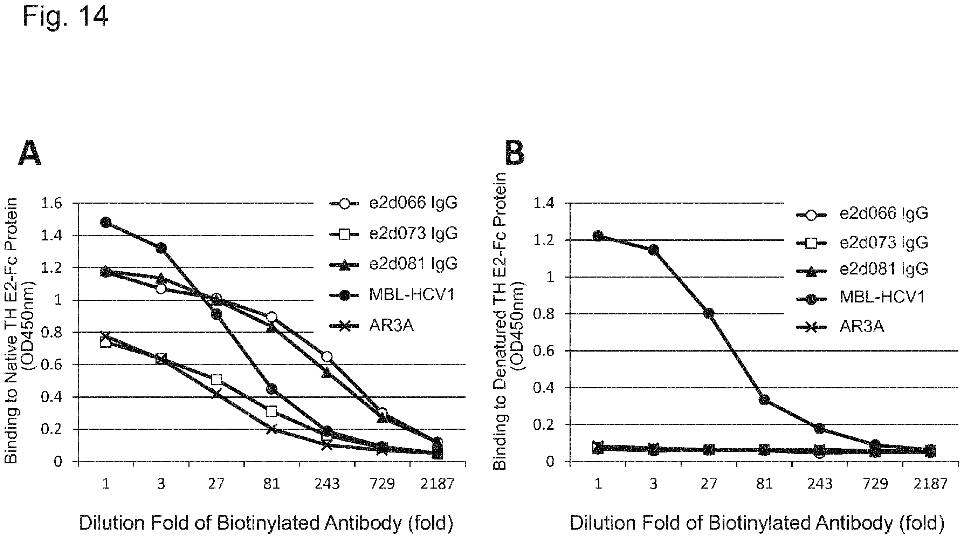

The results are shown in Figure 14. Figure 14A illustrates a binding property of each IgG antibody to the native TH E2-Fc protein, and Figure 14B illustrates a binding property of each IgG antibody to the denatured TH E2-Fc protein. The ordinate axis indicates the absorbance (at 450 nm), and the abscissa axis indicates the dilution ratio of the biotinylated antibody.

None of the e2d066 IgG, the e2d073 IgG, the e2d081 IgG and the AR3A antibody bound to the denatured TH E2-Fc protein, while the MBL-HCV1 antibody was reacted with both the denatured and native TH E2-Fc proteins (Figure 14).

Accordingly, it was suggested that the e2d066 IgG, the e2d073 IgG and the e2d081 IgG recognize a conformational epitope, unlike the MBL-HCV1 antibody.

### (Example 7) Epitope Analysis with Linear Peptide

It was suggested in Example 6 that the e2d066 IgG, the e2d073 IgG and the e2d081 IgG recognize a conformational epitope. Therefore, a group of linear peptides each consisting of 12 amino acid residues was used for verifying that these antibodies recognize a conformational epitope.

With respect to the amino acid sequence (SEQ ID NO: 36) corresponding to the E2 protein of the TH strain (i.e., the amino acid sequence corresponding to the amino acid residues from position 384 to position 717 on the assumption that the initiating methionine at the N-terminus of the precursor protein of the TH strain was in position 1), a series of peptides (peptides Nos. 1 to 82) of 12 continuous amino acid sequences designed to be shifted by 3 amino acids from the N-terminus were synthesized. Each peptide had the N-terminus biotinylated, and had glycine amide at the C-terminal.

Each peptide was dissolved in DMSO, and then dissolved in PBS to a concentration of 0.01 nmol/µL. The peptide solution was added at 50 µL to each well of a streptavidin coated plate (Thermo Fisher Scientific) to react at room temperature for 2 hours. The peptide solution was removed, and 200 µL of a Blocking one solution (Nacalai Tesque) prepared in accordance with the attached manual was added to each well, and the resultant was allowed to stand at 4°C overnight for blocking.

The blocking solution was removed, the well was washed with PBS containing 0.05% Tween 20 four times, and each antibody diluted to 1 µg/mL with PBS containing 0.05% Tween 20 was added at 50 µL to each well to react at room temperature for 1.5 hours. After completing the reaction, the antibody solution was removed, the well was washed with PBS containing 0.05% Tween 20 four times, and then an HRP-labeled anti-human IgG goat antibody (GE Healthcare) diluted 5000 times with PBS containing 0.05% Tween 20 was added at 50µL/well to react at room temperature for 1 hour. After the reaction, the antibody solution was removed, and the well was washed with PBS containing 0.05% Tween 20 five times. After washing, the well was color developed using a peroxidase coloring kit, and the absorbance was measured at 450 nm, thereby detecting an antibody binding to the peptide.

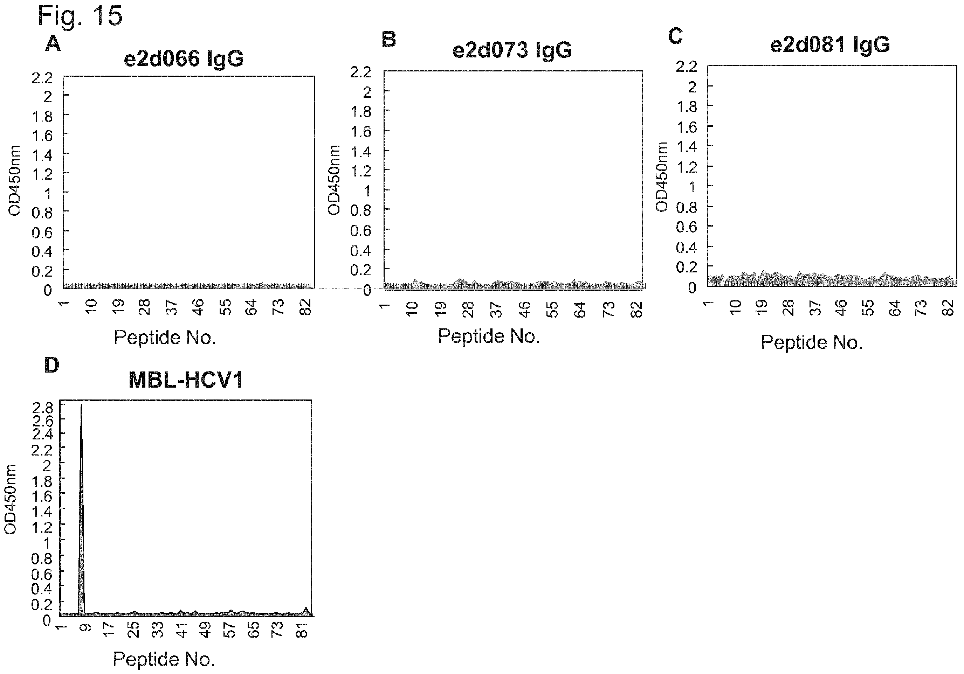

As a result, the MBL-HCV1 antibody bound to QLINTNGSWHIN (SEQ ID NO: 37) (Figure 15D), while none of the e2d066 IgG, the e2d073 IgG and the e2d081 IgG bound to any of the peptides (Figures 15A, 15B and 15C). In this manner, it was revealed that the MBL-HCV1 antibody recognizes a linear epitope and that the e2d066 IgG, the e2d073 IgG and the e2d081 IgG recognize a conformational epitope.

### (Example 8) Comparison of Epitopes by Competition ELISA

It was examined through competition ELISA whether or not the e2d066 IgG recognizing a conformational epitope recognizes the same epitope as the e2d073 IgG, the e2d081 IgG and the AR3A antibody recognizing a conformational epitope.

As control antibodies, a neutralizing antibody AR3A recognizing a conformational epitope, a neutralizing antibody MBL-HCV1 recognizing a linear epitope: QLINTNGSWHIN (SEQ ID NO: 37) (corresponding to a portion of the E2protein of the TH strain from Q at position 412 to N at position 423), and a non-neutralizing antibody 8D10-3 (International Publication No. WO2010/038789) recognizing a linear epitope (from D at position 522 to N at position 534) were used.

To each of the antibodies (3-fold stepwise diluted from 20 µg/mL), an equivalent amount of the biotinylated e2d066 IgG (described in Item 2 of Example 6) was added followed by stirring, and then, the resultant was added to an ELISA plate (Thermo Fisher Scientific) in which the TH E2-Fc protein (described in Example 6 above) had been immobilized (0.5 µg/well). After 1 hour, the plate was washed with PBS containing 0.05% Tween 20, and avidin-HRP (GE Healthcare) diluted 3000 times was added thereto to react. After 1 hour, the plate was washed with PBS containing 0.05% Tween 20, and color developed with ELISA POD substrate TMB solution (Nacalai Tesque), and the reaction was stopped with 1 mmol/L sulfuric acid, and absorbance (at 450 nm) was measured using a microplate reader.

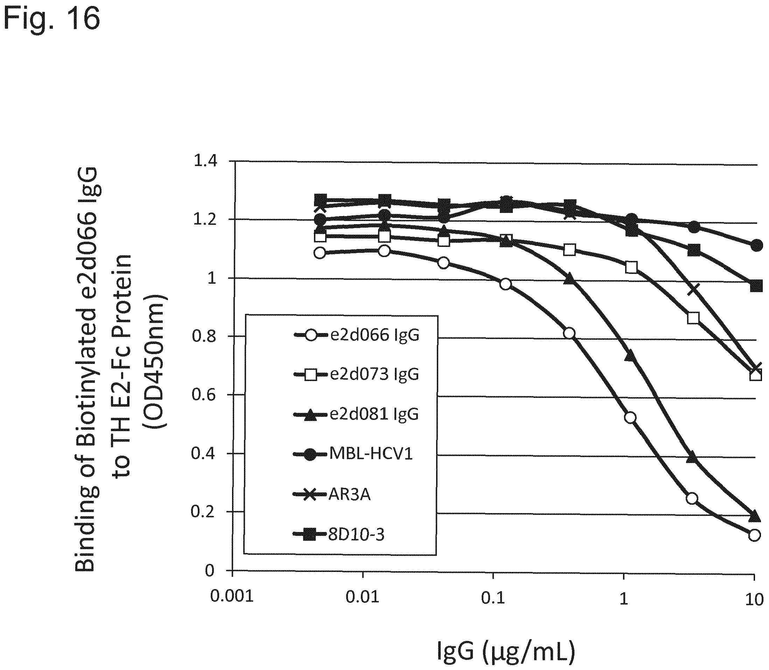

The results are shown in Figure 16. The ordinate axis of this Figure indicates the absorbance (at 450 nm), and the abscissa axis indicates the concentration of each antibody.

The binding of the biotinylated e2d066 IgG to the TH E2-Fc protein was inhibited by the e2d066 IgG and the e2d081 IgG, depending on the concentration of the antibody. Accordingly, it was revealed that the e2d066 IgG and the e2d081 IgG recognize the same epitope.

On the other hand, the e2d073 IgG, the AR3A antibody, the MBL-HCV1 antibody and the 8D10-3 antibody did not inhibit the binding of the biotinylated e2d066 IgG to the TH E2-Fc protein, and therefore, it was revealed that these antibodies recognize a different epitope from the e2d066 IgG.

The amino acid sequence of the VH region (SEQ ID NO: 2) of the e2d066 IgG is identical to the amino acid sequence of the VH region of the e2d081 IgG. Accordingly, it was revealed that there is a possibility that this VH region (the amino acid sequence: SEQ ID NO: 2), and in particular, the VH region containing the CDR1 shown by SEQ ID NO: 5, the CDR2 shown by SEQ ID NO: 6 and the CDR3 shown by SEQ ID NO: 7, contributes to the recognition of the same epitope.

Next, an HC-84.1 antibody (International Publication No. WO2013/033319) suggested to exhibit the escape mutant emergence suppressive property was produced on the basis of the literature by Krey et al. (PLOS Pathogens, e1003364, 2013), and was examined for whether the epitope is the same or different from the epitope of the e2d066 in a similar manner to the method described above. To a sample of each of the e2d066 IgG, the e2d081 IgG, the HC-84.1 antibody, the MBL-HCV1 antibody or the AR3A antibody 3-fold stepwise diluted from 20 µg/mL, an equivalent amount of the biotinylated e2d066 IgG (described in Item 2 of Example 6) was added followed by stirring, and then added to an ELISA plate (Thermo Fisher Scientific) in which the TH E2-Fc protein (described in Example 6 above) had been immobilized (0.5 µg/well). After 1 hour, the well was washed with PBS containing 0.05% Tween 20, and avidin-HRP (GE Healthcare) diluted 3000 times was added thereto to react. After 1 hour, the well was washed with PBS containing 0.05% Tween 20, and color developed with ELISA POD substrate TMB solution (Nacalai Tesque), and the reaction was stopped with 1 mmol/L sulfuric acid, and absorbance (at 450 nm) was measured using a microplate reader.

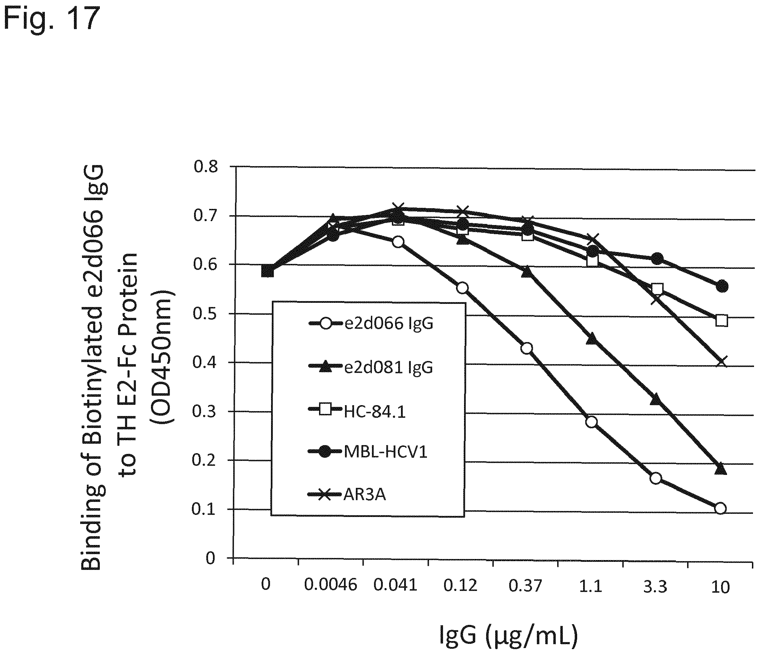

The results are shown in Figure 17. The ordinate axis of the Figure indicates the absorbance (at 450 nm) and the abscissa axis indicates the concentration of each antibody. The binding of the biotinylated e2d066 IgG to the TH E2-Fc protein was inhibited by the e2d066 IgG and the e2d081 IgG, depending on the concentration of the antibody.

On the other hand, the HC-84.1 antibody, the AR3A antibody and the MBL-HCV1 antibody did not inhibit the binding of the biotinylated ed2066 IgG to the TH E2-Fc protein, and it was thus revealed that the HC-84.1 antibody also recognizes a different epitope from the e2e066 IgG.

### (Example 9) Comparison of Binding Property of Various Monoclonal Antibodies to E2 Protein

The e2d066 IgG, the e2d081 IgG, the HC-84.1 antibody, the MBL-HCV1 antibody and the AR3A antibody were compared in the binding property to J6CF E2-Fc and the TH E2-Fc protein. A sample of each of the various monoclonal antibodies 10-fold stepwise diluted from 30 µg/mL was added to an ELISA plate (Thermo Fisher Scientific) in which the J6CF E2-Fc or TH E2-Fc protein (described in Example 6 above) had been immobilized (0.5 µg/well). After 1 hour, the well was washed with PBS containing 0.05% Tween 20, and then goat anti-human IgG F(ab')₂-HRP (Thermo Fisher Scientific) diluted 5000 times was added thereto to react. After 1 hour, the resultant was washed with PBS containing 0.05% Tween 20, and color developed with ELISA POD substrate TMB solution (Nacalai Tesque), and the reaction was stopped with 1 mol/L sulfuric acid, and absorbance (at 450 nm) was measured using a microplate reader.

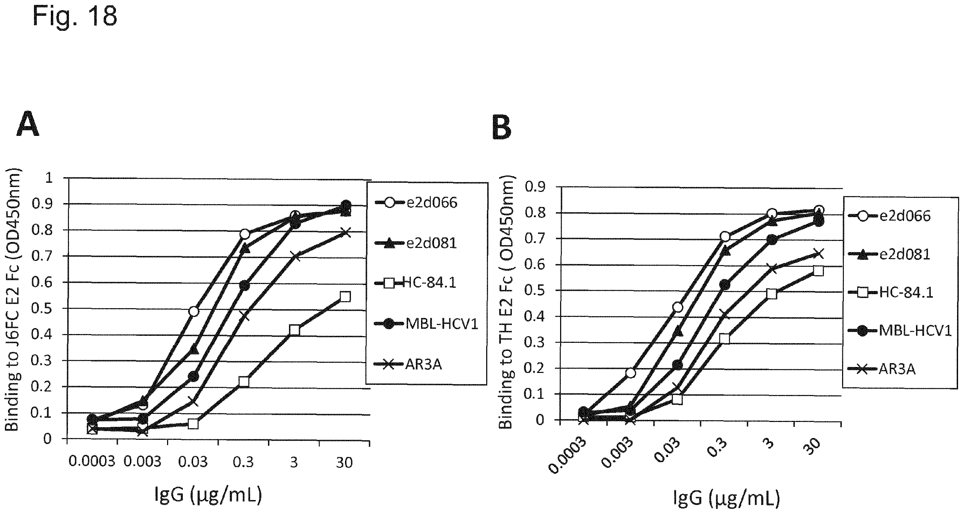

The results are shown in Figure 18. It was revealed that the e2d066 IgG and the e2d081 IgG have higher binding properties to the E2 proteins of the J6CF strain of genotype 2a and the TH strain of genotype 1b than the HC-84.1 antibody, the MBL-HCV1 antibody and the AR3A antibody.

### (Example 10) Inhibiting Effect of IgG Antibody against Infection Expansion of Hepatitis C Virus (HCV)

The effect of the e2d066 IgG on the HCV infection expansion was evaluated by treating, with the e2d066 IgG, cells infected with the HCVcc of genotype 2a (J6/JFH-1 HCVcc). For comparison, Telaprevir (VX-950) that is an inhibitor against protease activity of NS3 protein and interferon-α (IFN-α) were also evaluated.

On the previous day of the evaluation, Huh-7 cells (4 x 10⁴ cells/well; 10% FCS-DMEM medium (containing 1% MEM nonessential amino acid solution (Thermo Fisher Scientific), 10 mmol/L HEPES-Tris (pH7.3), and 1 mmol/L sodium pyruvate) were seeded and cultured in a 12-well plate. The HCVcc of genotype 2a (J6/JFH-1 HCVcc) produced in Item 1 of Example 4 described above was prepared with PBS at moi of 0.03, and then added to the Huh-7 cells, and thereafter, the Huh-7 cells were cultured in a 5% CO₂ incubator at 37°C for 4 hours.

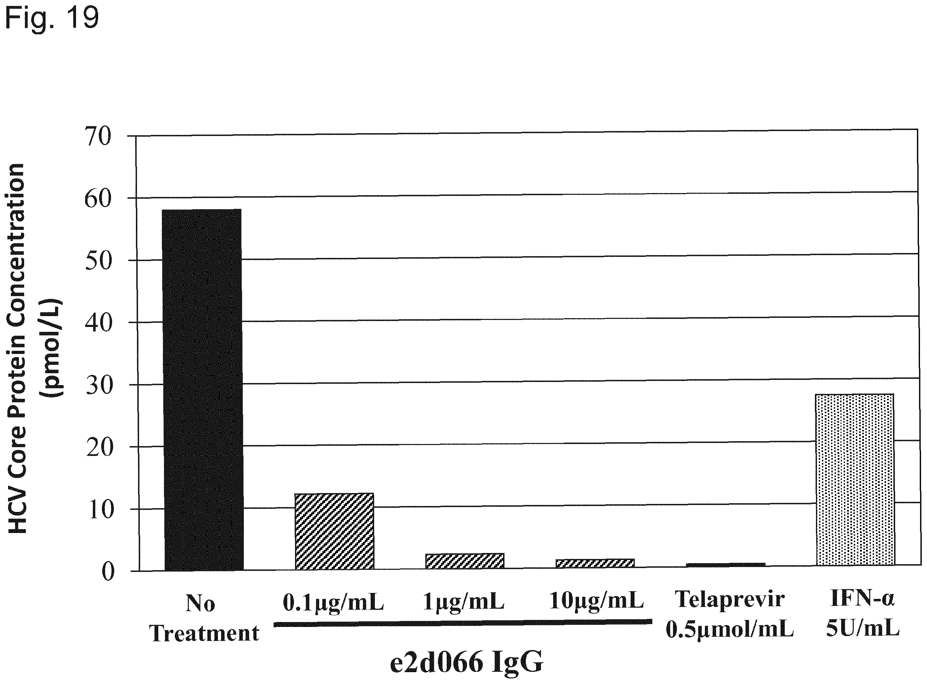

After 4 hours, a culture supernatant was removed and replaced with D-MEM containing the e2d066 IgG, Telaprevir (VX-950) or the IFN-α, and further cultured in a 5% CO₂ incubator at 37°C for 96 hours. Cells similarly cultured with the medium replaced with D-MEM containing none of the antibody and agents were also examined as a control ("No Treatment" on the abscissa axis of Figure 19). After the culture, a cell culture supernatant was collected. The HCV core protein contained in the collected cell culture supernatant was quantified using Lumipulse G1200 (Fujirebio Inc., Ortho Clinical Diagnostics).

The results are shown in Figure 19. The ordinate axis indicates the concentration (pmol/L) of the HCV core protein in the cell culture supernatant, and a smaller value indicates higher infection inhibiting activity. The abscissa axis indicates, from left to right, "No Treatment", and the antibody e2d066 IgG, the agent Telaprevir and IFN-α used for the treatment; and the concentrations (final concentrations after addition to the cells) thereof.

The treatment with the e2d066 IgG exhibited significantly lower concentrations of the HCV core protein in the cell culture supernatant as compared with the control ("No Treatment"), and the effect is equivalent to or higher than the effects of Telaprevir (VX-950) and IFN-α. Thus, the ed2066 IgG had an effect to inhibit the HCV production in HCV infected cells, or an effect to inhibit infection from HCV infected cells to surrounding HCV uninfected cells.

Accordingly, it was revealed that the antibody and the antigen-binding antibody of the present invention can inhibit the infection itself of cells with HCV as well as can suppress the HCV infection expansion even after establishment of the infection with HCV. Since the antibody and the antigen-binding antibody of the present invention have an effect to inhibit the HCV infection itself, they were revealed to have a preventive effect for, e.g., reinfection of HCV (recurrence of hepatitis C) in a liver transplant patient. Further, since they have an effect to suppress the HCV infection expansion after establishment of the infection with HCV, they were revealed to have a therapeutic effect in an HCV infection carrier, namely, a therapeutic effect for hepatitis C.

### Industrial Applicability

Since the antibody or the antigen-binding antibody fragment of the present invention has high HCV infection inhibiting activity and HCV infection expansion suppressing effect, it can be expected to be applied to a therapeutic agent or a preventive agent for hepatitis C, and in particular, to prevention of recurrence of hepatitis during living liver transplantation of a chronic hepatitis C patient.

All the publications, patents and patent applications cited herein are incorporated herein by reference.

### Free Text of Sequencing Listing

SEQ ID NO: 1: nucleotide sequence encoding VH region of e2d066 (and e2d081)
SEQ ID NO: 2: amino acid sequence of VH region of e2d066 (and e2d081)
SEQ ID NO: 3: nucleotide sequence encoding VL region of e2d066
SEQ ID NO: 4: amino acid sequence of VL region of e2d066
SEQ ID NO: 5: amino acid sequence of CDR1 of VH region of e2d066 (and e2d081)
SEQ ID NO: 6: amino acid sequence of CDR2 of VH region of e2d066 (and e2d081)
SEQ ID NO: 7: amino acid sequence of CDR3 of VH region of ed2066 (and e2d081)
SEQ ID NO: 8: amino acid sequence of CDR1 of VL region of e2d066
SEQ ID NO: 9: amino acid sequence of CDR2 of VL region of e2d066
SEQ ID NO: 10: amino acid sequence of CDR3 of VL region of e2d066
SEQ ID NO: 11: nucleotide sequence encoding VH region of e2d073
SEQ ID NO: 12: amino acid sequence of VH region of e2d073
SEQ ID NO: 13: nucleotide sequence encoding VL region of e2d073
SEQ ID NO: 14: amino acid sequence of VL region of e2d073
SEQ ID NO: 15: amino acid sequence of CDR1 of VH region of e2d073
SEQ ID NO: 16: amino acid sequence of CDR2 of VH region of e2d073
SEQ ID NO: 17: amino acid sequence of CDR3 of VH region of e2d073
SEQ ID NO: 18: amino acid sequence of CDR1 of VL region of e2d073
SEQ ID NO: 19: amino acid sequence of CDR2 of VL region of e2d073
SEQ ID NO: 20: amino acid sequence of CDR3 of VL region of e2d073
SEQ ID NO: 21: nucleotide sequence encoding VL region of e2d081
SEQ ID NO: 22: amino acid sequence of VL region of e2d081
SEQ ID NO: 23: amino acid sequence of CDR1 of VL region of e2d081
SEQ ID NO: 24: amino acid sequence of CDR2 of VL region of e2d081
SEQ ID NO: 25: amino acid sequence of CDR3 of VL region of e2d081
SEQ ID NO: 26: amino acid sequence of entire heavy chain of IgG antibody of e2d066 (e2d066 IgG)
SEQ ID NO: 27: amino acid sequence of entire light chain of IgG antibody of e2d066 (e2d066 IgG)
SEQ ID NO: 28: amino acid sequence of entire heavy chain of IgG antibody of e2d073 (e2d073 IgG)
SEQ ID NO: 29: amino acid sequence of entire light chain of IgG antibody of e2d073 (e2d073 IgG)
SEQ ID NO: 30: amino acid sequence of entire heavy chain of IgG antibody of e2d081 (e2d081 IgG)
SEQ ID NO: 31: amino acid sequence of entire light chain of IgG antibody of e2d081 (e2d081 IgG)
SEQ ID NO: 32: amino acid sequence of entire e2d066 scFv
SEQ ID NO: 33: amino acid sequence of entire e2d073 scFv
SEQ ID NO: 34: amino acid sequence of entire e2d081 scFv
SEQ ID NO: 35: amino acid sequence of linker
SEQ ID NO: 36: amino acid sequence of E2 protein of TH strain
SEQ ID NO: 37: amino acid sequence of TH E2 protein-derived peptide
SEQ ID NO: 38: amino acid sequence of CL region of e2d066
SEQ ID NO: 39: amino acid sequence of CL region of e2d073
SEQ ID NO: 40: amino acid sequence of CL region of e2d081
SEQ ID NO: 41: amino acid sequence of precursor protein of chimeric HCV particle of TH strain and JFH-1 strain (TH/JFH-1 HCVcc)

## Claims

1. An anti-hepatitis C virus E2 protein antibody or antigen-binding antibody fragment thereof, which has infection inhibiting activity against hepatitis C virus (HCV).

2. The antibody or antigen-binding antibody fragment according to claim 1, which exhibits an escape mutant emergence suppressive property.

3. The antibody or antigen-binding antibody fragment according to claim 1 or 2, wherein said antibody or antigen-binding antibody fragment comprises a heavy chain variable region of the following (a) or (b):
(a) a heavy chain variable region comprising a CDR1 consisting of the amino acid sequence shown by SEQ ID NO: 5, a CDR2 consisting of the amino acid sequence shown by SEQ ID NO: 6, and a CDR3 consisting of the amino acid sequence shown by SEQ ID NO: 7; or
(b) a heavy chain variable region comprising the amino acid sequence shown by SEQ ID NO: 2.

4. The antibody or antigen-binding antibody fragment according to any one of claims 1 to 3, wherein said antibody or antigen-binding antibody fragment comprises a light chain variable region of any one of the following (c) to (f):
(c) a light chain variable region comprising a CDR1 consisting of the amino acid sequence shown by SEQ ID NO: 8, a CDR2 consisting of the amino acid sequence shown by SEQ ID NO: 9 and a CDR3 consisting of the amino acid sequence shown by SEQ ID NO: 10;
(d) a light chain variable region comprising a CDR1 consisting of the amino acid sequence shown by SEQ ID NO: 23, a CDR2 consisting of the amino acid sequence shown by SEQ ID NO: 24 and a CDR3 consisting of the amino acid sequence shown by SEQ ID NO: 25;
(e) a light chain variable region comprising the amino acid sequence shown by SEQ ID NO: 4; and
(f) a light chain variable region comprising the amino acid sequence shown by SEQ ID NO: 22.

5. An antibody or antigen-binding antibody fragment, which recognizes the same conformational epitope as the antibody or antigen-binding antibody fragment according to claim 4.

6. The antibody or antigen-binding antibody fragment according to any one of claims 1 to 5, wherein said antibody or antigen-binding antibody fragment is an IgG, Fab, Fab', F(ab')₂, single chain antibody, dsFv, (scFv)₂, or single domain antibody.

7. The antibody or antigen-binding antibody fragment according to any one of claims 1 to 6, wherein said antibody is a human antibody or humanized antibody.

8. The antibody or antigen-binding antibody fragment according to any one of claims 1 to 7, wherein said antibody or antigen-binding antibody fragment is chemically modified.

9. A nucleic acid encoding the antibody or antigen-binding antibody fragment according to any one of claims 1 to 7.

10. A nucleic acid encoding a heavy chain variable region comprising a CDR1 consisting of the amino acid sequence shown by SEQ ID NO: 5, a CDR2 consisting of the amino acid sequence shown by SEQ ID NO: 6 and a CDR3 consisting of the amino acid sequence shown by SEQ ID NO: 7.

11. A medicament comprising the antibody or antigen-binding antibody fragment according to any one of claims 1 to 8 as an active ingredient.

12. The medicament according to claim 11, which is an agent for treatment or prevention of hepatitis C.


SEQUENCE LISTING

<110>  Japan as Represented by Director-General of National Institute of
       Infectious Diseases
       Institute for Antibodies Co., Ltd.
       Toray Industries, Inc.

<120>  Antibody having inhibitory activity against hepatitis C virus
       infection

<130>  PH-6117-PCT

<150>  JP 2014-058936
<151>  2014-03-20

<160>  41

<170>  PatentIn version 3.5

<210>  1
<211>  372
<212>  DNA
<213>  Artificial

<220>
<223>  DNA sequence of e2d066 or e2d081 VH region


<220>
<221>  CDS
<222>  (1)..(372)

<400>  1
cag gtg cag ctg gtg cag tct ggg tct gac gtg aag aag ccc ggg tcc        48
Gln Val Gln Leu Val Gln Ser Gly Ser Asp Val Lys Lys Pro Gly Ser
1               5                   10                  15

tcg gtg agg gtc tcc tgc aag gct tct gga ggc agc ttc aac agt tat        96
Ser Val Arg Val Ser Cys Lys Ala Ser Gly Gly Ser Phe Asn Ser Tyr
            20                  25                  30

gct gtg aac tgg gtg cgg cag gcc cct gga cag ggg ctt gaa tgg atg       144
Ala Val Asn Trp Val Arg Gln Ala Pro Gly Gln Gly Leu Glu Trp Met
        35                  40                  45

gga agg atc atg cct ttg gtt ggc tta cca gag tac gca gag agg ttc       192
Gly Arg Ile Met Pro Leu Val Gly Leu Pro Glu Tyr Ala Glu Arg Phe
    50                  55                  60

caa gag cga gtc acg ttt act gcg gac aaa tcc acg agg aca gcc tac       240
Gln Glu Arg Val Thr Phe Thr Ala Asp Lys Ser Thr Arg Thr Ala Tyr
65                  70                  75                  80

atg gac ctg acc agt ctg agt tct gac gac acg gcc ttt tac ttc tgt       288
Met Asp Leu Thr Ser Leu Ser Ser Asp Asp Thr Ala Phe Tyr Phe Cys
                85                  90                  95

gcg gtc ggt gtt atg aaa att ttt gga gaa gtt cca cta aac ctt gac       336
Ala Val Gly Val Met Lys Ile Phe Gly Glu Val Pro Leu Asn Leu Asp
            100                 105                 110

ttc tgg ggt cag ggc acc ctg gtc acc gtc tcg agc                       372
Phe Trp Gly Gln Gly Thr Leu Val Thr Val Ser Ser

115                 120


<210>  2
<211>  124
<212>  PRT
<213>  Artificial

<220>
<223>  Amino acid sequence of e2d066 or e2d081 VH region

<400>  2

Gln Val Gln Leu Val Gln Ser Gly Ser Asp Val Lys Lys Pro Gly Ser
1               5                   10                  15


Ser Val Arg Val Ser Cys Lys Ala Ser Gly Gly Ser Phe Asn Ser Tyr
            20                  25                  30


Ala Val Asn Trp Val Arg Gln Ala Pro Gly Gln Gly Leu Glu Trp Met
        35                  40                  45


Gly Arg Ile Met Pro Leu Val Gly Leu Pro Glu Tyr Ala Glu Arg Phe
    50                  55                  60


Gln Glu Arg Val Thr Phe Thr Ala Asp Lys Ser Thr Arg Thr Ala Tyr
65                  70                  75                  80


Met Asp Leu Thr Ser Leu Ser Ser Asp Asp Thr Ala Phe Tyr Phe Cys
                85                  90                  95


Ala Val Gly Val Met Lys Ile Phe Gly Glu Val Pro Leu Asn Leu Asp
            100                 105                 110


Phe Trp Gly Gln Gly Thr Leu Val Thr Val Ser Ser
        115                 120


<210>  3
<211>  330
<212>  DNA
<213>  Artificial

<220>
<223>  DNA sequence of e2d066 VL region


<220>
<221>  CDS
<222>  (1)..(330)

<400>  3
cag gct gtg ctc act cag ccg tct tcc gtg tct ggg tct cct gga cag        48
Gln Ala Val Leu Thr Gln Pro Ser Ser Val Ser Gly Ser Pro Gly Gln
1               5                   10                  15

tcg atc acc atc tcc tgc act gga acc agc aat gac gtg ggt agt tat          96
Ser Ile Thr Ile Ser Cys Thr Gly Thr Ser Asn Asp Val Gly Ser Tyr
            20                  25                  30

cgc tat gtc tcc tgg ttc caa cag cac cca ggc aaa gcc ccc aaa ctc          144
Arg Tyr Val Ser Trp Phe Gln Gln His Pro Gly Lys Ala Pro Lys Leu
        35                  40                  45

atg att tat gat gtc aat aaa cgg ccc tca ggg gtt tct agt cgc ttc          192
Met Ile Tyr Asp Val Asn Lys Arg Pro Ser Gly Val Ser Ser Arg Phe
    50                  55                  60

tct ggc tcc aag tct ggc aac acg gcc tcc ctg acc atc tct ggg ctc          240
Ser Gly Ser Lys Ser Gly Asn Thr Ala Ser Leu Thr Ile Ser Gly Leu
65                  70                  75                  80

cag gct gag gac gag gct aat tat tac tgc agc tca tat aca aga agc          288
Gln Ala Glu Asp Glu Ala Asn Tyr Tyr Cys Ser Ser Tyr Thr Arg Ser
                85                  90                  95

agc agt ttg gct ttc ggc gga ggg acc aaa ctg acc gtc cta                  330
Ser Ser Leu Ala Phe Gly Gly Gly Thr Lys Leu Thr Val Leu
            100                 105                 110


<210>  4
<211>  110
<212>  PRT
<213>  Artificial

<220>
<223>  Amino acid sequence of e2d066 VL region

<400>  4

Gln Ala Val Leu Thr Gln Pro Ser Ser Val Ser Gly Ser Pro Gly Gln
1               5                   10                  15


Ser Ile Thr Ile Ser Cys Thr Gly Thr Ser Asn Asp Val Gly Ser Tyr
            20                  25                  30


Arg Tyr Val Ser Trp Phe Gln Gln His Pro Gly Lys Ala Pro Lys Leu
        35                  40                  45


Met Ile Tyr Asp Val Asn Lys Arg Pro Ser Gly Val Ser Ser Arg Phe
    50                  55                  60


Ser Gly Ser Lys Ser Gly Asn Thr Ala Ser Leu Thr Ile Ser Gly Leu
65                  70                  75                  80


Gln Ala Glu Asp Glu Ala Asn Tyr Tyr Cys Ser Ser Tyr Thr Arg Ser
                85                  90                  95


Ser Ser Leu Ala Phe Gly Gly Gly Thr Lys Leu Thr Val Leu
            100                 105                 110

<210>  5
<211>  5
<212>  PRT
<213>  Artificial

<220>
<223>  Amino acid sequence of e2d066 or e2d081 VH CDR1

<400>  5

Ser Tyr Ala Val Asn
1               5


<210>  6
<211>  17
<212>  PRT
<213>  Artificial

<220>
<223>  Amino acid sequence of e2d066 or e2d081 VH CDR2

<400>  6

Arg Ile Met Pro Leu Val Gly Leu Pro Glu Tyr Ala Glu Arg Phe Gln
1               5                   10                  15


Glu


<210>  7
<211>  15
<212>  PRT
<213>  Artificial

<220>
<223>  Amino acid sequence of e2d066 or e2d081 VH CDR3

<400>  7

Gly Val Met Lys Ile Phe Gly Glu Val Pro Leu Asn Leu Asp Phe
1               5                   10                  15


<210>  8
<211>  14
<212>  PRT
<213>  Artificial

<220>
<223>  Amino acid sequence of e2d066 VL CDR1

<400>  8

Thr Gly Thr Ser Asn Asp Val Gly Ser Tyr Arg Tyr Val Ser
1               5                   10


<210>  9
<211>  7
<212>  PRT

<213>  Artificial

<220>
<223>  Amino acid sequence of e2d066 VL CDR2

<400>  9

Asp Val Asn Lys Arg Pro Ser
1               5


<210>  10
<211>  10
<212>  PRT
<213>  Artificial

<220>
<223>  Amino acid sequence of e2d066 VL CDR3

<400>  10

Ser Ser Tyr Thr Arg Ser Ser Ser Leu Ala
1               5                   10


<210>  11
<211>  378
<212>  DNA
<213>  Artificial

<220>
<223>  DNA sequence of e2d073 VH region


<220>
<221>  CDS
<222>  (1)..(378)

<400>  11
cag gtg cag ctg gtg cag tct ggg gct gag gtg aag aag cct ggg tcc       48
Gln Val Gln Leu Val Gln Ser Gly Ala Glu Val Lys Lys Pro Gly Ser
1               5                   10                  15

tcg gtg aag gtc tcc tgc aag gct tct gga gcc tcc ttc aat agc ttt       96
Ser Val Lys Val Ser Cys Lys Ala Ser Gly Ala Ser Phe Asn Ser Phe
            20                  25                  30

gct atc gac tgg gtg cga cag gcc cct gga caa ggg ctt gag tgg atg      144
Ala Ile Asp Trp Val Arg Gln Ala Pro Gly Gln Gly Leu Glu Trp Met
        35                  40                  45

gga agg atc atc ccc atc gca gat gtt tca gac tac gca cag aag ttc      192
Gly Arg Ile Ile Pro Ile Ala Asp Val Ser Asp Tyr Ala Gln Lys Phe
    50                  55                  60

cag ggc aga gtc aca att acc gcg gac aaa tcc acg agg aca gcc tac      240
Gln Gly Arg Val Thr Ile Thr Ala Asp Lys Ser Thr Arg Thr Ala Tyr
65                  70                  75                  80

atg gag ctg acc acc ctg aga tct gac gac acg gcc gta tat tac tgt      288
Met Glu Leu Thr Thr Leu Arg Ser Asp Asp Thr Ala Val Tyr Tyr Cys
                85                  90                  95

gcg acc tcc cct atg ctt acg ttt ggg gga cct aac gct ttt ggt gct          336
Ala Thr Ser Pro Met Leu Thr Phe Gly Gly Pro Asn Ala Phe Gly Ala
            100                 105                 110

ttt gat gtc tgg ggc cag ggg aca atg gtc acc gtc tcg agc                  378
Phe Asp Val Trp Gly Gln Gly Thr Met Val Thr Val Ser Ser
        115                 120                 125

<210>  12
<211>  126
<212>  PRT
<213>  Artificial

<220>
<223>  Amino acid sequence of e2d073 VH region

<400>  12

Gln Val Gln Leu Val Gln Ser Gly Ala Glu Val Lys Lys Pro Gly Ser
1               5                   10                  15

Ser Val Lys Val Ser Cys Lys Ala Ser Gly Ala Ser Phe Asn Ser Phe
            20                  25                  30

Ala Ile Asp Trp Val Arg Gln Ala Pro Gly Gln Gly Leu Glu Trp Met
        35                  40                  45

Gly Arg Ile Ile Pro Ile Ala Asp Val Ser Asp Tyr Ala Gln Lys Phe
    50                  55                  60

Gln Gly Arg Val Thr Ile Thr Ala Asp Lys Ser Thr Arg Thr Ala Tyr
65                  70                  75                  80

Met Glu Leu Thr Thr Leu Arg Ser Asp Asp Thr Ala Val Tyr Tyr Cys
                85                  90                  95

Ala Thr Ser Pro Met Leu Thr Phe Gly Gly Pro Asn Ala Phe Gly Ala
            100                 105                 110

Phe Asp Val Trp Gly Gln Gly Thr Met Val Thr Val Ser Ser
        115                 120                 125

<210>  13
<211>  324
<212>  DNA
<213>  Artificial

<220>
<223>  DNA sequence of e2d073 VL region

<220>
<221>  CDS
<222>  (1)..(324)

<400> 13
gac atc cag atg acc cag tct cca tcc tcc ctg tcc gca tct gta gga 48
Asp Ile Gln Met Thr Gln Ser Pro Ser Ser Leu Ser Ala Ser Val Gly
1               5                   10                  15

gac aga gtc acc atc act tgc cgg gca agt cag agc att agc agc tat 96
Asp Arg Val Thr Ile Thr Cys Arg Ala Ser Gln Ser Ile Ser Ser Tyr
            20                  25                  30

tta aat tgg tat cag cag aaa cca ggg aaa gcc cct aag ctc ctg atc 144
Leu Asn Trp Tyr Gln Gln Lys Pro Gly Lys Ala Pro Lys Leu Leu Ile
        35                  40                  45

tat gct gca tcc agt ttg caa agt ggg gtc cca tca agg ttc agt ggc 192
Tyr Ala Ala Ser Ser Leu Gln Ser Gly Val Pro Ser Arg Phe Ser Gly
    50                  55                  60

agt gga tct ggg aca gat ttc act ctc acc gtc agc agt ctg caa cct 240
Ser Gly Ser Gly Thr Asp Phe Thr Leu Thr Val Ser Ser Leu Gln Pro
65                  70                  75                  80

gaa gat ttt gca act tac tac tgt caa cag agt tac agt acc cct caa 288
Glu Asp Phe Ala Thr Tyr Tyr Cys Gln Gln Ser Tyr Ser Thr Pro Gln
                85                  90                  95

ttc act ttc ggc cct ggg acc aaa gtg gat atc aaa 324
Phe Thr Phe Gly Pro Gly Thr Lys Val Asp Ile Lys
            100                 105


<210> 14
<211> 108
<212> PRT
<213> Artificial

<220>
<223> Amino acid sequence of e2d073 VL region

<400> 14

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Ser Ser Tyr
            20                  25                  30


Leu Asn Trp Tyr Gln Gln Lys Pro Gly Lys Ala Pro Lys Leu Leu Ile
        35                  40                  45


Tyr Ala Ala Ser Ser Leu Gln Ser Gly Val Pro Ser Arg Phe Ser Gly
    50                  55                  60


Ser Gly Ser Gly Thr Asp Phe Thr Leu Thr Val Ser Ser Leu Gln Pro
65                  70                  75                  80


Glu Asp Phe Ala Thr Tyr Tyr Cys Gln Gln Ser Tyr Ser Thr Pro Gln
                85                  90                  95

Phe Thr Phe Gly Pro Gly Thr Lys Val Asp Ile Lys
            100                 105

<210>  15
<211>  5
<212>  PRT
<213>  Artificial

<220>
<223>  Amino acid sequence of e2d073 VH CDR1

<400>  15

Ser Phe Ala Ile Asp
1               5

<210>  16
<211>  17
<212>  PRT
<213>  Artificial

<220>
<223>  Amino acid sequence of e2d073 VH CDR2

<400>  16

Arg Ile Ile Pro Ile Ala Asp Val Ser Asp Tyr Ala Gln Lys Phe Gln
1               5                   10                  15

Gly

<210>  17
<211>  17
<212>  PRT
<213>  Artificial

<220>
<223>  Amino acid sequence of e2d073 VH CDR3

<400>  17

Ser Pro Met Leu Thr Phe Gly Gly Pro Asn Ala Phe Gly Ala Phe Asp
1               5                   10                  15

Val

<210>  18
<211>  11
<212>  PRT
<213>  Artificial

<220>
<223>  Amino acid sequence of e2d073 VL CDR1

<400>  18

Arg Ala Ser Gln Ser Ile Ser Ser Tyr Leu Asn
1               5                   10


<210>  19
<211>  7
<212>  PRT
<213>  Artificial

<220>
<223>  Amino acid sequence of e2d073 VL CDR2

<400>  19

Ala Ala Ser Ser Leu Gln Ser
1               5


<210>  20
<211>  10
<212>  PRT
<213>  Artificial

<220>
<223>  Amino acid sequence of e2d073 VL CDR3

<400>  20

Gln Gln Ser Tyr Ser Thr Pro Gln Phe Thr
1               5                   10


<210>  21
<211>  330
<212>  DNA
<213>  Artificial

<220>
<223>  DNA sequence of e2d081 VL region

<400>  21
cagtctgtgt tgacgcagcc gccctcagtg tctgcggccc caggacagaa ggtcaccatc      60

tcctgctctg gaaccagctc caacattggg gataattatg tatcctggta ccagcatctc     120

ccaggatcag cccccaaact cctcatttat gacaataata agcgaccctc agggattcct     180

gaccgattct ctggctccaa gtctggcacg tcagccaccc tgggcatcac cggactccag     240

actggagacg aggccgattt ttactgcgga acatgggata gcagcctgag ttctgtggtg     300

ttcggcggag ggaccaagct gaccgtccta                                      330


<210>  22
<211>  110
<212>  PRT
<213>  Artificial

<220>

<223>  Amino acid sequence of e2d081 VL region

<400>  22

Gln Ser Val Leu Thr Gln Pro Pro Ser Val Ser Ala Ala Pro Gly Gln
1               5                   10                  15

Lys Val Thr Ile Ser Cys Ser Gly Thr Ser Ser Asn Ile Gly Asp Asn
            20                  25                  30

Tyr Val Ser Trp Tyr Gln His Leu Pro Gly Ser Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Phe Tyr Cys Gly Thr Trp Asp Ser Ser Leu
                85                  90                  95

Ser Ser Val Val Phe Gly Gly Gly Thr Lys Leu Thr Val Leu
            100                 105                 110


<210>  23
<211>  13
<212>  PRT
<213>  Artificial

<220>
<223>  Amino acid sequence of e2d081 VL CDR1

<400>  23

Ser Gly Thr Ser Ser Asn Ile Gly Asp Asn Tyr Val Ser
1               5                   10


<210>  24
<211>  7
<212>  PRT
<213>  Artificial

<220>
<223>  Amino acid sequence of e2d081 VL CDR2

<400>  24

Asp Asn Asn Lys Arg Pro Ser
1               5


<210>  25
<211>  11
<212>  PRT

<213>  Artificial

<220>
<223>  Amino acid sequence of e2d081 VL CDR3

<400>  25

Gly Thr Trp Asp Ser Ser Leu Ser Ser Val Val
1               5                   10


<210>  26
<211>  454
<212>  PRT
<213>  Artificial

<220>
<223>  Amino acid sequence of entire heavy chain of e2d066 IgG antibody

<400>  26

Gln Val Gln Leu Val Gln Ser Gly Ser Asp Val Lys Lys Pro Gly Ser
1               5                   10                  15

Ser Val Arg Val Ser Cys Lys Ala Ser Gly Gly Ser Phe Asn Ser Tyr
            20                  25                  30

Ala Val Asn Trp Val Arg Gln Ala Pro Gly Gln Gly Leu Glu Trp Met
        35                  40                  45

Gly Arg Ile Met Pro Leu Val Gly Leu Pro Glu Tyr Ala Glu Arg Phe
    50                  55                  60

Gln Glu Arg Val Thr Phe Thr Ala Asp Lys Ser Thr Arg Thr Ala Tyr
65                  70                  75                  80

Met Asp Leu Thr Ser Leu Ser Ser Asp Asp Thr Ala Phe Tyr Phe Cys
                85                  90                  95

Ala Val Gly Val Met Lys Ile Phe Gly Glu Val Pro Leu Asn Leu Asp
            100                 105                 110

Phe Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Asp Glu Leu Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys

420                 425                 430

Ser Val Met His Glu Ala Pro His Asn His Tyr Thr Gln Lys Ser Leu
        435                 440                 445

Ser Leu Ser Pro Gly Lys
    450

<210>  27
<211>  215
<212>  PRT
<213>  Artificial

<220>
<223>  Amino acid sequence of entire light chain of e2d066 IgG antibody

<400>  27

Gln Ala Val Leu Thr Gln Pro Ser Ser Val Ser Gly Ser Pro Gly Gln
1               5                   10                  15

Ser Ile Thr Ile Ser Cys Thr Gly Thr Ser Asn Asp Val Gly Ser Tyr
            20                  25                  30

Arg Tyr Val Ser Trp Phe Gln Gln His Pro Gly Lys Ala Pro Lys Leu
        35                  40                  45

Met Ile Tyr Asp Val Asn Lys Arg Pro Ser Gly Val Ser Ser Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asn Tyr Tyr Cys Ser Ser Tyr Thr Arg Ser
                85                  90                  95

Ser Ser Leu Ala Phe Gly Gly Gly Thr Lys Leu Thr Val Leu Ser Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Lys Ser Tyr Ser Cys Gln Val Thr His Glu Gly Ser Thr Val Glu Lys
        195                 200                 205

Thr Val Ala Pro Thr Glu Cys
    210                 215

<210>  28
<211>  456
<212>  PRT
<213>  Artificial

<220>
<223>  Amino acid sequence of entire heavy chain of e2d073 IgG antibody

<400>  28

Gln Val Gln Leu Val Gln Ser Gly Ala Glu Val Lys Lys Pro Gly Ser
1               5                   10                  15

Ser Val Lys Val Ser Cys Lys Ala Ser Gly Ala Ser Phe Asn Ser Phe
            20                  25                  30

Ala Ile Asp Trp Val Arg Gln Ala Pro Gly Gln Gly Leu Glu Trp Met
        35                  40                  45

Gly Arg Ile Ile Pro Ile Ala Asp Val Ser Asp Tyr Ala Gln Lys Phe
    50                  55                  60

Gln Gly Arg Val Thr Ile Thr Ala Asp Lys Ser Thr Arg Thr Ala Tyr
65                  70                  75                  80

Met Glu Leu Thr Thr Leu Arg Ser Asp Asp Thr Ala Val Tyr Tyr Cys
                85                  90                  95

Ala Thr Ser Pro Met Leu Thr Phe Gly Gly Pro Asn Ala Phe Gly Ala
            100                 105                 110

Phe Asp Val Trp Gly Gln Gly Thr Met Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Pro His Asn His Tyr Thr Gln Lys
        435                 440                 445

Ser Leu Ser Leu Ser Pro Gly Lys
    450                 455

<210>  29
<211>  215
<212>  PRT
<213>  Artificial

<220>
<223>  Amino acid sequence of entire light chain of e2d073 IgG antibody

<400>  29

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Val Ser Ser Leu Gln Pro
65                  70                  75                  80

Glu Asp Phe Ala Thr Tyr Tyr Cys Gln Gln Ser Tyr Ser Thr Pro Gln
                85                  90                  95

Phe Thr Phe Gly Pro Gly Thr Lys Val Asp Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  30
<211>  454
<212>  PRT
<213>  Artificial

<220>
<223>  Amino acid sequence of entire heavy chain of e2d081 IgG antibody

<400>  30

Gln Val Gln Leu Val Gln Ser Gly Ser Asp Val Lys Lys Pro Gly Ser
1               5                   10                  15

Ser Val Arg Val Ser Cys Lys Ala Ser Gly Gly Ser Phe Asn Ser Tyr
            20                  25                  30

Ala Val Asn Trp Val Arg Gln Ala Pro Gly Gln Gly Leu Glu Trp Met
        35                  40                  45

Gly Arg Ile Met Pro Leu Val Gly Leu Pro Glu Tyr Ala Glu Arg Phe
    50                  55                  60

Gln Glu Arg Val Thr Phe Thr Ala Asp Lys Ser Thr Arg Thr Ala Tyr
65                  70                  75                  80

Met Asp Leu Thr Ser Leu Ser Ser Asp Asp Thr Ala Phe Tyr Phe Cys
                85                  90                  95

Ala Val Gly Val Met Lys Ile Phe Gly Glu Val Pro Leu Asn Leu Asp
            100                 105                 110

Phe Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Asp Glu Leu Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Pro His Asn His Tyr Thr Gln Lys Ser Leu
        435                 440                 445

Ser Leu Ser Pro Gly Lys
    450

<210>  31
<211>  215
<212>  PRT
<213>  Artificial

<220>
<223>  Amino acid sequence of entire light chain of e2d081 IgG antibody

<400>  31

Gln Ser Val Leu Thr Gln Pro Pro Ser Val Ser Ala Ala Pro Gly Gln
1               5                   10                  15

Lys Val Thr Ile Ser Cys Ser Gly Thr Ser Ser Asn Ile Gly Asp Asn
            20                  25                  30

Tyr Val Ser Trp Tyr Gln His Leu Pro Gly Ser Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Phe Tyr Cys Gly Thr Trp Asp Ser Ser Leu
                85                  90                  95

Ser Ser Val Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Lys Ser Tyr Ser Cys Gln Val Thr His Glu Gly Ser Thr Val Glu Lys
        195                 200                 205

Thr Val Ala Pro Thr Glu Cys
    210                 215

<210>  32
<211>  488
<212>  PRT
<213>  Artificial

<220>
<223>  Amino acid sequence of whole e2d066 scFv antibody

<400>  32

Gln Val Gln Leu Val Gln Ser Gly Ser Asp Val Lys Lys Pro Gly Ser
1               5                   10                  15

Ser Val Arg Val Ser Cys Lys Ala Ser Gly Gly Ser Phe Asn Ser Tyr
            20                  25                  30

Ala Val Asn Trp Val Arg Gln Ala Pro Gly Gln Gly Leu Glu Trp Met
        35                  40                  45

Gly Arg Ile Met Pro Leu Val Gly Leu Pro Glu Tyr Ala Glu Arg Phe
    50                  55                  60

Gln Glu Arg Val Thr Phe Thr Ala Asp Lys Ser Thr Arg Thr Ala Tyr
65                  70                  75                  80

Met Asp Leu Thr Ser Leu Ser Ser Asp Asp Thr Ala Phe Tyr Phe Cys
                85                  90                  95

Ala Val Gly Val Met Lys Ile Phe Gly Glu Val Pro Leu Asn Leu Asp
            100                 105                 110

Phe Trp Gly Gln Gly Thr Leu Val Thr Val Ser Ser Gly Gly Gly Gly
        115                 120                 125

Ser Gly Gly Gly Gly Ser Gly Gly Gly Gly Ser Met Ala Gln Ala Val

130                 135                 140

Leu Thr Gln Pro Ser Ser Val Ser Gly Ser Pro Gly Gln Ser Ile Thr
145                 150                 155                 160

Ile Ser Cys Thr Gly Thr Ser Asn Asp Val Gly Ser Tyr Arg Tyr Val
                165                 170                 175

Ser Trp Phe Gln Gln His Pro Gly Lys Ala Pro Lys Leu Met Ile Tyr
            180                 185                 190

Asp Val Asn Lys Arg Pro Ser Gly Val Ser Ser Arg Phe Ser Gly Ser
        195                 200                 205

Lys Ser Gly Asn Thr Ala Ser Leu Thr Ile Ser Gly Leu Gln Ala Glu
    210                 215                 220

Asp Glu Ala Asn Tyr Tyr Cys Ser Ser Tyr Thr Arg Ser Ser Ser Leu
225                 230                 235                 240

Ala Phe Gly Gly Gly Thr Lys Leu Thr Val Leu Ser Gln Pro Lys Ala
                245                 250                 255

Ala Pro Ser Val Thr Leu Phe Pro Pro Ser Ser Glu Glu Leu Gln Ala
            260                 265                 270

Asn Lys Ala Thr Leu Val Cys Leu Ile Ser Asp Phe Tyr Pro Gly Ala
        275                 280                 285

Val Thr Val Ala Trp Lys Ala Asp Ser Ser Pro Val Lys Ala Gly Val
    290                 295                 300

Glu Thr Thr Thr Pro Ser Lys Gln Ser Asn Asn Lys Tyr Ala Ala Ser
305                 310                 315                 320

Ser Tyr Leu Ser Leu Thr Pro Glu Gln Trp Lys Ser His Lys Ser Tyr
                325                 330                 335

Ser Cys Gln Val Thr His Glu Gly Ser Thr Val Glu Lys Thr Val Ala
            340                 345                 350

Pro Thr Glu Cys Ser Ala Arg Gln Ser Thr Ala Gln His Asp Glu Ala
        355                 360                 365

Val Asp Asn Lys Phe Asn Lys Glu Gln Gln Asn Ala Phe Tyr Glu Ile
    370                 375                 380

Leu His Leu Pro Asn Leu Asn Glu Glu Gln Arg Asn Ala Phe Ile Gln
385                 390                 395                 400

Ser Leu Lys Asp Asp Pro Ser Gln Ser Ala Asn Leu Leu Ala Glu Ala
                405                 410                 415

Lys Lys Leu Asn Asp Ala Gln Ala Pro Lys Val Asp Asn Lys Phe Asn
            420                 425                 430

Lys Glu Gln Gln Asn Ala Phe Tyr Glu Ile Leu His Leu Pro Asn Leu
        435                 440                 445

Asn Glu Glu Gln Arg Asn Ala Phe Ile Gln Ser Leu Lys Asp Asp Pro
    450                 455                 460

Ser Gln Ser Ala Asn Leu Leu Ala Glu Ala Lys Lys Leu Asn Asp Ala
465                 470                 475                 480

Gln Ala Pro Lys Val Asp Ala Asn
                485

<210>  33
<211>  490
<212>  PRT
<213>  Artificial

<220>
<223>  Amino acid sequence of whole e2d073 scFv antibody

<400>  33

Gln Val Gln Leu Val Gln Ser Gly Ala Glu Val Lys Lys Pro Gly Ser
1               5                   10                  15

Ser Val Lys Val Ser Cys Lys Ala Ser Gly Ala Ser Phe Asn Ser Phe
            20                  25                  30

Ala Ile Asp Trp Val Arg Gln Ala Pro Gly Gln Gly Leu Glu Trp Met
        35                  40                  45

Gly Arg Ile Ile Pro Ile Ala Asp Val Ser Asp Tyr Ala Gln Lys Phe
    50                  55                  60

Gln Gly Arg Val Thr Ile Thr Ala Asp Lys Ser Thr Arg Thr Ala Tyr
65                  70                  75                  80

Met Glu Leu Thr Thr Leu Arg Ser Asp Asp Thr Ala Val Tyr Tyr Cys
                85                  90                  95

Ala Thr Ser Pro Met Leu Thr Phe Gly Gly Pro Asn Ala Phe Gly Ala

            100                 105                 110

Phe Asp Val Trp Gly Gln Gly Thr Met Val Thr Val Ser Ser Gly Gly
        115                 120                 125

Gly Gly Ser Gly Gly Gly Gly Ser Gly Gly Gly Gly Ser Met Ala Asp
    130                 135                 140

Ile Gln Met Thr Gln Ser Pro Ser Ser Leu Ser Ala Ser Val Gly Asp
145                 150                 155                 160

Arg Val Thr Ile Thr Cys Arg Ala Ser Gln Ser Ile Ser Ser Tyr Leu
                165                 170                 175

Asn Trp Tyr Gln Gln Lys Pro Gly Lys Ala Pro Lys Leu Leu Ile Tyr
            180                 185                 190

Ala Ala Ser Ser Leu Gln Ser Gly Val Pro Ser Arg Phe Ser Gly Ser
        195                 200                 205

Gly Ser Gly Thr Asp Phe Thr Leu Thr Val Ser Ser Leu Gln Pro Glu
    210                 215                 220

Asp Phe Ala Thr Tyr Tyr Cys Gln Gln Ser Tyr Ser Thr Pro Gln Phe
225                 230                 235                 240

Thr Phe Gly Pro Gly Thr Lys Val Asp Ile Lys Arg Thr Val Ala Ala
                245                 250                 255

Pro Ser Val Phe Ile Phe Pro Pro Ser Asp Glu Gln Leu Lys Ser Gly
            260                 265                 270

Thr Ala Ser Val Val Cys Leu Leu Asn Asn Phe Tyr Pro Arg Glu Ala
        275                 280                 285

Lys Val Gln Trp Lys Val Asp Asn Ala Leu Gln Ser Gly Asn Ser Gln
    290                 295                 300

Glu Ser Val Thr Glu Gln Asp Ser Lys Asp Ser Thr Tyr Ser Leu Ser
305                 310                 315                 320

Ser Thr Leu Thr Leu Ser Lys Ala Asp Tyr Glu Lys His Lys Val Tyr
                325                 330                 335

Ala Cys Glu Val Thr His Gln Gly Leu Ser Ser Pro Val Thr Lys Ser
            340                 345                 350

Phe Asn Arg Gly Glu Cys Ser Ala Arg Gln Ser Thr Ala Gln His Asp
        355                 360                 365

Glu Ala Val Asp Asn Lys Phe Asn Lys Glu Gln Gln Asn Ala Phe Tyr
    370                 375                 380

Glu Ile Leu His Leu Pro Asn Leu Asn Glu Glu Gln Arg Asn Ala Phe
385                 390                 395                 400

Ile Gln Ser Leu Lys Asp Asp Pro Ser Gln Ser Ala Asn Leu Leu Ala
                405                 410                 415

Glu Ala Lys Lys Leu Asn Asp Ala Gln Ala Pro Lys Val Asp Asn Lys
            420                 425                 430

Phe Asn Lys Glu Gln Gln Asn Ala Phe Tyr Glu Ile Leu His Leu Pro
        435                 440                 445

Asn Leu Asn Glu Glu Gln Arg Asn Ala Phe Ile Gln Ser Leu Lys Asp
    450                 455                 460

Asp Pro Ser Gln Ser Ala Asn Leu Leu Ala Glu Ala Lys Lys Leu Asn
465                 470                 475                 480

Asp Ala Gln Ala Pro Lys Val Asp Ala Asn
                485                 490

<210>  34
<211>  488
<212>  PRT
<213>  Artificial

<220>
<223>  Amino acid sequence of whole e2d081 scFv antibody

<400>  34

Gln Val Gln Leu Val Gln Ser Gly Ser Asp Val Lys Lys Pro Gly Ser
1               5                   10                  15

Ser Val Arg Val Ser Cys Lys Ala Ser Gly Gly Ser Phe Asn Ser Tyr
            20                  25                  30

Ala Val Asn Trp Val Arg Gln Ala Pro Gly Gln Gly Leu Glu Trp Met
        35                  40                  45

Gly Arg Ile Met Pro Leu Val Gly Leu Pro Glu Tyr Ala Glu Arg Phe
    50                  55                  60

Gln Glu Arg Val Thr Phe Thr Ala Asp Lys Ser Thr Arg Thr Ala Tyr

65                  70                  75                  80

Met Asp Leu Thr Ser Leu Ser Ser Asp Asp Thr Ala Phe Tyr Phe Cys
                85                  90                  95

Ala Val Gly Val Met Lys Ile Phe Gly Glu Val Pro Leu Asn Leu Asp
            100                 105                 110

Phe Trp Gly Gln Gly Thr Leu Val Thr Val Ser Ser Gly Gly Gly Gly
        115                 120                 125

Ser Gly Gly Gly Gly Ser Gly Gly Gly Gly Ser Met Ala Gln Ser Val
    130                 135                 140

Leu Thr Gln Pro Pro Ser Val Ser Ala Ala Pro Gly Gln Lys Val Thr
145                 150                 155                 160

Ile Ser Cys Ser Gly Thr Ser Ser Asn Ile Gly Asp Asn Tyr Val Ser
                165                 170                 175

Trp Tyr Gln His Leu Pro Gly Ser Ala Pro Lys Leu Leu Ile Tyr Asp
            180                 185                 190

Asn Asn Lys Arg Pro Ser Gly Ile Pro Asp Arg Phe Ser Gly Ser Lys
        195                 200                 205

Ser Gly Thr Ser Ala Thr Leu Gly Ile Thr Gly Leu Gln Thr Gly Asp
    210                 215                 220

Glu Ala Asp Phe Tyr Cys Gly Thr Trp Asp Ser Ser Leu Ser Ser Val
225                 230                 235                 240

Val Phe Gly Gly Gly Thr Lys Leu Thr Val Leu Gly Gln Pro Lys Ala
                245                 250                 255

Ala Pro Ser Val Thr Leu Phe Pro Pro Ser Ser Glu Glu Leu Gln Ala
            260                 265                 270

Asn Lys Ala Thr Leu Val Cys Leu Ile Ser Asp Phe Tyr Pro Gly Ala
        275                 280                 285

Val Thr Val Ala Trp Lys Ala Asp Ser Ser Pro Val Lys Ala Gly Val
    290                 295                 300

Glu Thr Thr Thr Pro Ser Lys Gln Ser Asn Asn Lys Tyr Ala Ala Ser
305                 310                 315                 320

Ser Tyr Leu Ser Leu Thr Pro Glu Gln Trp Lys Ser His Lys Ser Tyr
                325                 330                 335

Ser Cys Gln Val Thr His Glu Gly Ser Thr Val Glu Lys Thr Val Ala
            340                 345                 350

Pro Thr Glu Cys Ser Ala Arg Gln Ser Thr Ala Gln His Asp Glu Ala
        355                 360                 365

Val Asp Asn Lys Phe Asn Lys Glu Gln Gln Asn Ala Phe Tyr Glu Ile
    370                 375                 380

Leu His Leu Pro Asn Leu Asn Glu Glu Gln Arg Asn Ala Phe Ile Gln
385                 390                 395                 400

Ser Leu Lys Asp Asp Pro Ser Gln Ser Ala Asn Leu Leu Ala Glu Ala
                405                 410                 415

Lys Lys Leu Asn Asp Ala Gln Ala Pro Lys Val Asp Asn Lys Phe Asn
            420                 425                 430

Lys Glu Gln Gln Asn Ala Phe Tyr Glu Ile Leu His Leu Pro Asn Leu
        435                 440                 445

Asn Glu Glu Gln Arg Asn Ala Phe Ile Gln Ser Leu Lys Asp Asp Pro
    450                 455                 460

Ser Gln Ser Ala Asn Leu Leu Ala Glu Ala Lys Lys Leu Asn Asp Ala
465                 470                 475                 480

Gln Ala Pro Lys Val Asp Ala Asn
                485

<210>  35
<211>  15
<212>  PRT
<213>  Artificial

<220>
<223>  Amino acid sequence of linker

<400>  35

Gly Gly Gly Gly Ser Gly Gly Gly Gly Ser Gly Gly Gly Gly Ser
1               5                   10                  15

<210>  36
<211>  333
<212>  PRT
<213>  Artificial

<220>
<223>  Amino acid sequence of E2 protein of TH strain

<400>  36

Ala Thr Tyr Val Thr Gly Gly Ser Glu Ala Arg Gly Ala Ser Gly Leu
1               5                   10                  15

Ala Asn Leu Phe Ser Phe Gly Ala Ser Gln Lys Ile Gln Leu Ile Asn
            20                  25                  30

Thr Asn Gly Ser Trp His Ile Asn Arg Thr Ala Leu Asn Cys Asn Asp
        35                  40                  45

Ser Leu His Thr Gly Phe Leu Ala Ala Leu Phe Tyr Thr His Lys Phe
    50                  55                  60

Asn Ala Ser Gly Cys Pro Glu Arg Met Ala Ser Cys Arg Pro Ile Glu
65                  70                  75                  80

Glu Phe Ala Gln Gly Tyr Gly Pro Ile Thr Tyr Ala Glu Pro Ser Pro
                85                  90                  95

Ser Asp Gln Arg Pro Tyr Cys Trp His Tyr Ala Pro Arg Pro Cys Gly
            100                 105                 110

Ile Ile Pro Ala Ser Gln Val Cys Gly Pro Val Tyr Cys Phe Thr Pro
        115                 120                 125

Ser Pro Val Val Val Gly Thr Thr Asp Arg Ser Gly Ala Pro Thr Tyr
    130                 135                 140

Asn Trp Gly Ala Asn Glu Thr Asp Val Leu Tyr Leu Asn Asn Thr Arg
145                 150                 155                 160

Pro Pro Gln Gly Asn Trp Phe Gly Cys Thr Trp Met Asn Gly Thr Gly
                165                 170                 175

Phe Thr Lys Thr Cys Gly Gly Pro Pro Cys Asn Ile Gly Gly Gly Gly
            180                 185                 190

Asn Asn Asn Thr Leu Thr Cys Pro Thr Asp Cys Phe Arg Lys His Pro
        195                 200                 205

Glu Ala Thr Tyr Thr Lys Cys Gly Ser Gly Pro Trp Leu Thr Pro Arg
    210                 215                 220

Cys Met Val Asp Tyr Pro Tyr Arg Leu Trp His Tyr Pro Cys Thr Val
225                 230                 235                 240

Asn Phe Thr Ile Phe Lys Val Arg Met Tyr Val Gly Gly Val Glu His
                245                 250                 255

Arg Leu Asn Ala Ala Cys Asn Trp Thr Arg Gly Glu Arg Cys Asn Leu
            260                 265                 270

Glu Asp Arg Asp Arg Ser Glu Leu Ser Pro Leu Leu Leu Ser Thr Thr
        275                 280                 285

Glu Trp Gln Val Leu Pro Cys Ser Phe Thr Thr Leu Pro Ala Leu Ser
    290                 295                 300

Thr Gly Leu Ile His Leu His Gln Asn Ile Val Asp Val Gln Tyr Leu
305                 310                 315                 320

Tyr Gly Ile Gly Ser Ala Val Val Ser Tyr Ala Ile Lys
                325                 330

<210>  37
<211>  12
<212>  PRT
<213>  Artificial

<220>
<223>  Amino acid sequence of TH E2 protein-derived peptide

<400>  37

Gln Leu Ile Asn Thr Asn Gly Ser Trp His Ile Asn
1               5                   10

<210>  38
<211>  105
<212>  PRT
<213>  Artificial

<220>
<223>  Amino acid sequence of e2d066 CL region

<400>  38

Ser Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn

50                  55                  60

Lys Tyr Ala Ala Ser Ser Tyr Leu Ser Leu Thr Pro Glu Gln Trp Lys
65                  70                  75                  80

Ser His Lys Ser Tyr Ser Cys Gln Val Thr His Glu Gly Ser Thr Val
                85                  90                  95

Glu Lys Thr Val Ala Pro Thr Glu Cys
            100                 105

<210>  39
<211>  107
<212>  PRT
<213>  Artificial

<220>
<223>  Amino acid sequence of e2d073 CL region

<400>  39

<210>  40
<211>  105
<212>  PRT
<213>  Artificial

<220>
<223>  Amino acid sequence of e2d081 CL region

<400>  40

Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Lys Ser Tyr Ser Cys Gln Val Thr His Glu Gly Ser Thr Val
                85                  90                  95

Glu Lys Thr Val Ala Pro Thr Glu Cys
            100                 105

<210>  41
<211>  3030
<212>  PRT
<213>  Artificial

<220>
<223>  precursor protein of chimeric HCV particle of TH strain and JFH-1
strain
(TH/JFH-1 HCVcc)

<400>  41

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Asp Arg Arg Pro Glu Gly Arg Ala Trp Ala Gln Pro Gly
65                  70                  75                  80

Tyr Pro Trp Pro Leu Tyr Gly Asn Glu Gly Met Gly Trp Ala Gly Trp
                85                  90                  95

Leu Leu Ser Pro Arg Gly Ser Arg Pro Ser Trp Gly Pro Asn Asp Pro
        100                 105                 110

Arg Arg Arg Ser Arg Asn Leu Gly Lys Val Ile Asp Thr Leu Thr Cys
    115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala Tyr
            180                 185                 190

Glu Val Arg Asn Val Ser Gly Val Tyr His Val Thr Asn Asp Cys Ser
        195                 200                 205

Asn Ser Ser Ile Val Tyr Glu Thr Gly Asp Met Ile Met His Thr Pro
    210                 215                 220

Gly Cys Val Pro Cys Val Arg Glu Asn Asn Ser Ser Arg Cys Trp Ala
225                 230                 235                 240

Ala Leu Thr Pro Thr Leu Ala Ala Arg Asn Ala Ser Val Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg His Glu Thr Val Gln Asp Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Thr Ala Leu Leu Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Ala Gly Asn Trp
        355                 360                 365

Ala Lys Val Leu Ile Val Leu Leu Leu Phe Ala Gly Val Asp Gly Ala
    370                 375                 380

Thr Tyr Val Thr Gly Gly Ser Glu Ala Arg Gly Ala Ser Gly Leu Ala
385                 390                 395                 400

Asn Leu Phe Ser Phe Gly Ala Ser Gln Lys Ile Gln Leu Ile Asn Thr
                405                 410                 415

Asn Gly Ser Trp His Ile Asn Arg Thr Ala Leu Asn Cys Asn Asp Ser
            420                 425                 430

Leu His Thr Gly Phe Leu Ala Ala Leu Phe Tyr Thr His Lys Phe Asn
        435                 440                 445

Ala Ser Gly Cys Pro Glu Arg Met Ala Ser Cys Arg Pro Ile Glu Glu
    450                 455                 460

Phe Ala Gln Gly Tyr Gly Pro Ile Thr Tyr Ala Glu Pro Ser Pro Ser
465                 470                 475                 480

Asp Gln Arg Pro Tyr Cys Trp His Tyr Ala Pro Arg Pro Cys Gly Ile
                485                 490                 495

Ile Pro Ala Ser Gln Val Cys Gly Pro Val Tyr Cys Phe Thr Pro Ser
            500                 505                 510

Pro Val Val Val Gly Thr Thr Asp Arg Ser Gly Ala Pro Thr Tyr Asn
        515                 520                 525

Trp Gly Ala Asn Glu Thr Asp Val Leu Tyr Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asn Ile Gly Gly Gly Gly Asn
                565                 570                 575

Asn Asn Thr Leu Thr Cys Pro Thr Asp Cys Phe Arg Lys His Pro Glu
            580                 585                 590

Ala Thr Tyr Thr Lys Cys Gly Ser Gly Pro Trp Leu Thr Pro Arg Cys

595                 600                 605

Met Val Asp Tyr Pro Tyr Arg Leu Trp His Tyr Pro Cys Thr Val Asn
    610                 615                 620

Phe Thr Ile Phe Lys Val Arg Met Tyr Val Gly Gly Val Glu His Arg
625                 630                 635                 640

Leu Asn Ala Ala Cys Asn Trp Thr Arg Gly Glu Arg Cys Asn Leu Glu
                645                 650                 655

Asp Arg Asp Arg Ser Glu Leu Ser Pro Leu Leu Leu Ser Thr Thr Glu
            660                 665                 670

Trp Gln Val Leu Pro Cys Ser Phe Thr Thr Leu Pro Ala Leu Ser Thr
        675                 680                 685

Gly Leu Ile His Leu His Gln Asn Ile Val Asp Val Gln Tyr Leu Tyr
    690                 695                 700

Gly Ile Gly Ser Ala Val Val Ser Tyr Ala Ile Lys Trp Glu Tyr Val
705                 710                 715                 720

Leu Leu Leu Phe Leu Leu Leu Ala Asp Ala Arg Val Cys Ala Cys Leu
                725                 730                 735

Trp Met Met Leu Leu Ile Ala Gln Ala Glu Ala Ala Leu Glu Asn Leu
            740                 745                 750

Val Val Leu Asn Ala Ala Ser Leu Ala Gly Ala His Gly Leu Leu Ser
        755                 760                 765

Phe Leu Val Phe Phe Cys Ala Ala Trp Tyr Ile Lys Gly Arg Leu Ile
    770                 775                 780

Pro Gly Ala Ala Tyr Ala Phe Tyr Gly Val Trp Pro Leu Leu Leu Leu
785                 790                 795                 800

Leu Leu Ala Leu Pro Pro Arg Ala Tyr Ala Met Asp Arg Glu Met Ala
                805                 810                 815

Ala Ser Cys Gly Gly Ala Val Phe Val Gly Leu Ala Phe Leu Thr Leu
            820                 825                 830

Ser Pro His Tyr Lys Ala Phe Leu Ala Lys Leu Leu Trp Trp Leu Cys
        835                 840                 845

Tyr Leu Leu Thr Leu Gly Glu Ala Met Ile Gln Glu Trp Val Pro Pro
    850                 855                 860

Met Gln Val Arg Gly Gly Arg Asp Gly Ile Ala Trp Ala Val Thr Ile
865                 870                 875                 880

Phe Cys Pro Gly Val Val Phe Asp Ile Thr Lys Trp Leu Leu Ala Leu
                885                 890                 895

Leu Gly Pro Ala Tyr Leu Leu Arg Ala Ala Leu Thr His Val Pro Tyr
            900                 905                 910

Phe Val Arg Ala His Ala Leu Ile Arg Val Cys Ala Leu Val Lys Gln
        915                 920                 925

Leu Ala Gly Gly Arg Tyr Val Gln Val Ala Leu Leu Ala Leu Gly Arg
    930                 935                 940

Trp Thr Gly Thr Tyr Ile Tyr Asp His Leu Thr Pro Met Ser Asp Trp
945                 950                 955                 960

Ala Ala Ser Gly Leu Arg Asp Leu Ala Val Ala Val Glu Pro Ile Ile
                965                 970                 975

Phe Ser Pro Met Glu Lys Lys Val Ile Val Trp Gly Ala Glu Thr Ala
            980                 985                 990

Ala Cys Gly Asp Ile Leu His Gly  Leu Pro Val Ser Ala  Arg Leu Gly
        995                 1000                1005

Gln Glu  Ile Leu Leu Gly Pro  Ala Asp Gly Tyr Thr  Ser Lys Gly
    1010                1015                1020

Trp Lys  Leu Leu Ala Pro Ile  Thr Ala Tyr Ala Gln  Gln Thr Arg
    1025                1030                1035

Gly Leu  Leu Gly Ala Ile Val  Val Ser Met Thr Gly  Arg Asp Arg
    1040                1045                1050

Thr Glu  Gln Ala Gly Glu Val  Gln Ile Leu Ser Thr  Val Ser Gln
    1055                1060                1065

Ser Phe  Leu Gly Thr Thr Ile  Ser Gly Val Leu Trp  Thr Val Tyr
    1070                1075                1080

His Gly  Ala Gly Asn Lys Thr  Leu Ala Gly Leu Arg  Gly Pro Val
    1085                1090                1095

Thr Gln Met Tyr Ser Ser Ala Glu Gly Asp Leu Val Gly Trp Pro
    1100                1105                1110

Ser Pro Pro Gly Thr Lys Ser Leu Glu Pro Cys Lys Cys Gly Ala
    1115                1120                1125

Val Asp Leu Tyr Leu Val Thr Arg Asn Ala Asp Val Ile Pro Ala
    1130                1135                1140

Arg Arg Arg Gly Asp Lys Arg Gly Ala Leu Leu Ser Pro Arg Pro
    1145                1150                1155

Ile Ser Thr Leu Lys Gly Ser Ser Gly Gly Pro Val Leu Cys Pro
    1160                1165                1170

Arg Gly His Val Val Gly Leu Phe Arg Ala Ala Val Cys Ser Arg
    1175                1180                1185

Gly Val Ala Lys Ser Ile Asp Phe Ile Pro Val Glu Thr Leu Asp
    1190                1195                1200

Val Val Thr Arg Ser Pro Thr Phe Ser Asp Asn Ser Thr Pro Pro
    1205                1210                1215

Ala Val Pro Gln Thr Tyr Gln Val Gly Tyr Leu His Ala Pro Thr
    1220                1225                1230

Gly Ser Gly Lys Ser Thr Lys Val Pro Val Ala Tyr Ala Ala Gln
    1235                1240                1245

Gly Tyr Lys Val Leu Val Leu Asn Pro Ser Val Ala Ala Thr Leu
    1250                1255                1260

Gly Phe Gly Ala Tyr Leu Ser Lys Ala His Gly Ile Asn Pro Asn
    1265                1270                1275

Ile Arg Thr Gly Val Arg Thr Val Met Thr Gly Glu Ala Ile Thr
    1280                1285                1290

Tyr Ser Thr Tyr Gly Lys Phe Leu Ala Asp Gly Gly Cys Ala Ser
    1295                1300                1305

Gly Ala Tyr Asp Ile Ile Ile Cys Asp Glu Cys His Ala Val Asp
    1310                1315                1320

Ala Thr Ser Ile Leu Gly Ile Gly Thr Val Leu Asp Gln Ala Glu
    1325                1330                1335

Thr Ala Gly Val Arg Leu Thr Val Leu Ala Thr Ala Thr Pro Pro
    1340                1345                1350

Gly Ser Val Thr Thr Pro His Pro Asp Ile Glu Glu Val Gly Leu
    1355                1360                1365

Gly Arg Glu Gly Glu Ile Pro Phe Tyr Gly Arg Ala Ile Pro Leu
    1370                1375                1380

Ser Cys Ile Lys Gly Gly Arg His Leu Ile Phe Cys His Ser Lys
    1385                1390                1395

Lys Lys Cys Asp Glu Leu Ala Ala Ala Leu Arg Gly Met Gly Leu
    1400                1405                1410

Asn Ala Val Ala Tyr Tyr Arg Gly Leu Asp Val Ser Ile Ile Pro
    1415                1420                1425

Ala Gln Gly Asp Val Val Val Val Ala Thr Asp Ala Leu Met Thr
    1430                1435                1440

Gly Tyr Thr Gly Asp Phe Asp Ser Val Ile Asp Cys Asn Val Ala
    1445                1450                1455

Val Thr Gln Ala Val Asp Phe Ser Leu Asp Pro Thr Phe Thr Ile
    1460                1465                1470

Thr Thr Gln Thr Val Pro Gln Asp Ala Val Ser Arg Ser Gln Arg
    1475                1480                1485

Arg Gly Arg Thr Gly Arg Gly Arg Gln Gly Thr Tyr Arg Tyr Val
    1490                1495                1500

Ser Thr Gly Glu Arg Ala Ser Gly Met Phe Asp Ser Val Val Leu
    1505                1510                1515

Cys Glu Cys Tyr Asp Ala Gly Ala Ala Trp Tyr Asp Leu Thr Pro
    1520                1525                1530

Ala Glu Thr Thr Val Arg Leu Arg Ala Tyr Phe Asn Thr Pro Gly
    1535                1540                1545

Leu Pro Val Cys Gln Asp His Leu Glu Phe Trp Glu Ala Val Phe
    1550                1555                1560

Thr Gly Leu Thr His Ile Asp Ala His Phe Leu Ser Gln Thr Lys

1565                1570                1575

Gln Ala Gly Glu Asn Phe Ala Tyr Leu Val Ala Tyr Gln Ala Thr
    1580                1585                1590

Val Cys Ala Arg Ala Lys Ala Pro Pro Pro Ser Trp Asp Ala Met
    1595                1600                1605

Trp Lys Cys Leu Ala Arg Leu Lys Pro Thr Leu Ala Gly Pro Thr
    1610                1615                1620

Pro Leu Leu Tyr Arg Leu Gly Pro Ile Thr Asn Glu Val Thr Leu
    1625                1630                1635

Thr His Pro Gly Thr Lys Tyr Ile Ala Thr Cys Met Gln Ala Asp
    1640                1645                1650

Leu Glu Val Met Thr Ser Thr Trp Val Leu Ala Gly Gly Val Leu
    1655                1660                1665

Ala Ala Val Ala Ala Tyr Cys Leu Ala Thr Gly Cys Val Ser Ile
    1670                1675                1680

Ile Gly Arg Leu His Val Asn Gln Arg Val Val Val Ala Pro Asp
    1685                1690                1695

Lys Glu Val Leu Tyr Glu Ala Phe Asp Glu Met Glu Glu Cys Ala
    1700                1705                1710

Ser Arg Ala Ala Leu Ile Glu Glu Gly Gln Arg Ile Ala Glu Met
    1715                1720                1725

Leu Lys Ser Lys Ile Gln Gly Leu Leu Gln Gln Ala Ser Lys Gln
    1730                1735                1740

Ala Gln Asp Ile Gln Pro Ala Met Gln Ala Ser Trp Pro Lys Val
    1745                1750                1755

Glu Gln Phe Trp Ala Arg His Met Trp Asn Phe Ile Ser Gly Ile
    1760                1765                1770

Gln Tyr Leu Ala Gly Leu Ser Thr Leu Pro Gly Asn Pro Ala Val
    1775                1780                1785

Ala Ser Met Met Ala Phe Ser Ala Ala Leu Thr Ser Pro Leu Ser
    1790                1795                1800

Thr Ser Thr Thr Ile Leu Leu Asn Ile Met Gly Gly Trp Leu Ala
    1805                1810                1815

Ser Gln Ile Ala Pro Pro Ala Gly Ala Thr Gly Phe Val Val Ser
    1820                1825                1830

Gly Leu Val Gly Ala Ala Val Gly Ser Ile Gly Leu Gly Lys Val
    1835                1840                1845

Leu Val Asp Ile Leu Ala Gly Tyr Gly Ala Gly Ile Ser Gly Ala
    1850                1855                1860

Leu Val Ala Phe Lys Ile Met Ser Gly Glu Lys Pro Ser Met Glu
    1865                1870                1875

Asp Val Ile Asn Leu Leu Pro Gly Ile Leu Ser Pro Gly Ala Leu
    1880                1885                1890

Val Val Gly Val Ile Cys Ala Ala Ile Leu Arg Arg His Val Gly
    1895                1900                1905

Pro Gly Glu Gly Ala Val Gln Trp Met Asn Arg Leu Ile Ala Phe
    1910                1915                1920

Ala Ser Arg Gly Asn His Val Ala Pro Thr His Tyr Val Thr Glu
    1925                1930                1935

Ser Asp Ala Ser Gln Arg Val Thr Gln Leu Leu Gly Ser Leu Thr
    1940                1945                1950

Ile Thr Ser Leu Leu Arg Arg Leu His Asn Trp Ile Thr Glu Asp
    1955                1960                1965

Cys Pro Ile Pro Cys Ser Gly Ser Trp Leu Arg Asp Val Trp Asp
    1970                1975                1980

Trp Val Cys Thr Ile Leu Thr Asp Phe Lys Asn Trp Leu Thr Ser
    1985                1990                1995

Lys Leu Phe Pro Lys Leu Pro Gly Leu Pro Phe Ile Ser Cys Gln
    2000                2005                2010

Lys Gly Tyr Lys Gly Val Trp Ala Gly Thr Gly Ile Met Thr Thr
    2015                2020                2025

Arg Cys Pro Cys Gly Ala Asn Ile Ser Gly Asn Val Arg Leu Gly
    2030                2035                2040

Ser Met Arg Ile Thr Gly Pro Lys Thr Cys Met Asn Thr Trp Gln
    2045                2050                2055

Gly Thr Phe Pro Ile Asn Cys Tyr Thr Glu Gly Gln Cys Ala Pro
    2060                2065                2070

Lys Pro Pro Thr Asn Tyr Lys Thr Ala Ile Trp Arg Val Ala Ala
    2075                2080                2085

Ser Glu Tyr Ala Glu Val Thr Gln His Gly Ser Tyr Ser Tyr Val
    2090                2095                2100

Thr Gly Leu Thr Thr Asp Asn Leu Lys Ile Pro Cys Gln Leu Pro
    2105                2110                2115

Ser Pro Glu Phe Phe Ser Trp Val Asp Gly Val Gln Ile His Arg
    2120                2125                2130

Phe Ala Pro Thr Pro Lys Pro Phe Phe Arg Asp Glu Val Ser Phe
    2135                2140                2145

Cys Val Gly Leu Asn Ser Tyr Ala Val Gly Ser Gln Leu Pro Cys
    2150                2155                2160

Glu Pro Glu Pro Asp Ala Asp Val Leu Arg Ser Met Leu Thr Asp
    2165                2170                2175

Pro Pro His Ile Thr Ala Glu Thr Ala Ala Arg Arg Leu Ala Arg
    2180                2185                2190

Gly Ser Pro Pro Ser Glu Ala Ser Ser Ser Val Ser Gln Leu Ser
    2195                2200                2205

Ala Pro Ser Leu Arg Ala Thr Cys Thr Thr His Ser Asn Thr Tyr
    2210                2215                2220

Asp Val Asp Met Val Asp Ala Asn Leu Leu Met Glu Gly Gly Val
    2225                2230                2235

Ala Gln Thr Glu Pro Glu Ser Arg Val Pro Val Leu Asp Phe Leu
    2240                2245                2250

Glu Pro Met Ala Glu Glu Glu Ser Asp Leu Glu Pro Ser Ile Pro
    2255                2260                2265

Ser Glu Cys Met Leu Pro Arg Ser Gly Phe Pro Arg Ala Leu Pro
    2270                2275                2280

Ala Trp Ala Arg Pro Asp Tyr Asn Pro Pro Leu Val Glu Ser Trp
    2285                2290                2295

Arg Arg Pro Asp Tyr Gln Pro Pro Thr Val Ala Gly Cys Ala Leu
    2300                2305                2310

Pro Pro Pro Lys Lys Ala Pro Thr Pro Pro Pro Arg Arg Arg Arg
    2315                2320                2325

Thr Val Gly Leu Ser Glu Ser Thr Ile Ser Glu Ala Leu Gln Gln
    2330                2335                2340

Leu Ala Ile Lys Thr Phe Gly Gln Pro Pro Ser Ser Gly Asp Ala
    2345                2350                2355

Gly Ser Ser Thr Gly Ala Gly Ala Ala Glu Ser Gly Gly Pro Thr
    2360                2365                2370

Ser Pro Gly Glu Pro Ala Pro Ser Glu Thr Gly Ser Ala Ser Ser
    2375                2380                2385

Met Pro Pro Leu Glu Gly Glu Pro Gly Asp Pro Asp Leu Glu Ser
    2390                2395                2400

Asp Gln Val Glu Leu Gln Pro Pro Pro Gln Gly Gly Gly Val Ala
    2405                2410                2415

Pro Gly Ser Gly Ser Gly Ser Trp Ser Thr Cys Ser Glu Glu Asp
    2420                2425                2430

Asp Thr Thr Val Cys Cys Ser Met Ser Tyr Ser Trp Thr Gly Ala
    2435                2440                2445

Leu Ile Thr Pro Cys Ser Pro Glu Glu Glu Lys Leu Pro Ile Asn
    2450                2455                2460

Pro Leu Ser Asn Ser Leu Leu Arg Tyr His Asn Lys Val Tyr Cys
    2465                2470                2475

Thr Thr Ser Lys Ser Ala Ser Gln Arg Ala Lys Lys Val Thr Phe
    2480                2485                2490

Asp Arg Thr Gln Val Leu Asp Ala His Tyr Asp Ser Val Leu Lys
    2495                2500                2505

Asp Ile Lys Leu Ala Ala Ser Lys Val Ser Ala Arg Leu Leu Thr

2510                2515                2520

Leu Glu Glu Ala Cys Gln Leu Thr Pro Pro His Ser Ala Arg Ser
    2525                2530                2535

Lys Tyr Gly Phe Gly Ala Lys Glu Val Arg Ser Leu Ser Gly Arg
    2540                2545                2550

Ala Val Asn His Ile Lys Ser Val Trp Lys Asp Leu Leu Glu Asp
    2555                2560                2565

Pro Gln Thr Pro Ile Pro Thr Thr Ile Met Ala Lys Asn Glu Val
    2570                2575                2580

Phe Cys Val Asp Pro Ala Lys Gly Gly Lys Lys Pro Ala Arg Leu
    2585                2590                2595

Ile Val Tyr Pro Asp Leu Gly Val Arg Val Cys Glu Lys Met Ala
    2600                2605                2610

Leu Tyr Asp Ile Thr Gln Lys Leu Pro Gln Ala Val Met Gly Ala
    2615                2620                2625

Ser Tyr Gly Phe Gln Tyr Ser Pro Ala Gln Arg Val Glu Tyr Leu
    2630                2635                2640

Leu Lys Ala Trp Ala Glu Lys Lys Asp Pro Met Gly Phe Ser Tyr
    2645                2650                2655

Asp Thr Arg Cys Phe Asp Ser Thr Val Thr Glu Arg Asp Ile Arg
    2660                2665                2670

Thr Glu Glu Ser Ile Tyr Gln Ala Cys Ser Leu Pro Glu Glu Ala
    2675                2680                2685

Arg Thr Ala Ile His Ser Leu Thr Glu Arg Leu Tyr Val Gly Gly
    2690                2695                2700

Pro Met Phe Asn Ser Lys Gly Gln Thr Cys Gly Tyr Arg Arg Cys
    2705                2710                2715

Arg Ala Ser Gly Val Leu Thr Thr Ser Met Gly Asn Thr Ile Thr
    2720                2725                2730

Cys Tyr Val Lys Ala Leu Ala Ala Cys Lys Ala Ala Gly Ile Val
    2735                2740                2745

Ala Pro Thr Met Leu Val Cys Gly Asp Asp Leu Val Val Ile Ser
    2750                2755                2760

Glu Ser Gln Gly Thr Glu Glu Asp Glu Arg Asn Leu Arg Ala Phe
    2765                2770                2775

Thr Glu Ala Met Thr Arg Tyr Ser Ala Pro Pro Gly Asp Pro Pro
    2780                2785                2790

Arg Pro Glu Tyr Asp Leu Glu Leu Ile Thr Ser Cys Ser Ser Asn
    2795                2800                2805

Val Ser Val Ala Leu Gly Pro Arg Gly Arg Arg Arg Tyr Tyr Leu
    2810                2815                2820

Thr Arg Asp Pro Thr Thr Pro Leu Ala Arg Ala Ala Trp Glu Thr
    2825                2830                2835

Val Arg His Ser Pro Ile Asn Ser Trp Leu Gly Asn Ile Ile Gln
    2840                2845                2850

Tyr Ala Pro Thr Ile Trp Val Arg Met Val Leu Met Thr His Phe
    2855                2860                2865

Phe Ser Ile Leu Met Val Gln Asp Thr Leu Asp Gln Asn Leu Asn
    2870                2875                2880

Phe Glu Met Tyr Gly Ser Val Tyr Ser Val Asn Pro Leu Asp Leu
    2885                2890                2895

Pro Ala Ile Ile Glu Arg Leu His Gly Leu Asp Ala Phe Ser Met
    2900                2905                2910

His Thr Tyr Ser His His Glu Leu Thr Arg Val Ala Ser Ala Leu
    2915                2920                2925

Arg Lys Leu Gly Ala Pro Pro Leu Arg Val Trp Lys Ser Arg Ala
    2930                2935                2940

Arg Ala Val Arg Ala Ser Leu Ile Ser Arg Gly Gly Lys Ala Ala
    2945                2950                2955

Val Cys Gly Arg Tyr Leu Phe Asn Trp Ala Val Lys Thr Lys Leu
    2960                2965                2970

Lys Leu Thr Pro Leu Pro Glu Ala Arg Leu Leu Asp Leu Ser Ser
    2975                2980                2985

Trp Phe Thr Val Gly Ala Gly Gly Gly Asp Ile Phe His Ser Val
    2990                2995                3000

Ser Arg Ala Arg Pro Arg Ser Leu Leu Phe Gly Leu Leu Leu Leu
    3005                3010                3015

Phe Val Gly Val Gly Leu Phe Leu Leu Pro Ala Arg
    3020                3025                3030